# EUROPEAN PATENT APPLICATION

(11) **EP 1 816 191 A1**
(43) Date of publication of application: **08.08.2007**
(21) Application number: 05809338.6
(22) Date of filing: 18.11.2005
(51) Int. Cl.: C12N 15/00, A61K 31/00, C12N 1/15, C12N 1/19, C12N 1/21, C12N 5/10, C12N 15/09, C12Q 1/68, G06F 19/00

(54) **METHOD OF SCREENING COMPOUND REGULATING THE TRANSLATION OF SPECIFIC mRNA**

(30) Priority: 19.11.2004 JP 2004336267
(71) Applicant: Takeda Pharmaceutical Company Limited, Osaka-shi, Osaka 541-0045 (JP)
(72) Inventor: NAKAMURA, Shingo, TAKEDA PHARMACEUTICAL COMP. LTD., Osaka-shi, Osaka 5328686 (JP); UENO, Hiroyuki, TAKEDA PHARMACEUTICAL COMP. LTD., Osaka-shi, Osaka 5328686 (JP)
(74) Representative: Jones, Nicholas Andrew
(86) International application number: PCT/JP2005/021622
(87) International publication number: WO 2006/054788

(57) **Abstract**

The present invention provides a screening method for a compound capable of preventing/treating a particular disease, which comprises (1) a step for selecting one or more mRNAs whose regulation of translation can result in the prevention/treatment of the disease, (2) a step for searching for mRNA(s) having a sequence, in the molecule, capable of assuming a local secondary structure capable of regulating the translation of the mRNA(s) from among the mRNAs selected in (1) above, (3) a step for selecting a particular sequence from among sequences capable of assuming a local secondary structure in the particular mRNA extracted in (2) above, (4) a step for confirming that a partial structure capable of interacting with the compound in the particular sequence selected in (3) above is not present in the region involved in the regulation of the translation of other mRNA, or even if present, is incapable of regulating the translation, and (5) a step for bringing an RNA strand comprising a particular sequence confirmed to be not present in the region involved in the regulation of the translation of other mRNA in (4) above and a test compound into contact with each other, and measuring changes in the stability of the secondary structure of the RNA strand.

SEQUENCE LISTING

<110>  Takeda Pharmaceutical Company Limited

<120>  METHOD OF SCREENING COMPOUND REGULATING THE TRANSLATION OF SPECIFIC mRNA

<130>  09834

<150>  JP 2004-336267
<151>  2004-11-19

<160>  47

<170>  PatentIn version 3.2

<210>  1
<211>  48
<212>  RNA
<213>  Homo sapiens

<400>  1
ggcagaggug gcggcggcgg caugggugcc ccgacguugc ccccugcc                48


<210>  2
<211>  24
<212>  DNA
<213>  Artificial

<220>
<223>  Primer

<400>  2
aagttcacca cggcccccaa gctt                                          24


<210>  3
<211>  24
<212>  DNA
<213>  Artificial

<220>
<223>  Primer

<400>  3
aagttcacca cggcccccaa gctt                                          24


<210>  4
<211>  26
<212>  DNA
<213>  Artificial

<220>
<223>  Primer

<400>  4
aggtcctatg attatgtccg gttatg                                        26


<210>  5
<211>  23
<212>  DNA
<213>  Artificial

<220>
<223>  Primer

<400>  5
gaatgtagcc atccatcctt gtc                                              23


<210>  6
<211>  24
<212>  DNA
<213>  Artificial

<220>
<223>  Probe

<400>  6
aaacaatccg gaagcgacca acgc                                             24


<210>  7
<211>  22
<212>  DNA
<213>  Artificial

<220>
<223>  Primer

<400>  7
ctgaccctga agttcatctg ca                                               22


<210>  8
<211>  23
<212>  DNA
<213>  Artificial

<220>
<223>  Primer

<400>  8
gaagaagtcg tgctgcttca tgt                                              23


<210>  9
<211>  16
<212>  DNA
<213>  Artificial

<220>
<223>  Probe

<400>  9
ccaccctgac ctacgg                                                      16


<210>  10
<211>  87
<212>  DNA
<213>  Artificial

<220>
<223>  Sense oligomer

<400>  10
aagcttccgc cagatttgaa tcgcgggacc cgttggcaga ggtggcggcg gcggcatggg      60

tgccccgacg ttgcccctg ccatgga                                           87


<210>  11
<211>  87

<212>  DNA
<213>  Artificial

<220>
<223>  Antisense oligomer

<400>  11
ccatggcagg gggcaacgtc ggggcaccca tgccgccgcc gccacctctg ccaacgggtc      60

ccgcgattca aatctggcgg aagctta                                          87

<210>  12
<211>  89
<212>  DNA
<213>  Artificial

<220>
<223>  Sense oligomer

<400>  12
aagcttccgc cagatttgaa tcgcgggacc cgttggcaga ggtggcggcg tcggggcatg      60

ggtgccccga cgttgccccc tgccatgga                                        89

<210>  13
<211>  89
<212>  DNA
<213>  Artificial

<220>
<223>  Antisense oligomer

<400>  13
ccatggcagg gggcaacgtc ggggcaccca tgccccgacg ccgccacctc tgccaacggg      60

tcccgcgatt caaatctggc ggaagctta                                        89

<210>  14
<211>  87
<212>  DNA
<213>  Artificial

<220>
<223>  Sense oligomer

<400>  14
   aagcttccgc cagatttgaa tcgcgggacc cgttggcagg gggcggcgtc ggggcatggg      60
   tgccccgacg ttgccccctg ccatgga                                           87

<210>  15
<211>  87
<212>  DNA
<213>  Artificial

<220>
<223>  Antisense oligomer

<400>  15
ccatggcagg gggcaacgtc ggggcaccca tgccccgacg ccgccccctg ccaacgggtc       60

ccgcgattca aatctggcgg aagctta                                          87

<210>  16
<211>  79
<212>  DNA
<213>  Artificial

<220>
<223>  Sense oligomer

<400>  16
aagcttggcc gcagctcccc ggcggaggca agaggtggtt gggggggacc atggctgacg      60

ttttcccggg caacatgta                                                  79


<210>  17
<211>  79
<212>  DNA
<213>  Artificial

<220>
<223>  Antisense oligomer

<400>  17
acatgttgcc cgggaaaacg tcagccatgg tcccccccaa ccacctcttg cctccgccgg      60

ggagctgcgg ccaagctta                                                  79


<210>  18
<211>  81
<212>  DNA
<213>  Artificial

<220>
<223>  Sense oligomer

<400>  18
aagcttggcc gcagctcccc ggcggaggcg tcagaggtgg ttggggggga ccatggctga      60

cgttttcccg ggcaacatgt a                                               81


<210>  19
<211>  80
<212>  DNA
<213>  Artificial

<220>
<223>  Antisense oligomer

<400>  19
acatgttgcc cgggaaaacg tcagccatgg tcccccccaa ccacctctga cgcctccgcc      60

ggggagctgc ggccaagctt                                                 80


<210>  20
<211>  81
<212>  DNA
<213>  Artificial

<220>
<223>  Sense oligomer

<400>  20
aagcttggcc gcagctcccc ggcggaggcg tcagccgtgg ttggggggga ccatggctga      60

cgttttcccg ggcaacatgt a                                               81

<210>  21
<211>  81
<212>  DNA
<213>  Artificial

<220>
<223>  Antisense oligomer

<400>  21
acatgttgcc cgggaaaacg tcagccatgg tcccccccaa ccacggctga cgcctccgcc      60

ggggagctgc ggccaagctt a                                                81


<210>  22
<211>  83
<212>  DNA
<213>  Artificial

<220>
<223>  Sense oligomer

<400>  22
aagctttgcc ttgaccagga cttgggactt tgcgaaagga tcgcggggcc cggagaggtg      60

ttggagagca caatggccat gga                                              83


<210>  23
<211>  83
<212>  DNA
<213>  Artificial

<220>
<223>  Antisense oligomer

<400>  23
ccatggccat tgtgctctcc aacacctctc cgggccccgc gatcctttcg caaagtccca      60

agtcctggtc aaggcaaagc tta                                              83


<210>  24
<211>  87
<212>  DNA
<213>  Artificial

<220>
<223>  Sense oligomer

<400>  24
aagctttgcc ttgaccagga cttctctggg actttgcgaa aggatcgcgg ggcccggaga      60

ggtgttggag agcacaatgg ccatgga                                          87


<210>  25
<211>  86
<212>  DNA
<213>  Artificial

<220>
<223>  Antisense oligomer

<400>  25
ccatggccat tgtgctctcc aacacctctc cgggccccgc gatcctttcg caaagtccca      60

gagaagtcct ggtcaaggca aagctt                                          86


<210>  26
<211>  87
<212>  DNA
<213>  Artificial

<220>
<223>  Sense oligomer

<400>  26
ggaagcttcg caggaggccg ccgcccgggc gcagagggca gccggtgggg aggcatgccg      60

ccgctcctgg cgcccctgcc catggct                                          87


<210>  27
<211>  87
<212>  DNA
<213>  Artificial

<220>
<223>  Antisense oligomer

<400>  27
agccatgggc aggggcgcca ggagcggcgg catgcctccc caccggctgc cctctgcgcc      60

cgggcggcgg cctcctgcga agcttcc                                          87


<210>  28
<211>  85
<212>  DNA
<213>  Artificial

<220>
<223>  Sense oligomer

<400>  28
ggaagcttcg caggaggccg ccgcccgggc gcagagggca gccaggggcg gcatgccgcc      60

gctcctggcg cccctgccca tggct                                            85


<210>  29
<211>  85
<212>  DNA
<213>  Artificial

<220>
<223>  Antisense oligomer

<400>  29
agccatgggc aggggcgcca ggagcggcgg catgccgccc ctggctgccc tctgcgcccg      60

ggcggcggcc tcctgcgaag cttcc                                            85


<210>  30
<211>  99
<212>  DNA
<213>  Artificial

<220>
<223>  Sense oligomer

<400>  30
ggaagcttgg cgggggaggc tctctaaggc ctccgcctct gcctctcccg cccccttacc      60

cgccccggag cgggaagcgg cggaggctcc gccatggcc                             99


<210>  31
<211>  99
<212>  DNA
<213>  Artificial

<220>
<223>  Antisense oligomer

<400>  31
ggccatggcg gagcctccgc cgcttcccgc tccggggcgg gtaagggggc gggagaggca      60

gaggcggagg ccttagagag cctcccccgc caagcttcc                             99


<210>  32
<211>  95
<212>  DNA
<213>  Artificial

<220>
<223>  Sense oligomer

<400>  32
ggaagcttgg cgggggaggc aaaggcctcc gcctctgcct ctcccgcccc cttacccgcc      60

ccggagcggg aagcggcgga ggctccgcca tggcc                                 95


<210>  33
<211>  95
<212>  DNA
<213>  Artificial

<220>
<223>  Antisense oligomer

<400>  33
ggccatggcg gagcctccgc cgcttcccgc tccggggcgg gtaagggggc gggagaggca      60

gaggcggagg cctttgcctc ccccgccaag cttcc                                 95


<210>  34
<211>  95
<212>  DNA
<213>  Artificial

<220>
<223>  Sense oligomer

<400>  34
ggaagcttgg cgggggaggc agaggcctcc gcctctgcct ctcccgcccc cttacccgcc      60

ccggagcggg aagcggcgga ggctccgcca tggcc                                 95


<210>  35
<211>  95
<212>  DNA
<213>  Artificial

<220>

<223>  Antisense oligomer

<400>  35
ggccatggcg gagcctccgc cgcttcccgc tccggggcgg gtaagggggc gggagaggca      60

gaggcggagg cctctgcctc ccccgccaag cttcc                                 95


<210>  36
<211>  95
<212>  DNA
<213>  Artificial

<220>
<223>  Sense oligomer

<400>  36
ggaagcttgg cgggggaggc agaggcctcc gcctctgcct cccccgcccc cttacccgcc      60

ccggagcggg aagcggcgga ggctccgcca tggcc                                 95


<210>  37
<211>  95
<212>  DNA
<213>  Artificial

<220>
<223>  Antisense oligomer

<400>  37
ggccatggcg gagcctccgc cgcttcccgc tccggggcgg gtaagggggc gggggaggca      60

gaggcggagg cctctgcctc ccccgccaag cttcc                                 95


<210>  38
<211>  53
<212>  RNA
<213>  Homo sapiens

<400>  38
cccggcggag gcaagaggug guuggggggg accauggcug acguuuuccc ggg             53


<210>  39
<211>  40
<212>  RNA
<213>  Homo sapiens

<400>  39
acuugggacu uugcgaaagg aucgcggggc ccggagaggu                            40


<210>  40
<211>  48
<212>  RNA
<213>  Homo sapiens

<400>  40
gcagagggca gccggugggg aggcaugccg cccuccuggc gccccugc                   48


<210>  41
<211>  44
<212>  RNA
<213>  Homo sapiens

<400>  41
ggcgggggag gcucucuaag gccuccgccu cugccucucc cgcc                    44


<210>  42
<211>  36
<212>  RNA
<213>  Artificial

<220>
<223>  FRET probe

<400>  42
agcggcggcg gcaugggugc cccgacguug cuaaaa                             36


<210>  43
<211>  38
<212>  RNA
<213>  Artificial

<220>
<223>  FRET probe

<400>  43
gugauuauuu auuauuuauu uauuauuuau uuauuuag                           38


<210>  44
<211>  42
<212>  RNA
<213>  Artificial

<220>
<223>  FRET probe

<400>  44
uccguccccc gcccgccgcg ccaugccugu ggccggcucg ga                      42


<210>  45
<211>  38
<212>  RNA
<213>  Artificial

<220>
<223>  FRET probe

<400>  45
aggcaagagg ugguuggggg ggaccauggc ugacguuu                           38


<210>  46
<211>  32
<212>  DNA
<213>  Artificial

<220>
<223>  Sense oligomer

<400>  46
gctagctaat acgactcact ataggaagct ta                                 32


<210>  47
<211>  32
<212>  DNA
<213>  Artificial

<220>
<223>  Antisense oligomer

<400>  47
aagcttccta tagtgagtcg tattagctag ca                                    32

## Description

### Technical Field

The present invention relates to a screening method for compounds capable of regulating translation from particular mRNAs, particularly from the mRNAs of genes encoding proteins whose expression are involved in the onset or progression of particular diseases, particularly for compounds having properties advantageous for pharmaceuticals, like lipophilic low-molecular compounds, a selection method for base sequences that serve as the target sites of mRNAs targeted by the compounds, and a tool therefor, particularly computer software and the like.

### Background Art

Target molecules in ongoing pharmaceutical developments are mostly proteins. Pharmaceuticals are being developed with the expectation for a particular drug effect by binding to a particular protein. On the other hand, a protein in vivo is synthesized (translated) on the basis of genetic information written in mRNA, which is synthesized from DNA (transcribed). Hence, there should be cases wherein the same effects as that obtained by regulating the function of proteins are obtained by regulating the translation from particular mRNAs.

Such idea of obtaining drug effects by regulating the translation from mRNAs exactly represents the concept seen in the development of antisense pharmaceuticals and RNAi pharmaceuticals, and has surely been successful to some extent. For example, Vitravene, developed by ISIS Pharmaceuticals Company, is already available in the market. Also, pharmaceuticals using RNAi have recently been drawing increased attention. Such pharmaceuticals are difficult to handle because much remains unknown; nevertheless, this year Acuity Pharmaceuticals filed an application for proceeding to Phase I for an RNAi pharmaceutical. However, although the concept of drug discovery research that targets a particular mRNA is wonderful per se, all drugs that have been developed to date are nucleic acid (or derivative thereof) pharmaceuticals. Because nucleic acid pharmaceuticals are not only problematic with respect to mass-synthesis, disposition, and metabolic stability, but are also in violation of Lipinski's rule, they are by far ideal forms of pharmaceuticals. After all, low-molecular compounds are desirable for pharmaceuticals.

On the other hand, RNA molecules can assume higher order structures capable of recognizing low-molecular compounds. A large number of low-molecular compound-binding motifs of RNA that selectively recognize particular low-molecular compounds (aptamers; motifs developed to bind to particular low-molecular compounds by combinatorial techniques called SELEX or in vitro selection) have been reported to date. In fact, it has been found that in nature certain RNAs can bind to naturally occurring or artificial low-molecular compounds without the involvement of any proteins. Low-molecular pharmaceuticals for such RNAs have long been developed using compounds discovered in naturally occurring substances. For example, aminoglycoside antibiotics, represented by geneticin, tobramycin and kanamycin, bind to rRNA to inhibit the function of ribosome. The binding mode thereof has already been confirmed by crystallographic analysis and the like. Taking the hints from the results, and to avoid serious adverse reactions observed in the same series of antibiotics, Abbott Company performed low-molecular compound drug discovery that targeted the rRNA. Thus, Abbott succeeded in obtaining RNA binding low-molecular compounds by high-throughput screening using NMR (Yu, L. et al., J. Am. Chem. Soc., 125, 4444-4450 (2003)). There have also been reported a number of attempts for drug discovery that targets viral RNA (Swayze, E.E. and Griffey, R.H., Expert Opin. Ther. Pat., 12, 1367-1374 (2002)).

However, such attempts have been directed only to RNAs considered to "have a structure and function". A very big problem of this approach resides in that because molecular mechanisms utilizing RNAs having functions are usually ubiquitously present in the human body, such compounds targeting such RNAs cannot become pharmaceuticals for treating particular diseases. Hence, such compounds can be developed only as antibiotics utilizing the species difference. It is also problematic that the low-molecular compounds thus obtained behave remarkably differently from existing protein-targeting pharmaceuticals so that it is difficult to take appropriate measures in synthesis.

Since it has recently been realized that drug discovery is possible for RNAs that "have a (function and) structure" as described above, drug discovery research into structures on mRNAs has been conducted. In the 21st century, gene expression regulatory mechanisms based on interactions between mRNAs and low-molecular compounds (riboswitch) has been observed experimentally in prokaryotic organisms (WO 2004/027035). To date, it has been reported that such mechanisms are present for vitamin B12, thiamin pyrophosphate, FMN, S-adenosylmethionine, guanine, adenine, L-lysine, and glucosamine-6-phosphate. For example, in B. subtilis, it is considered that a minimum of 2% (putatively not less than 10%) of the total gene expression thereof is under the control of the riboswitch mode. The xpt-pbuX mRNA of B. subtilis recognizes guanine, which has a molecular weight of as low as 151.13, at the nM level (Kd < 5 nM). Moreover, LysC mRNA selectively recognizes L-lysine (molecular weight 146.19; Kd = 1 µM), though it is not even a derivative of RNA. These low-molecular substances are capable of controlling gene expression by being coordinated to mRNAs to alter the secondary and tertiary structures of the mRNAs. In the mechanism for glucosamine-6-phosphate, the mRNA exhibits ribozyme-like self-cleavage to irreversibly inactivate the mRNA. For these mechanisms, attempts for drug discovery with modification of natural products are being made.

On the other hand, it has been reported that in an artificial system comprising aptamer motifs experimentally introduced to mRNAs, protein translation in eukaryotic organisms can be suppressed using low-molecular compounds that bind to the motifs. These facts can be viewed as suggesting feasibility of drug discovery for low-molecular pharmaceuticals that target mRNA molecules.

Furthermore, in 2002 and 2004, drug discovery for structures on natural mRNAs was reported. The Alzheimer's amyloid precursor protein (APP) gene is the responsible gene for Alzheimer's disease, and it had been known that a somewhat stable local higher order structure (motif) is present on the mRNA thereof. Hence, screening for low-molecular compounds was performed using a reporter gene incorporating the motif, and hit compounds that suppress the translation level by not less than 95% were obtained (Rogers, J.T. et al., J. Mol. Neurosci., 19, 77-82 (2002)). VEGF, a cancer-related gene, had also been known to have an IRES-like higher order structure. Screening for low-molecular compounds was performed using a reporter gene incorporating the motif, and hit compound were obtained (Liangxian, C. et al., EORTC-AACR, poster # 144 (2004)).

Truly desired is a drug discovery with low-molecular compounds. However, the number of mRNAs "that were reported to have structures" expected to recognize low-molecular substance is limited, so that subject diseases are highly limited. Furthermore, low-molecular compounds that bind to RNAs found by conventional screening for such numerically limited structures are mostly compounds of excess water solubility, including aminoglycoside antibiotics, or compounds that intercalate to nucleic acid strands nonselectively. Because such compounds are poor in disposition, and also because the possibility of binding to other unforeseeable RNAs cannot be ruled out, they may cause serious adverse reactions. In addition, those compounds are difficult to be modified chemically to improve their disposition and selectivity for their targets, and hence it cannot be said that they have been successful from the viewpoint of pharmaceutical development (Swayze, E.E. and Griffey, R.H. (2002), described above).

What is demanded is to freely regulate the translation of particular proteins using low-molecular compounds that bind only to desired motifs on particular desired mRNAs. To achieve this, it is necessary to find the motifs that allow conventional type low-molecular pharmaceuticals to bind selectively onto the targeted mRNAs to regulate the translation thereof.

### Disclosure of the Invention

It is an object of the present invention to provide a means for searching for local secondary structures (motifs) on targeted mRNAs that allow some low-molecular compounds to bind selectively to the mRNAs, and to provide low-molecular pharmaceuticals that exhibit drug effects by screening for the low-molecular compounds that binds only to the motifs found using the means in a real system, and regulating the translation of desired mRNAs.

The present inventors conceptualized that any single-stranded RNAs can be structured spontaneously in accomplishing the above-described object. The reality is that the RNA is not sufficiently stable to have a function in vivo or just does not exhibit the function (because such in vivo structures do not appear as outputs of any molecular biological phenomena, they have not drawn any attention in molecular biology, and therefore, none have been aware of whether or not they have structures); because RNAs are readily interacting biopolymers, they are considered to always have a structure. The structure, if any, of RNAs can be stabilized by low-molecular substances.

Nowadays human cDNA libraries are available, including a database of Celera Company and Japan's H-invitational, and individual sequence data of mRNAs are easy to obtain. Hence, with the aid of some RNA secondary structure prediction programs, the higher order structure of mRNA can be predicted from the sequence data thereof.

However, with existing RNA secondary structure prediction programs, represented by mfold (GCG Software; see Proc. Natl. Acad. Sci. USA, 86: 7706-10 (1989)), a large amount of data cannot be processed in a significant time. Furthermore, when long (for example, 300 bases or more) RNA strands are handled, the accuracy decreases remarkably. This is because mfold does not predict the higher order structure according to a certain physical property of the RNA strand much, but rather simply performs pattern matching. That is, remote stems are often forcibly formed, with the entropic disadvantage due to the pairing of remote intramolecular RNA strands being virtually neglected. Even if such remote pairing occurs in an in vitro system, it is unlikely that the pairing occurs in vivo. Of course, such a pattern matching technique is fully significant in estimating local short structures (100 bases or less) (mainly because the loss of entropy due to pairing is small in the case of prediction of the local structures). However, mfold has many drawbacks, including the little significance of the use of mfold in handling local structures and the unsuitability of the outputting mode for comprehensive search. This is because mfold is designed to specialize in secondary structure prediction, so that structures judged by mfold to be invalid are not outputted. Because the reliability of mfold is not 100%, there can be failures in outputs from mfold. It is essential to establish a new comprehensive search "technique".

Hence, the present inventors searched data on natural mRNA sequences for secondary structures with speculation by pattern matching, comprehensively searched for portions likely to allow development of low-molecular pharmaceuticals, and actually conducted drug discovery against the portions. That is, the present inventors have developed a "technique" program that comprehensively searches a human cDNA database for higher order structures to which conventional type lipophilic low-molecular compounds can bind (named "multisl.pl" program). This program is capable of comprehensively finding out all motifs having a desired character from a large amount of data using an algorithm for deformation dynamic programming.

Specifically, by selecting stem-loop structures as the desired motifs, inputting parameters of particular stem-loop structures, executing the above-described program to search for and extract data on mRNAs with a sequence capable of assuming the structure in the molecule thereof from a data set comprising a large amount of mRNA information recorded, selecting particular mRNAs (e.g., a group of mRNAs that encodes Survivin) and particular target sequences in the mRNAs from among the mRNAs thus extracted with subject diseases (e.g., cancer), the exact position of the stem-loop structures on the molecules (e.g., vicinity of start codon) and the like as an index, further searching for the specificity of the target sequence selected from the mRNA data set by executing the above-described program, and confirming that the target sequence is not present in the region involved in the regulation of the translation of other mRNAs, the present inventors succeeded in obtaining a target sequence to which low-molecular compounds can bind specifically.

The present inventors also succeeded in constructing an evaluation system for screening for compounds capable of stabilizing the stem-loop structures of RNA strands comprising particular sequences obtained as described above to regulate the translation of mRNAs having the sequences, by bringing the RNA strands into contact with test compounds, and measuring and comparing changes in the stability of the stem-loop structures of the RNA strands in the presence and absence of the test compounds.

The present inventors conducted further investigations based on these findings, and developed the present invention.

Accordingly, the present invention provides:
[1] a method of selecting a sequence capable of assuming a particular local secondary structure in a particular mRNA for screening for a compound capable of regulating the translation of the mRNA, which comprises the following steps:
   (1) a step wherein an mRNA search means searches an mRNA data set inputted and extracts mRNA(s) having a sequence, in the molecule, capable of assuming a local secondary structure capable of regulating the translation of the mRNA(s),
   (2) a step for selecting a particular mRNA from among the one or more mRNAs extracted in (1) above,
   (3) a step for selecting a particular sequence from among one or more sequences capable of assuming a local secondary structure in the particular mRNA selected in (2) above,
   (4) a step wherein a specificity confirmation means searches an mRNA data set inputted and confirms that a partial structure capable of interacting with the compound in the particular sequence selected in (3) above is not present in the region involved in the regulation of the translation of other mRNA, or even if present, is incapable of regulating the translation;
[2] the method described in [1] above, wherein the local secondary structure is a stem-loop structure;
[3] the method described in [2] above, wherein the stem-loop structure has two or more stem portions and has a bubble portion not having complementarity between the stems;
[4] the method described in [2] or [3] above, wherein the compound that regulates the translation of a particular mRNA is capable of interacting with the loop portion or bubble portion of the stem-loop structure;
[5] a method of selecting a sequence capable of assuming a particular local secondary structure in a particular mRNA for screening for a compound capable of suppressing the translation of the mRNA, which comprises the following steps:
   (1) a step wherein an mRNA search means searches an mRNA data set inputted and extracts mRNA(s) having a sequence, in the molecule, capable of assuming a local secondary structure capable of suppressing the translation of the mRNA(s),
   (2) a step for selecting a particular mRNA from among the one or more mRNAs extracted in (1) above,
   (3) a step for selecting a particular sequence from among one or more sequence capable of assuming a local secondary structure in the particular mRNA selected in (2) above,
   (4) a step wherein a specificity confirmation means searches an mRNA data set inputted and confirms that a partial structure capable of interacting with the compound in the particular sequence selected in (3) above is not present in the region involved in the regulation of the translation of other mRNA, or even if present, is incapable of suppressing the translation
[6] the method described in [5] above, wherein the sequence capable of assuming a particular local secondary structure is present in a region consisting of a 5' non-translational region and a coding region;
[7] the method described in [6] above, wherein the sequence capable of assuming a particular local secondary structure is present in a region consisting of a translation initiation point and the vicinity thereof;
[8] the method described in [1] above, wherein regulating the translation of the particular mRNA is effective in the prophylaxis/treatment of one or more diseases;
[9] a method of selecting a sequence capable of assuming a particular local secondary structure in a particular mRNA, for screening for a compound capable of preventing/treating a particular disease, which comprises the following steps:
   (1) a step for selecting one or more mRNAs whose regulation of translation can result in the prevention/treatment of the disease,
   (2) a step wherein an mRNA search means searches a data set of the one or more mRNAs selected in (1) above, and extracts mRNA(s) having a sequence, in the molecule, capable of assuming a local secondary structure capable of regulating the translation of the mRNA(s),
   (3) a step for selecting a particular mRNA from among the one or more mRNAs extracted in (2) above,
   (4) a step for selecting a particular sequence from among one or more sequences capable of assuming a local secondary structure in the particular mRNA selected in (3) above,
   (5) a step wherein a specificity confirmation means searches an mRNA data set inputted and confirms that a partial structure capable of interacting with the compound in the particular sequence selected in (4) above is not present in the region involved in the translation of other mRNA, or even if present, is incapable of regulating the translation;
[10] a screening method for a compound that interacts with a sequence capable of assuming a particular local secondary structure selected by the method described in [1] above to regulate the translation of a particular mRNA, which comprises a step for bringing an RNA strand comprising the sequence and a test compound into contact with each other, and measuring changes in the stability of the secondary structure of the RNA strand;
[11] a screening method for a compound that regulates the translation of a particular mRNA, which comprises the following steps:
   (1) a step for searching mRNA(s) having a sequence, in the molecule, capable of assuming a local secondary structure capable of regulating the translation of the mRNA(s),
   (2) a step for bringing an RNA strand comprising a sequence capable of assuming a particular local secondary structure in the particular mRNA extracted in (1) above and a test compound into contact with each other, and measuring changes in the stability of the secondary structure of the RNA strand;
[12] a screening method for a compound that suppresses the translation of a particular mRNA, which comprises the following steps:
   (1) a step for searching mRNA(s) having a sequence, in the molecule, capable of assuming a local secondary structure capable of suppressing the translation of the mRNA(s),
   (2) a step for bringing an RNA strand comprising a sequence capable of assuming a particular local secondary structure in the particular mRNA extracted in (1) above and a test compound into contact with each other, and measuring changes in the stability of the secondary structure of the RNA strand;
[13] a screening method for a compound capable of preventing/treating a particular disease, which comprises the following steps:
   (1) a step for selecting one or more mRNAs whose regulation of translation can result in the prevention/treatment of the disease,
   (2) a step for searching mRNA(s) having a sequence, in the molecule, capable of assuming a local secondary structure capable of regulating the translation of the mRNA(s) from among the mRNAs selected in (1) above,
   (3) a step for selecting a particular sequence from among sequences capable of assuming a local secondary structure in the particular mRNA extracted in (2) above,
   (4) a step for confirming that a partial structure capable of interacting with the compound in the particular sequence selected in (3) above is not present in the region involved in the regulation of the translation of other mRNA, or even if present, is incapable of regulating the translation,
   (5) a step for bringing an RNA strand comprising a particular sequence confirmed to be not present in the region involved in the regulation of the translation of other mRNA in (4) above and a test compound into contact with each other, and measuring changes in the stability of the secondary structure of the RNA strand;
[14] a method of predicting a compound that exhibits an adverse reaction by regulating the translation of mRNA, which comprises the following steps:
   (1) a step for searching mRNA(s) having a sequence, in the molecule, capable of assuming a local secondary structure capable of regulating the translation of the mRNA(s),
   (2) a step for bringing an RNA strand comprising a sequence capable of assuming a local secondary structure in the mRNA extracted in (1) above and a test compound into contact with each other, and detecting the presence or absence of an RNA strand whose secondary structure is stabilized in the presence of the compound;
[15] a nucleic acid substantially consisting of the same or substantially the same base sequence as the base sequence shown by any of SEQ ID NO:1 and 38 to 41;
[16] the nucleic acid described in [15] above, wherein one or more base pairs can be formed in a bubble by introducing a mutation;
[17] an expression vector comprising an expression cassette having the nucleic acid described in [15] or [16] above inserted between a promoter and a reporter gene;
[18] a transformant obtained by transforming a host cell with the expression vector described in [17] above;
[19] a kit for screening for a compound capable of suppressing the translation of an mRNA that encodes a protein selected from the group consisting of Survivin, PKCα, A20, NOTCH1 and NUP, which comprises the nucleic acid described in [15] above, the expression vector described in [17] above or the transformant described in [18] above;
[20] a prophylactic/therapeutic agent for cancer comprising a compound capable of suppressing the translation from an mRNA that encodes a protein selected from the group consisting of Survivin, PKCα, A20, NOTCH1 and NUP, wherein the translation is suppressed by the compound that causes the mRNA to form a local secondary structure in the molecule of the mRNA; and
[21] the agent described in [20] above, wherein the local secondary structure is formed in the base sequence shown by any of SEQ ID NO:1 and 38 to 41; and the like.

According to the method of the present invention, unlike conventional mRNA targeting pharmaceuticals, it is possible to screen for a low-molecular compound more advantageous in terms of physical properties of pharmaceuticals, such as molecular weight, water-solubility, lipophilicity, and hydrogen binding score. The method is also applicable to drug discovery for low-molecular pharmaceuticals that target any mRNA as a rule, and RNAs having other structure that specifically acts in vivo (for example, micro-RNA (miRNA) and the like). Furthermore, unlike drug discovery that targets proteins, which necessitates newly constructing an HTS system according to subject molecule, all the steps to the obtainment of a lead compound are the same irrespective of subject disease, so that time saving and streamlining of drug discovery scheme and cost reduction are possible.

### Brief Description of the Drawings

Figure 1 shows the structures of stem-loop A (Figure 1A) and stem-loop B (Figure 1B).
Figure 2 schematically shows the differences between stem-loop A and stem-loop B from the viewpoint of the concept of dynamic range.
Figure 3 shows a system configuration of an apparatus for executing the sequence selection method of the present invention (Figure 3A) and a flow of the processing executed by the apparatus (Figure 3B).
Figure 4 shows breakdown of the action sites of antisense pharmaceuticals automatically acquired from patent information (Figure 4A) and breakdown of the action sites of antisense pharmaceuticals confirmed by comprehensive automatic search processing from a cDNA database (Figure 4B).
Figure 5 shows the stem-loop B that appears in hCT18953.
Figure 6 shows EGFP translation levels (Figure 6A left), EGFP mRNA contents (Figure 6A right), luciferase translation levels (Figure 6B left) and luciferase mRNA contents (Figure 6B right) after introduction of plasmids (pEGFP/SL31/IC, pEGFP/SL31/IS, pEGFP/SL31/IT).

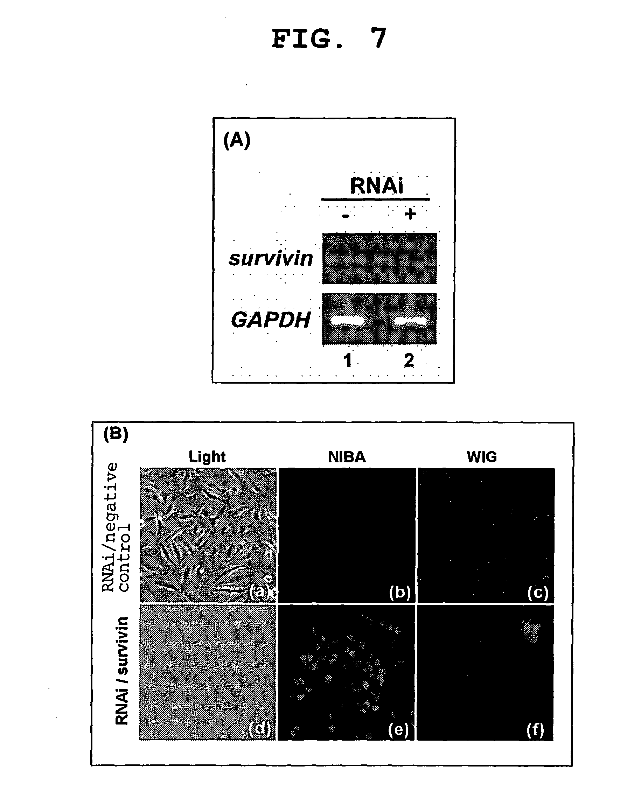

Figure 7 shows the results of RT-PCR for the Survivin mRNA content and GAPDH mRNA content contained in the total RNA extracted from HeLa cells incorporating control siRNA (lane 1) or siRNA for Survivin (lane 2), cultured for 48 hours (Figure 7A), and detection of mitochondrial membrane collapse in the cells (negative control incorporated control siRNA; Survivin incorporated siRNA for Survivin) (Figure 7B). In Figure 7B, Light indicates an observation of cells in a bright-field, NIBA indicates an observation of fluorescence with an NIBA filter (excitation wavelength 470-490 nm, absorption wavelength 515-550 nm), and WIG indicates an observation of fluorescence with an WIG filter (excitation wavelength 520-550 nm, absorption wavelength 580 nm).
Figure 8A schematically shows the secondary structures of Suv SL1, Suv SL1m1 and Suv SL1m2. Figure 8B shows the 5'UTR structure of the pGL3/SL1 reporter plasmid. Figure 8C shows the concept of an experiment wherein pGL3/Suv SL1, pGL3/Suv SL1m1, or pGL3/Suv SL1m2 is introduced to CHO cells simultaneously with pEGFP-N1, and luciferase and EGFP translation levels are measured.
Figure 9 shows luciferase translation levels (Figure 9A left), luciferase mRNA contents (Figure 9A right), EGFP translation levels (Figure 9B left) and EGFP mRNA contents (Figure 9B right) in cells transfected with pGL3/SL1, pGL/SL1m1, or pGL/SL1m2.
Figure 10 shows luciferase translation levels in cells transfected with pGL3/PKC SL1, pGL/PKC SL1m1, pGL/PKC SL1m2, pGL3/A20 SL1, or pGL/A20 SL1m1.
Figure 11 shows luciferase translation levels (Figure 11A) and luciferase mRNA contents (Figure 11B) in cells transfected with pGL3/NOTCH1 SL1 or pGL/ NOTCH1 SL1m1.
Figure 12 shows luciferase translation levels (Figure 12A) and luciferase mRNA contents (Figure 12B) in cells transfected with pGL3/NUP SL1, pGL/NUP SL1m1, pGL/NUP SL1m2, or pGL/NUP SL1m3.

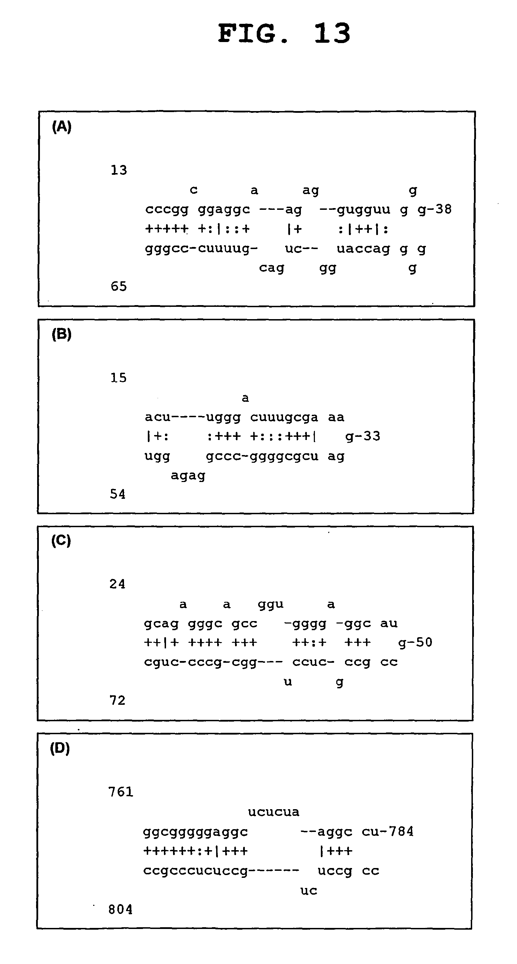

Figure 13 shows stem-loop B that appears in hCT20429 (Figure 13A), hCT7828 (Figure 13B), hCT1961915.1 (Figure 13C), and hCT2252375 (Figure 13D), respectively.
Figure 14 shows the concentration dependencies of the effects of kanamycin in stabilizing the secondary structures of Suv SL1 (■) and PKC SL1 (▲).

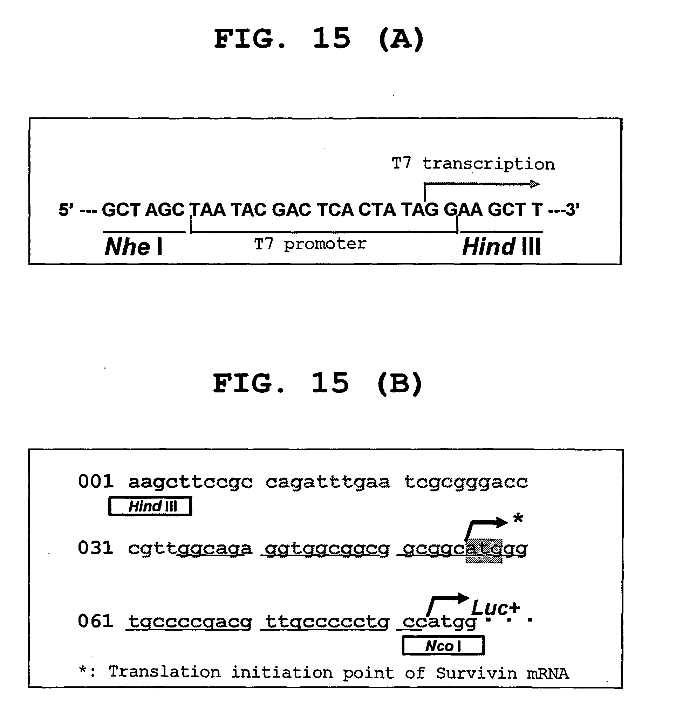

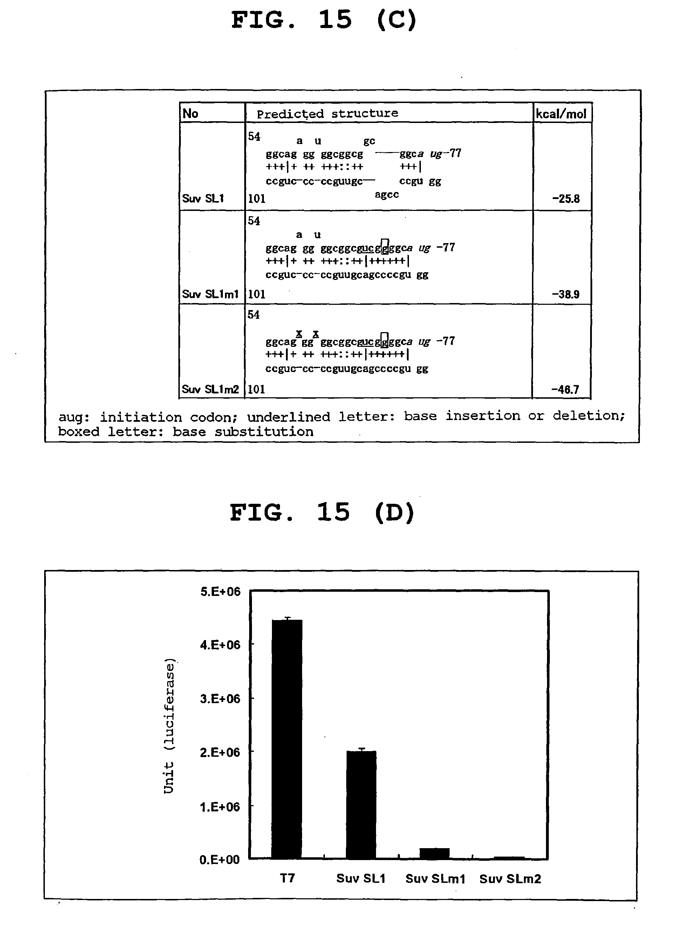

Figure 15 shows the T7 promoter sequence introduced to pGL3/Suv SL1/T7 (Figure 15A), the 5'-terminal sequence of the transcription product of pGL3/Suv SL1/T7 (Figure 15B; the underlined portion indicates Suv SL1, * indicates the translation initiation point of Survivin mRNA, and Luc+ indicates the translation initiation point of luciferase), the secondary structures of Suv SL1, Suv SL1m1 and Suv SL1m2 (Figure 15C; aug indicates the initiation codon, the underlined portion indicates a nucleotide insertion or deletion, and each boxed letter indicates a nucleotide substitution), and the results of an in vitro translation experiment (Figure 15D), respectively.

### Best Mode for Embodying the Invention

The present invention provides a method of selecting a sequence capable of assuming a particular local secondary structure in a particular mRNA, for screening for a compound capable of regulating the translation of the mRNA (hereinafter sometimes abbreviated "the sequence selection method of the present invention"). The method is hereinafter described in detail with reference to the configuration of a program allowing a computer to execute the method.

The sequence selection method of the present invention (and the program to execute the method) has steps (1) to (4), as described in [1] above. The program to execute the method comprises commands for a computer to serve at least as an mRNA search means in step (1) and a specificity confirmation means in step (4).

As used herein, "an mRNA data set" is referred to as a set of data on an optionally chosen number of optionally chosen mRNAs and pre-mRNAs, comprising at least the name thereof (including some indication enabling the identification of the mRNAs, such as ID numbers) and base sequence information. The base sequence information may be sequence information on the mRNAs per se, or sequence information on corresponding cDNAs. This data set concerns with the mRNAs that constitute the same, and may further comprise supplementary information on, for example, the positions of initiation and termination codons, the names and function outlines of the proteins encoded, positions on the genome, number and positions of splicing points, number and names of splicing variants, and the like. These information may be converted from RNA to DNA or the reverse whenever necessary, or the information regarding the order of arrangement of base sequences may be reverted.

The number of mRNAs contained in this data set is not subject to limitation, and the data set may consist of, for example, all virtual mRNAs obtained from genome information and the like (for example, in the case of humans, about 40000 to about 70000); for example, when it is intended to search for a low-molecular pharmaceutical that targets an mRNA involved in a disease, the data set may consist of mRNAs known to be involved in a disease (for example, target mRNAs whose antisense nucleic acid or siRNA is already in the clinical or preclinical stage as a pharmaceutical; for example, mRNAs corresponding to the cDNAs shown in Table 1-1 to Table 1-11 and the like). Alternatively, when it is intended to search for a low-molecular pharmaceutical that targets an mRNA involved in a particular disease, the data set may consist of mRNAs known to be involved in the disease (for example, mRNAs corresponding to the cDNAs shown in Table 2-1 to Table 2-3 in Example 1 below). The data set may also be the entire genome.

As examples of the comprehensive human cDNA data set, "CHGD_transcripts_R27f.fasta", which is supplied by Celera Genomics, "nuc_all.fa", which is supplied by H-invitational, and the like can be utilized. As examples of the data set of mRNAs known to be involved in an optionally chosen or particular disease, a subdata set obtained by extracting a target mRNA for an antisense pharmaceutical candidate nucleic acid for an optionally chosen or particular disease, from the above-described comprehensive cDNA data set, obtained from the antisense pharmaceutical pipeline information of Isis Pharmaceuticals Company, and the like can be mentioned.

Alternatively, a particular mRNA may be selected as a target in advance. In this case, after step (1), step (2) may be skipped to step (3).

**Table 1-1**

| entry# | stage | names | therapeutic area | misc | H-inv | Celera |
|---|---|---|---|---|---|---|
| 192214 | Preclinical | ISIS-1082 | Antisense Therapy; Antiviral Drugs | | NO | NO |
| 196023 | Phase II | ISIS-2105; I-2105; IP-2105;Afovirsen sodium | Antisense Therapy; Antiviral Drugs | Oligonucleotides | NO | NO |
| 196030 | Launched-1998 | ISIS-2922;Fomivirsen sodium;Vitraven | Anti-Cytomegalovirus Drugs | DNA Polymerase Inhibitors | NO | NO |
| 203661 | Phase II | GEM-9; Gene expression modulator 91; Trecovirsen1 | Anti-HIV Agents; Antisense Therapy | Oligonucleotides | NO | NO |
| 205912 | Phase III | ISIS-2302;Alicaforse n sodium | Antiarthritic Drugs; Antipsoriatics: Antisense Therapy; Inflammatory Bowel Disease, Agents for | ICAM1 Expression Inhibitors; Oligonucleotides | BC015969 | hCT2336738;hCT19627 |
| 209641 | Biological Testing | | Antisense Therapy; Oncolytic Drugs | | BC007585 | hCT29713 |
| 213106 | Biological Testing | | Antisense Therapy; Restenosis Treatment of | | BC002466:BC007514 | hCT2257033;hCT2257035;hCT20300 |
| 213109 | Biological Testing | | Antisense Therapy: Restenosis Treatment of | | B0000141;BC000917 | hCT2348021;hCT6947;hCT2297650;hCT2297651 |
| 21313 | Biological Testing | | Antisense Therapy; Restenosis Treatment of | | NO | NO |
| 215350 | Biological Testing | | Restenosis Treatment of | | NO | NO |
| 215351 | Biological Testing | | Restenosis Treatment of | | NO | NO |
| 215728 | Biological Testing | | Antisense Therapy | | BC015969 | hCT19627 |
| 215918 | Preclinical | ISIS-3466 | Antisense Therapy; Oncolytic Drugs | NOLI Expression Inhibitors; Oligonucleotides | AK025002 | NO |
| 217966 | Preclinical | ISIS-3082;IP-3082 | Antisense Therapy; Immunomodulators | Non-Steroidal Antiinflammatory | NO | NO |
| 219302 | Phase I/lI | AR-177; T30177; | Anti-HIV Agents; Antisense Therapy | HIV Integrase Inhibitors | NO | hCT8432 |
| 221576 | Biological Testing | ISIS-1570 | Antisense Therapy: Oncolytic Drugs | | BC015969 | hCT19627 |
| 221577 | Biological Testing | ISIS-1939 | Antisense Therapy. Oncolytic Drugs | | BC015969 | hCT2336738;hCT19627 |
| 223825 | Phase II | ISIS-5132; CGP-69846A: ODN-698 | Antisense Therapy: Breast Cancer Therapy; Ovarian Cancer Therapy; Solid Tumors Therapy | Oligonucleotides: RAFI Expression Inhibitors | BC018119 | hCT19462;hCT2300428 |

**Table 1-2**

| | | | | | | |
|---|---|---|---|---|---|---|
| 224350 | Phase III | ISI-641: LY-900003;ISIS-3521; CGP-64128A: Aprinocarsen sodium: Affinitak; Affinitac | I Antisense Therapy; Breast Cancer Therapy; Non-Hodgkin's Lymphoma Therapy: Non-Small Cell Lung Cancer Therapy; Ovarian | Inhibitors of Signal Transduction Pathways; Oligonucleotides; PKCA Expression Inhibitors | NO | hCT20429 |
| 226493 | Phase I | GPs-0193; Navin | Anti-HIV Agents; Antisense Therapy | | NO | NO |
| 230388 | Preregistered | G-3139; NSC-683428; Augmerosen; Oblimersen sodium; Genasense | Antisense Therapy: Breast Cancer Therapy-, Colorectal Cancer Therapy: Gastric Cancer Therapy: Leukemia Therapy: Liver Cancer Therapy; Lymphocytic Leukemia Therapy; Lymphoma Therapy; Melanoma Therapy: Multiple Myeloma Therapy; Myeloid Leukemia Therapy; Non-Hodgkin's Lymphoma Therapy; Non-Small Cell Lung Cancer Therapy; Oncolytic Drugs; Pancreatic Cancer Therapy; Prostate Cancer Therapy; Renal Cancer Therapy; Small Call Lung Cancer Therapy; Solid Tumors | Apoptosis Inducers; BCL2 Expression Inhibitors; Oligonucleotides | BC027258 | hCT2250746;hCT25293:hCT2250747:hCT2250748 |
| 235142 | Biological Testing | 707 | Antisense Therapy: Oncolytic Drugs | | NO | NO |
| 236390 | Phase II | GEM-132 | Anti-Cytomegalovirus Drugs; Antisense Therapy | | NO | NO |
| 236859 | Biological Testing | ISIS-7597 | Antisense Therapy: Multidrug Resistance | | NO | hCT1784923;hCT2290449;hCT2290445;hCT28464;hCT1784918;hCTI7 84920;hCT1784921;hCT1784922;hCT2290453;hCT2290444;hCT22904 52;hCT2290451;hCT2290441;hCT2290440 |
| 236916 | Phase I/II | LR-3280; BW-4003W94: TA-3280: INX-3280 | Antisense Therapy: Lymphoma Therapy; Oncolytic Drugs; Restenosis Treatment of, Solid Tumors Therapy | Inhibitors of Signal Transduction Pathways; MYC Expression Inhibitors; Oligonucleotides | BC000141;BC000917 | hCT2348021;hCT8947;hCT2297850;hCT2297651 |
| 238018 | Preclinical | PT-11061 | Antisense Therapy; Immunosuppressant | | NO | NO |
| 238464 | Biological Testing | | Antisense Therapy; Oncolytic Drugs | | NO | hCT2308850:hCT1798029 |
| 238717 | Biological Testing | | Antisense Therapy: Oncolytic Drugs | | AK097580 | hCT1954118:hCT19614;hCT1954120 |

**Table 1-3**

| | | | | | | |
|---|---|---|---|---|---|---|
| 240177 | Preclinical | F PT-9125: IP-1125;ISIS-9125 | Antisense Therapy; Immunosuppressant s: Inflammatory Bowel Disease. | Anti-ICAM-1 Agents; Oligonucleotides | NO | NO |
| 241116 | Biological Testing | | Antisense Therapy: Oncolytic Drugs | | NO | NO |
| 241117 | Biological Testing | | Antisense Therapy; Oncolytic Drugs | | NO | NO |
| 250519 | Preclinical | I HYB-101584 | Antisense Therapy; Oncolytic Drugs | | NO | NO |
| 252433 | Biological Testing | I CN-70 | Antisense Therapy; Oncolytic Drugs | IL8 Expression Inhibitors; Oligonucleotides | NO | hCT7408;hCT2327544 |
| 253298 | Biological Testing | I CN-133 | Antisense Therapy; Oncolytic Drugs | ILB Expression Inhibitors; Oligonucleotides | BC007790;BC013615 | HCT1952531;hCT2296169;hCT2296170;hCT2296173;hCT14045;hCT74 08:hCT2327544 |
| 253953 | Preclinical | ISIS-4015 | Anti-Herpes Simplex Virus Drugs; Antiviral | | NO | NO |
| 257201 | Biological Testing | | Antiviral Drugs | | NO | NO |
| 259651 | Preclinical | NIH-351; MP-351; 2-5A-RSV | Antisense Therapy; Antiviral Drugs | | NO | NO |
| 261639 | Biological Testing | | Antisense Therapy; Oncolytic Drugs | | NO | NO |
| 261658 | Discontinued | EPI-2010; Durason | Antiallergy/Antiasth matic Drugs; Antisense Therapy | ADORA1 Expression Inhibitors; Oligonucleotides | BC026340;AK095060 | hCT14222 |
| 270040 | Biological Testing | I ISIS-10639 | Antisense Therapy; Oncolytic Drugs | Oligonucleotides | BC004490;AK097379 | hCT13449 |
| 270877 | Preclinical | Y5 receptor antisense | Treatment of Nutritional | | NO | NO |
| 271044 | Biological Testing | HYB-0432 | Antisense Therapy; Oncolytic Drugs | Thymidylate Synthase Inhibitors | NO | hCT2274765;hCT2343416;hCT2274763;hCT2274764 |
| 271457 | Preclinical | | Antisense Therapy | | AK097580 | hCT1954118;hCT19614;hCT1954120 |
| 271811 | Phase I | ORI-1001; MBI-1121 | Anti-Papilloma Virus Drugs: Antisense Therapy | Oligonucleotides | NO | NO |
| 272787 | Phase I | INX-6295; INX-3281:INXC-6295 | Antisense Therapy; Lymphoma Therapy; Solid Tumors Therapy | Inhibitors of Signal Transduction Pathways; MYC Expression Inhibitors; Oligonucleotides | BC000141:BC000917 | hCT2348021;hCT6947;hCT2297650;hCT2297651 |
| 274087 | Biological Testing | ISIS-12539 | Antisense Therapy; Oncolytic Drugs | Oligonucleotides | NO | hCT2277439;hCT1950805:hCT14846:hCT2277438:hCT2277435:hCT22 77436;hCT2277431;hCT2277430;hCT2277429;hCT2277433;hCT22774 |
| 274505 | Preclinical | ISIS-16009 | Antisense Therapy; Oncolytic Drugs | BCL2L1 Expression Inhibitors; Oligonucleotides | BC019307 | hCT2313721;hCT2313724;hCT30057;hCT2313722 |
| 274769 | Preclinical | AS5-2H | Antisense Therapy; Oncolytic Drugs | MDM2 Expression Inhibitors; Oligonucleotides | NO | hCT2307014;hCT2307004;hCT2306995 |
| 275588 | Phase II | ISIS-14803 | Anti-Hepatitis C Virus Drugs; Antisense Therapy | Oligonucleotides | NO | NO |
| 277049 | Preclinical | ISIS-6547 | Anti-Hepatitis C Virus Drugs: | | NO | NO |

**Table 1-4**

| | | | | | | |
|---|---|---|---|---|---|---|
| 2777481 | Phase II | GTI-2040 | Antisense Therapy: Breast Cancer Therapy: Colorectal Cancer Therapy: Lymphoma Therapy: Myeloid Leukemia Therapy; Non-Small Cell Lung Cancer Therapy: Renal Cancer Therapy; Solid Tumors Therapy | Oligonucleotides: RRM2 Expression Inhibitors | BC001886;BC030154;AK092671 | hCT2343740;hCT2267461:hCT14945 |
| 2809001 | Phase I/II | I HYB-165; GEM-231; HYB-0165 | Antisense Therapy: Solid Tumors Therapy | Angiogenesis Inhibitors: Drugs Inhibiting Gene Expression; Oligonucleotides | AK097580 | hCT1954118;hCT19614;hCT1954120 |
| 281330 | Biological Testing | I SIS-16518 | Antisense Therapy; Oncolytic Drugs | Oligonucleotides | NO | hCT2307011;hCT2307014;hCT2307009;hCT2307005;hCT2307004:hCT 2306995;hCT2306997;hCT2307001;hCT2306992 |
| 281331 | Biological Testing | ISIS-16507 | Antisense Therapy; Oncolytic Drugs | Oligonucleotides | NO | hCT2307011;hCT2307014;hCT2307009;hCT2307005;hCT2307004;hCT 2307002;hCT2306995;hCT2306997;hCT2307001;hCT2306992 |
| 281332 | Biological Testing | ISIS-21927 | Antisense Therapy; Oncolytic Drugs | Oligonucleotides | NO | NO |
| 281352 | Biological Testing | ISIS-12884 | Antisense Therapy: Oncolytic Drugs | Oligonucleotides | NO | hCT2294818;hCT19324 |
| 281353 | Biological Testing | ISIS-9002 | Antisense Therapy: Oncolytic Drugs | Oligonucleotides | NO | hCT2294818:hCT19324 |
| 281877 | Biological Testing | ON-5543 | Antisense Therapy: Oncolytic Drugs | Apoptosis Inducers; Cell Adhesion Inhibitors | NO | hCT2290980;hCT12330 |
| 283041 | Biological Testing | ISIS-18669 | Antiarthritic Drugs: Antisense Therapy: Oncolytic Drugs | Oligonucleotides | BC012419 | hCT2312893;hCT2312891;hCT1957438;hCT31269 |
| 283042 | Biological Testing | ISIS-18623 | Antiarthritic Drugs; Antisense Therapy; Oncolytic Drugs | Oligonucleotides | BC012419 | hCT1957437;hCT2312893;hCT2312891;hCT2312896;hCT1957438;hCT 31269 |
| 283043 | Biological Testing | ISIS-18624 | AntiartMitic Drugs; Antisense Therapy; Oncolytic Drugs | Oligonucleotides | BC012419 | hCT1957437;hCT2312893;hCT2312891;hCT2312885;hCT1957438:hCT 31269 |
| 283044 | Biological Testing | ISIS-18629 | Antiarthritic Drugs; Antisense Therapy: Oncolytic Drugs | Oligonucleotides | BC012419 | hCT2312893;hCT2312991;hCT2312895;hCT1957438;hCT31269 |
| 283045 | Biological Testing | ISIS-18704 | Antiarthritic Drugs: Antisense Therapy; Oncolytic Drugs | Oligonucleotides | BC012419 | hCT1957437;hCT2312893;hCT2312891;hCT2312895;hCT1957438;hCT 31269 |
| 283749 | Biological Testing | AS-II-626-20 | Antisense Therapy; Oncolytic Drugs | Oligonucleotides | BC001886;BC030154:AK092671 | hCT2343740;hCT2267461;hCT14945 |
| 284344 | Biological Testing | ISIS-17509 | Antisense Therapy; Oncolytic Drugs | Oligonucleotides | BC016281 | hCT1954749;hCT1833851;hCT201188 |
| 284345 | Biological Testing | ISIS-20416 | Oncolytic Drugs | Oligonucleotides | BC017197 | hCT2280551;hCT2280554;hCT2280552 |
| 284607 | Biological Testing | ISIS-14864 | Antisense Therapy; Oncolytic Drugs | Oligonucleotides | NO | hCT20429 |
| 284608 | Biological Testing | ISIS-27616 | Treatment of Shock | Oligonucleotides | NO | hCT32453 |
| 284687 | Biological Testing | ISIS-19493 | Antiarthritic Drugs; Oncolytic Drugs | Oligonucleotides | BC010140;AK056611 | hCT2286539:hCT15432 |
| 284688 | Biological Testing | ISIS-19465 | Antiarthritic Drugs; Oncolytic Drugs | Oligonucleotides | BC010140;AK056611 | hCT2286539;hCT15432 |
| 284689 | Biological Testing | ISIS-19472 | Antiarthritic Drugs; Oncolytic Drugs | Oligonucleotides | BC010140:AK056611 | hCT2286539;hCT15432 |

**Table 1-5**

| | | | | | | |
|---|---|---|---|---|---|---|
| 284690 | Biological Testing | ISIS-19476 | Antiarthritic Drugs; Oncolytic Drugs | Oligonucleotides | BC010140;AK056611 | hCT2286539;hCT15432 |
| 284691 | Biological Testing | ISIS-19478 | Antiarthritic Drugs; Oncolytic Drugs | Oligonucleotides | NO | NO |
| 284692 | Biological Testing | ISIS-19496 | Antiarthritic Drugs; Oncolytic Drugs | Oligonucleotides | BC010140;AK056611 | hCT2286539;hCT15432 |
| 284693 | Biological Testing | ISIS-19505 | Antiarthritic Drugs; Oncolytic Drugs | Oligonucleotides | BC010140;AK056611 | hCT2286539:hCT15432 |
| 284694 | Biological Testing | ISIS-19536 | Antiarthritic Drugs; Oncolytic Drugs | Oligonucleotides | BC010140;AK056611 | hCT2286539:hCT15432 |
| 285044 | Biological Testing | ISIS-28313 | Antisense Therapy. Oncolytic Drugs | Oligonucleotides | AK026690 | hCT13618;hCT22B9154 |
| 285051 | Biological Testing | HCL-113; HCL-102R2 | Antisense Therapy: Oncolytic Drugs | Oligonucleotides | NO | hCT2350563;hCT1966585;hCT1966588;hCT2350564;hCT2350562 |
| 285223 | Biological Testing | | Liver Cancer Therapy | Oligonucleotides | BC027881 | hCT6218 |
| 285563 | Biological Testing | ISIS-18268 | Antipsoriatics; Atopic Dermatitis, Agents for | Anti-ICAM-1 Agents; Oligonucleotides | BC015969 | hCT19627 |
| 285639 | Biological Testing | | Antisense Therapy; Oncolytic Drugs | Angiogenesis Inhibitors; Oligonucleotides | AK056914 | hCT1963326;hCT10063:hCT2285905;hCT2285910;hCT2285909;hCT22 85904;hCT1963325 |
| 286398 | Biological Testing | | Oncolytic Drugs | Angiogenesis Inhibitors; Oligonucleotides | BC000666;BC001142;BC001719;BC005 116;BC007313;BC010405;BC010614;B C015009;BC015090;BC017247;BC0182 99;BC024294;BC018766;BC025951;BC 028168;BC032400;BC033821;AB05872 1;AB058677;AB051442;AB033068;AB02 0706;D87470;D87433;AK026431;AK026 141;AK026052;AK025061;AK024603;AK 000555;AK000460;AK024427;AK057462 AK057174;AK056890;AK056767;AK056 506;AK056117;AK055944;AK055873;AK 055603;AK054847;AK054793;AK027640 AK024330;AK023749;AK023507;AK023 430;AK023224;AK023200;AK023036;AK 023004;AK022605;AK022564;AK002110 AK001155;AK090455;AK098752;AK097 637;AK097405;AK097294;AK095781;AK 095396;AK094824:AK094794;AK09478 ;AK094496;AK094155;AK093774;AK093 642;AK092890;AK092237;AK092221;AK 092057;AK091622;AK091600;AK091399 ;AK091394;AL834326;AL834308;AL834 280:AL834278:AL833926;AL833210;AL 832718:AL831913;AL359590:AL162032; AL137597;AL133636;AL117431;AL080 | NO |
| 287657 | Phase II | AVI-4126;Oncomyc-NG - | Antisense Therapy: Breast Cancer Therapy; Lung Cancer Therapy: Lymphoma Therapy Pancreatic Cancer Therapy: Prostate Cancer Therapy: Renal Cancer Therapy: Restenosis Treatment of | Inhibitors of Signal Transduction Pathways: MYC Expression Inhibitors; Oligonucleotides | BC000141:BC000917 | hCT2348021;hCT6947;hCT2297650;hCT2297651 |
| 287817 | Biological Testing | ISIS-25564 | Oncolytic Drugs | Oligonucleotides | BC001360;BC005976 | hCT2286520;hCT11211;hCT2286519 |

**Table 1-6**

| | | | | | | |
|---|---|---|---|---|---|---|
| 287819 | Biological Testing | ISIS-25547 | Oncolytic Drugs | Oligonucleotides | BC001360;BC005976 | hCT2286519 |
| 287820 | Biological Testing | ISIS-25554 | Oncolytic Drugs | Oligonucleotides | BC001360;BC005976 | hCT2286520;hCT11211:hCT2286519 |
| 287822 | Biological Testing | ISIS-25561 | Oncolytic Drugs | Oligonucleotides | BC001360;BC005976 | hCT2286520;hCT11211;hCT2286519 |
| 287823 | Biological Testing | ISIS-25563 | Oncolytic Drugs | Oligonucleotides | BC001360;BC005976 | hCT2286520;hCT11211:hCT2286519 |
| 287824 | Biological Testing | ISIS-24501 | | Antiinflammatory Drugs; Oligonucleotides | NO | hCT1951448 |
| 287825 | Biological Testing | ISIS-24490 | | Antiinflammatory Drugs: Oligonucleotides | NO | hCT1951448 |
| 287827 | Biological Testing | ISIS-24493 | | Antiinflammatory Drugs; Oligonucleotides | NO | hCT1951448 |
| 287828 | Biological Testing | ISIS-24494 | | Antiinflammatory Drugs: Oligonucleotides | NO | hCT1951448 |
| 287829 | Biological Testing | ISIS-24502 | | Antiinflammatory Drugs; Oligonucleotides | NO | hCT1951448 |
| 287978 | Biological Testing | ISIS-24121 | Oncolytic Drugs; Treatment of Transplant | Oligonucleotides | NO | hCT6175 |
| 287979 | Biological Testing | ISIS-24089 | Oncolytic Drugs; Treatment of Transplant | Oligonucleotides | NO | hCT6175 |
| 287980 | Biological Testing | ISIS-24117 | Oncolytic Drugs; Treatment of Transplant | Oligonucleotides | NO | hCT6175 |
| 287981 | Biological Testing | ISIS-24132 | Oncolytic Drugs; Treatment of Transplant | Oligonucleotides | NO | hCT6175 |
| 287982 | Biological Testing | ISIS-25323 | Oncolytic Drugs | Oligonucleotides | BC007245;BC009177;AK094474 | hCT2278786;hCT2278785 |
| 287983 | Biological Testing | ISIS-25307 | Oncolytic Drugs | Oligonucleotides | BC007245;BC009177;AK094474 | hCT2278786;hCT2278778;hCT2278785 |
| 287984 | Biological Testing | ISIS-25340 | Oncolytic Drugs | Oligonucleotides | BC007245;BC009177;AK094474 | hCT2278786;hCT2278778;hCT2278785 |
| 287985 | Biological Testing | ISIS-25380 | Oncolytic Drugs | Oligonucleotides | BC007245;BC009177;AK094474 | hCT2278786;hCT2278778;hCT2278785 |
| 287986 | Biological Testing | ISIS-25434 | Oncolytic Drugs | Oligonucleotides | NO | hCT2266410;hCT22918 |
| 287987 | Biological Testing | ISIS-25442 | Oncolytic Drugs | Oligonucleotides | NO | hCT2266410;hCT22918 |
| 287988 | Biological Testing | ISIS-25459 | Oncolytic Drugs | Oligonucleotides | NO | hCT2266410;hCT22918 |
| 288144 | Preclinical | AS-AT1R-ODN mRNA | Antisense Therapy; Hypertension, Treatment of | Oligonucleotides | NO | NO |
| 288159 | Biological Testing | ISIS-25513 | Oncolytic Drugs | Oligonucleotides | NO | hCT2273848;hCT2273843;hCT2273845;hCT7194 |
| 288160 | Biological Testing | ISIS-25534 | Oncolytic Drugs | Oligonucleotides | NO | hCT2273848;hCT2273843;hCT2273845;hCT7194 |
| 288161 | Biological Testing | ISIS-25543 | Oncolytic Drugs | Oligonucleotides | NO | NO |
| 288162 | Preclinical | ISIS-23722 | Antisense Therapy. Oncolytic Drugs | Apoptosis Inducers; Drugs Inhibiting Gene Expression; Oligonucleotides | BC000784;BC007606.BC008718;BCO 12 164;BC034148 | hCT1958629;hCT2336837;hCT2336882;hCT2336835;hCT1967440;hCT 1982328:hCT18953 |
| 288164 | Biological Testing | ISIS-23665 | Oncolytic Drugs | Oligonucleotides | BC000784;BC008718:BC034148 | hCT1958829;hCT2336837;hCT2336835;hCT1967440;hCTt1962326;hCT 18953 |
| 288165 | Biological Testing | ISIS-23705 | Oncolytic Drugs | Oligonucleotides | BC000764;BC008718:BC034148 | hCT1958629;hCT2336837;hCT2336835;hCT 1967440;hCT 1962326;hCT 18953 |
| 288200 | Biological Testing | ISIS-19387 | Oncolytic Drugs | Oligonucleotides | BC001188 | hCT2287773;hCT1965327;hCT13179 |
| 288201 | Biological Testing | ISIS-18814 | Oncolytic Drugs | Oligonucleotides | BC001188 | hCT2287773;hCT1965327;hCT13179 |
| 288202 | Biological Testing | ISIS-18854 | Oncolytic Drugs | Oligonucleotides | BC001188 | hCT2287773;hCT1965327;hCT13179 |
| 288203 | Biological Testing | ISIS-18859 | Oncolytic Drugs | Oligonucleotides | BC001188 | hCT2287773;hCT1965327;hCT13179 |
| 288204 | Biological Testing | ISIS-23905 | Oncolytic Drugs | Oligonucleotides | NO | hCT2250682;hCT2250685;hCT1960109;hCT13972;hCT1960108 |
| 288205 | Biological Testing | ISIS-23937 | Oncolytic Drugs | Oligonucleotides | NO | hCT2250682;hCT2250685;hCT1960109;hCT13972;hCT1960108 |
| 288206 | Biological Testing | ISIS-23946 | Oncolytic Drugs | Oligonucleotides | NO | hCT2250682;hCT2250685;hCT1960109;hCT13972;hCT1960108 |

**Table 1-7**

| | | | | | | |
|---|---|---|---|---|---|---|
| 288795 | Biological Testing | ISIS-22783 | Antisense Therapy; Oncolytic Drugs | BCL2L1 Expression Inhibitors: Oligonucleotides | BC019307 | hCT2313721:hCT2313724;hCT30057:hCT2313722 |
| 288800 | Phase II | ISIS-104838 | Antipsoriatics: Inflammatory Bowel Disease. Agents for Rheumatoid Arthritis, Treatment | Oligonucleotides; TNF-alpha Production Inhibitors | BC028148 | hCT35001 |
| 288805 | Biological Testing | ISIS-16834 | Oncolytic Drugs | Oligonucleotides | BC032410;BC033810;AK054886 | hCT2316245;hCT2318240:hCT2318248;hCT2318247;hCT18775:hCT19 58140;hCT1958141;hCT2318242 |
| 288806 | Biological Testing | ISIS-15910 | Oncolytic Drugs | | BC031052 | hCT2268155;hCT2268158;hCT2268156 |
| 289146 | Preclinical | ISIS-17470 | Antisense Therapy: Inflammatory Bowel Disease, Agents for. Treatment of Transplant | Anti-ICAM-1 Agents; Cell Adhesion inhibitors: Oligonucleotides | NO | NO |
| 289902 | Preclinical | | Antipsoriatics | Oligonucleotides | NO | hCT2269330;hCT1640854 |
| 289903 | Preclinical | | Antipsoriatics | Oligonucleotides | NO | hCT1818658;hCT2265047;hCT1961915;hCT2269330;hCT1640854 |
| 289904 | Preclinical | | Antipsoriatics | Oligonucleotides | AK002038;AL136944 | hCT15918;hCT1954506;hCT2269330;hCT1640854 |
| 291056 | Biological Testing | ISIS-20772 | Oncolytic Drugs | Oligonucleotides | NO | hCT1960916;hCT2262305;hCT1961254 |
| 291057 | Biological Testing | ISIS-20753 | Oncolytic Drugs | Oligonucleotides | NO | HCT1960916;hCT2262305;hCT1961254;hCT1961253 |
| 291058 | Biological Testing | ISIS-20764 | Oncolytic Drugs | Oligonucleotides | NO | hCT1960916;hCT2262305;hCT1961254 |
| 291059 | Biological Testing | ISIS-20776 | Oncolytic Drugs | Oligonucleotides | NO | hCT1960916;hCT2262305;hCT1961254 |
| 295436 | Preclinical | ISIS-25302 | Antisense Therapy; Inflammatory Bowel Disease, Agents for | Oligonucleotides: TNF Expression Inhibitors | NO | NO |
| 297290 | Biological Testing | ISIS-105990 | Antisense Therapy; Oncolytic Drugs | Oligonucleotides; PPARG2 Expression Inhibitors | NO | hCT2275112;hCT19464 |
| 297292 | Biological Testing | ISIS-105989 | Antisense Therapy: Oncolytic Drugs | Oligonucleotides; PPARG2 Expression Inhibitors | NO | hCT2275112;hCT19464 |
| 297293 | Biological Testing | ISIS-106008 | Antisense Therapy; Oncolytic Drugs | Oligonucleotides; PPARG2 Expression Inhibitors | NO | hCT2275112;hCT19464 |
| 297294 | Biological Testing | ISIS-106011 | Antisense Therapy | Drugs Acting on Peroxisome Proliferator-Activated Receptors (PPAR) | NO | hCT2275112;hCT19464 |
| 297741 | Preclinical | PB2-as | Anti-Influenza A Virus Drugs; Antisense Therapy | Oligonucleotides; PB2 Expression Inhibitors | NO | NO |
| 298321 | Preclinical | ODN1 0x5 OMe | Anti-Cytomegalovirus Drugs: Genital Warts, Treatment | Oligonucleotides | NO | NO |
| 298553 | Biological Testing | ISIS-112043 | Antisense Therapy; Oncolytic Drugs | HNRNPA1 Expression Inhibitors; Oligonucleotides | BC002355;BC012158;BC009600;BC033 714;AK094423;AK091082 | hCT2345413;hCT 1971122;hCT2321107;hCT 1782979;hCT2297386;hCT 2350412;hCT2292881:hCT1970790;hCT2311220;hCT2323737;hCT225 0896;hCT2311219;hCT2254987;hCT1644759;hCT1817010;hCT182960 9;hCT29166;hCT2316338;hCT2316337;hCT2316339;hCT2316340;hCT 2259481;hCT2259480;hCT2258078;hCT2250895;hCT2311228:hCT231 1229;hCT2311224;hCT2311226;hCT2311225;hCT2311227;hCT231122 3;hCT2311222;hCT1779207;hCT1641256;hCT2254988;hCT2254990;hC T2254989 |
| 298554 | Biological Testing | ISIS-112050 | Antisense Therapy; Oncolytic Drugs | HNRNPA1 Expression Inhibitors; Oligonucleotides | BC002355;BC012158;BC009600;BC033 714 | hCT2311220:hCT2311219:hCT2264600:hCT2311228:hCT2311229:hCT 2311224;hCT2311226;hCT2311225;hCT2311227;hCT231122 |

**Table 1-8**

| | | | | | | |
|---|---|---|---|---|---|---|
| 298555 | Biological Testing | ISIS-112052 | Antisense Therapy: Oncolytic Drugs | HNRNPA1 Expression Inhibitors; Oligonucleotides | BC002355;BCO1258;BC009600;BC033 714;AK094423;AK091082 | hCT1782979;hCT2350412;hCT1970790:hCT2311220;hCT2311219:hCT 2254987;hCT2316338:hCT2316337:hCT2316339:hCT2316340:hCT231 1228:hCT23f 1229:hCT2311224;hCT2311226;hCT2311225:hCT231122 7;hCT2311222;hCT2266735;hCT2254988;hCT2254990;hCT2254989 |
| 299021 | Preclinical | | Antisense Therapy; Hypertension. Treatment of | Oligonucleotides | NO | hCT31091 |
| 299324 | Biological Testing | ANG-1051 | Antipsoriatics; Antisense Therapy: Oncolytic Drugs; Restenosis Treatment of | CCNE1 Expression Inhibitors; Oligonucleotides | NO | hCT1965835;hCT2256463;hCT11514 |
| 299326 | Biological Testing | ISIS-28030 | Antisense Therapy; Oncolytic Drugs | Oligonucleotides; P13 Kinase p85 Expression Inhibitors | BC030815;AK000121;AK094785 | hCT28562;hCT2266529 |
| 299330 | Biological Testing | ISIS-102453 | Antisense Therapy; Oncolytic Drugs | CTNNB1 Expression Inhibitors; Oligonucleotides | NO | hCT2277746;hCT18957;hCT2277743 |
| 299333 | Biological Testing | ISIS-25237 | Antisense Therapy; Oncolytic Drugs | ITGB3 Expression Inhibitors; Oligonucleotides | NO | hCT2272751;hCT2272750;hCT2272752;hCT18745 |
| 299334 | Biological Testing | ISIS-29714 | Antisense Therapy; Oncolytic Drugs | ETS2 Expression Inhibitors; Oligonucleotides | BC017040;AK096841 | hCT2295169;hCT2295171;hCT401223 |
| 299335 | Biological Testing | ISIS-29764 | Antisense Therapy; Oncolytic Drugs | ETS2 Expression Inhibitors; Oligonucleotides | BC017040;AK096841 | hCT2295169;hCT2295171 ;hCT401223 |
| 299336 | Biological Testing | ISIS-25962 | Antisense Therapy; Oncolytic Drugs | GNAI3 Expression Inhibitors; Oligonucleotides | BC025285 | hCT2279450 |
| 299337 | Biological Testing | ISIS-25949 | Antisense Therapy: Oncolytic Drugs | GNAI3 Expression Inhibitors; Oligonucleotides | BC025285 | hCT2279450 |
| 299392 | Biological Testing | ISIS-21329 | Antisense Therapy; Oncolytic Drugs | GNAS1 Expression Inhibitors; Oligonucleotides | AK026564;AK054862;AK093534 | hCT2314454;hCT1956030;hCT1956029;hCT2314435;hCT2314452;hCT 2314438;hCT2314443;hCT2314458;hCT1956031;hCT1950751;hCT195 0750;hCT1827487;hCT1956027;hCT2314459;hCT1956028;hCT195603 2;hCT2314450 |
| 299393 | Biological Testing | ISIS-21331 | Antisense Therapy; Oncolytic Drugs | GNAS1 Expression Inhibitors; Oligonucleotides | AK026564;AK054862;AK093534 | hCT2314454;hCT1956030;hCT1956029;hCT2314435;hCT2314452;hCT 2314438;hCT2314443;hCT2314458:hCT1956031:hCT1950751;hCT195 0750;hCT1827487;hCT1956027;hCT2314459;hCT1956028;hCT195603 2;hCT2314450 |
| 299394 | Biological Testing | ISIS-101528 | Antisense Therapy; Oncolytic Drugs | MAP2K7 Expression Inhibitors; Oligonucleotides | NO | hCT1951272;hCT2290661;hCT2290660;hCT13651 |
| 299395 | Biological Testing | ISIS-101542 | Antisense Therapy; Oncolytic Drugs | MAP2K7 Expression inhibitors: Oligonucleotides | NO | hCT1951272;hCT2290663;hCT2290661;hCT2290660;hCT13651 |
| 299396 | Biological Testing | ISIS-101549 | Antisense Therapy: Oncolytic Drugs | MAP2K7 Expression Inhibitors; Oligonucleotides | NO | hCT1951272;hCT2290663;hCT2290661;hCT2290660;hCT13651 |
| 299493 | Preclinical | ORF4-PE | Antisense Therapy; Stroke, Treatment | Oligonucleotides; TNF Expression Inhibitors | NO | hCT35001 |
| 300745 | Biological Testing | ISIS-29176 | Antisense Therapy; Oncolytic Drugs | AKT3 Expression Inhibitors; Oligonucleotides | AL117525 | hCT2293796;hCT2293797;hCT6401 |
| 300746 | Biological Testing | ISIS-29177 | Antisense Therapy; Oncolytic Drugs | AKT3 Expression Inhibitors; Oligonucleotides | AL117525 | hCT2293796;hCT2293797;hCT6401 |

**Table 1-9**

| | | | | | | |
|---|---|---|---|---|---|---|
| 300749 | Biological Testing | ISIS-32162 | Antiallergy/Antiasth matic Drugs; Antiarthritic Drugs; Antisense Therapy | Oligonucleotides; P13 Kinase p110 Expression Inhibitors | NO | hCT2292008;hCT2292011;hCT1787138;hCT2292009 |
| 301668 | Phase II | ISIS-113715 | Antisense Therapy; Type 2 Diabetes. Agents for | Oligonucleotides; PTPN1 Expression Inhibitors | BC015660;BC018164 | hCT2314156;hCT28364 |
| 302457 | Preclinical | ISIS-15421 | Antisense Therapy | Inhibitors of Signal Transduction Pathways; Oligonucleotides; PTK2 Expression | AK094999;AL832961 | hCT2298336;hCT2298338;hCT2298337;hCT11819;hCT229834 |
| 302582 | Preclinical | Anti-SCF | Antiellergy/Antiasth matic Drugs; Antisense Therapy | Oligonucleotides; SCF Expression Inhibitors | NO | hCT2307114;hCT2307116;hCT18442;hCT18443 |
| 304370 | Preclinical | AS TRPM2 ODN | Antineoplastic Enhancing Agents; Antisense Therapy | CLU Expression Inhibitors; Oligonucleotides | BC010514;BC019588;AK093399 | hCT2273160;hCT2273163;hCT1961589;hCT8333;hCT2273164;hCT227 3161 |
| 304953 | Preclinical | | Alzheimer's Dementia, Treatment of ; Antisense Therapy | APP Expression Inhibitors: Oligonucleotides | NO | NO |
| 309413 | Preclinical | AS-MOR: AS-MOR1 | Antisense Therapy: Pharmacological Tools | | BC015735;BC016649;AB007940;D1468 9;AK074403;AK026089;AK025735;AK05 7158;AK091971 | hCT2345555;hCT2340736;hCT2322995;hCT2343102;hCT2343085;hCT 2296417;hCT2333938;hCT1959548;hCT2269902;hCT2296416:hCT232 3176;hCT1950614;hCT21359;hCT1643002;hCT28292;hCT2296412;hC T2296415;hCT2296419;hCT2296413;hCT1950206;hCT1643777;hCT60 |
| 309423 | Biological Testing | ISIS-29895 | Antisense Therapy | NCOA1 (SCR1) Expression Inhibitors; Oligonucleotides | NO | hCT2266054;hCT2266053;hCT12467 |
| 310112 | Biological Testing | ISIS-126129 | Antidiabetic Drugs; Antisense Therapy. Oncolytic Drugs | ADPRT2 Expression Inhibitors; Oligonucleotides | AK001980 | hCT1967705;hCT31740 |
| 310113 | Biological Testing | ISIS-113037 | Antisense Therapy-. Atherosclerosis Therapy; Oncolytic Drugs | E2F1 Expression Inhibitors; Oligonucleotides | NO | hCT2313231;hCT29147 |
| 310114 | Biological Testing | ISIS-114232 | Antisense Therapy: Oncolytic Drugs | E2F3 Expression Inhibitors; Oligonucleotides | BC016847;D38550 | hCT1952441;hCT2251810;hCT27983 |
| 311065 | Biological Testing | ISIS-100750 | Antiarthritic Drugs; Antisense Therapy | ADAM10 Expression Inhibitors; Oligonucleotides | AK023460 | hCT31293 |
| 312591 | Preclinical | | Antisense Therapy; Leukemia Therapy | MYB Expression Inhibitors; Oligonucleotides | NO | hCT2331429;hCT2331427;hCT2331431;hCT23568 |
| 313482 | Biological Testing | ISIS-17946 | Antisense Therapy, Oncolytic Drugs | Oligonucleotides; TERT Expression | NO | hCT2268566;hCT2268568 |
| 313607 | Biological Testing | ISIS-124701 | Antisense Therapy; Oncolytic Drugs | ID1 Expression Inhibitors; Oligonucleotides | BC00-0613;BC012420 | hCT28373;hCT1968043;hCT1968044 |
| 314554 | Biological Testing | ISIS-117386 | Antidiabetic Drugs: Antiobasity Drugs; Antisense Therapy; Oncolytic Drugs | CEBPA Expression Inhibitors: Oligonucleotides | NO | hCT11217 |
| 314555 | Biological Testing | ISIS-116508 | Antidiabetic Drugs; Antiobesity Drugs; Antisense Therapy; Oncolytic Drugs | CEBPB Expression Inhibitors; Oligonucleotides | BC007538:BC021931 | hCT28128 |

**Table 1-10**

| | | | | | | |
|---|---|---|---|---|---|---|
| 317151 | Biological Testing | | Antisense Therapy; Oncolytic Drugs | Oligonucleotides | NO | hCT1823775:hCT1969179;hCT1989180;hCT18480 |
| 317264 | Biological Testing | ISIS-19558 | Antisense Therapy; Oncolytic Drugs | ERBB3 Expression Inhibitors; Oligonucleotides | BC002706 | hCT2307681:hCT2307680;hCT2307676;hCT2307678;hCT16106 |
| 317265 | Biological Testing | ISIS-120366 | Antisense Therapy-, Lipoprotein Disorders, Treatment of | LYPLA1 Expression Inhibitors: Oligonucleotides | NO | NO |
| 317266 | Biological Testing | ISIS-120367 | Antisense Therapy: Lipoprotein Disorders, Treatment of | LYPLA1 Expression Inhibitors: Oligonucleotides | NO | NO |
| 318118 | Biological Testing | ISIS-124904 | Antisense Therapy; Oncolytic Drugs | NCOA1 (SCR1) Expression Inhibitors; Oligonucleotides | BC013000 | hCT2308523;hCT2309355;hCT2309356;hCT2309354;hCT30920 |
| 318838 | Preclinical | ODNas750/15 | Antisense Therapy: Prostate Cancer Therapy | AR Expression Inhibitors; Oligonucleotides | BC030254;BC033676;AB007935;AK057 090;AK056580;AKOO1437;AK097716;AK 092673 | HCT1640468;hCT1955257:hCT13206;hCT1955256;hCT2256475;hCT22 56476;hCT31091;hCT2263098;hCT2263104;hCT2252047;hCT2282185; hCT2252377;hCT28187;hCT2271789;hCT2271790;hCT20246;hCT2252 375;hCT2314076;hCT30060;hCT2314077;hCT7807;hCT2315674;hCT2 315670;hCT20312;hCT1822951;hCT2282184;hCT2282183;hCT228544 1;hCT28273;hCT2271966 |
| 318927 | Biological Testing | F275-AS; CHIR3-3 275 | Antisense Therapy; Oncolytic Drugs | LAMB3 Expression inhibitors: Oligonucleotides | NO | hCT12368 |
| 319468 | Preclinical | AS-143; BetaC AS-ODN | Antiallergy/Antiasth matic Drugs; Antisense Therapy | CSF2RB Expression Inhibitors; Oligonucleotides | NO | NO |
| 320263 | Biological Testing | GEM-91H | Anti-HIV Agents; Gene Therapy | Oligonucleotides | NO | NO |
| 320316 | Biological Testing | ISIS-105352 | Antisense Therapy; Oncolytic Drugs | MAP4K4 Expression Inhibitors: Oligonucleotides | AB014587 | hCT 1955204;hCT1640566;hCT1962990;hCT2306239;hCT1955205;hCT 2351135 |
| 320683 | Biological Testing | ISIS-29977 | Antisense Therapy; Oncolytic Drugs | NCOA2 (SCR2) Expression Inhibitors: Oligonucleotides | NO | hCT9508;hCT2258951 |
| 322976 | Biological Testing | IGFBP-2 ASO | Antisense Therapy; Prostate Cancer Therapy | IGFBP2 Expression Inhibitors; Oligonucleotides | BC004312;BC009902;BC02769 | hCT7420 |
| 324091 | Biological Testing | ISIS-127695 | Antisense Therapy; Oncolytic Drugs | Oligonucleotides; STK15 Expression Inhibitors | BC001280;BC002499;BC006423;BC027 464 | hCT2311845;hCT2311850:hCT1953376;hCT1965747;hCT1953374;hCT 1950770:hCT30342;hCT1953375 |
| 324093 | Biological Testing | ISIS-124773 | Antisense Therapy, Oncolytic Drugs | Oligonucleotides; TNFAIP3 Expression Inhibitors | NO | hCT7828:hCT2331264 |
| 324098 | Biological Testing | | Antisense Therapy-, Oncolytic Drugs | MAP3K3 Expression Inhibitors; Oligonucleotides | AL834303 | hCT2295836:hCT33098 |
| 324100 | Biological Testing | ISIS-114117 | Antisense Therapy: Oncolytic Drugs | E2F2 Expression Inhibitors; Oligonucleotides | NO | hCT2350481;hCT28817;hCT2256381;hCT2350480 |
| 324102 | Biological Testing | ISIS-118473 | Antidiabetic Drugs; Antisense Therapy | Oligonucleotides; PHKA1 Expression Inhibitors | NO | hCT9697:hCT1640369:hCT2272237 |
| 324105 | Biological Testing | ISIS-128456 | Antipsoriatics; Antisense Therapy; Oncolytic Drugs | EGFR Expression Inhibitors; Oligonucleotides | NO | hCT2259143;hCT2259141:hCT1952578 |

**Table 1-11**

| | | | | | | |
|---|---|---|---|---|---|---|
| 324106 | Biological Testing | ISIS-123119 | Antisense Therapy; Oncolytic Drugs | MAP3K4 Expression Inhibitors; Oligonucleotides | D86968 | hCTI950009;hCT24617 |
| 326724 | Preclinical | PA-432; PNA-432 | Antibacterial Drugs; Antisense Therapy | FtsZ Expression Inhibitors | AK024536;AK027364;AK023183;AK022 432;AK002033;AK001012;AK097218;AL 137687 | NO |
| 335673 | Biological Testing | ASO | Antineoplastic Enhancing Agents; Antisense Therapy | IGF1R Expression Inhibitors; Oligonucleotides | NO | hCT2269330;hCT1640854 |
| 336935 | Preclinical | KDR/Flk-1-ASO | Antisense Therapy; Gastric Cancer Therapy | KDR (VEGFR2) Expression Inhibitors; Oligonucleotides | NO | hCT2326452:hCT2326453;hCT22753 |
| 354190 | Preclinical | G4 AS ODN | Antineoplastic Enhancing Agents: Antisense Therapy; Non-Small Cell Lung Cancer | Apoptosis Inducers; BIRC4 Expression Inhibitors; Oligonucleotides | BC032729 | hCT5866:hCT1967493:hCT1953707:hCT2253836 |
| 358744 | Phase I | VEGF-AS;AS-3; Veglin | Antisense Therapy; Oncolytic Drugs | Angiogenesis Inhibitors: Drugs Inhibiting Gene Expression: Oligonucleotides | AK056914 | hCT1963326;hCT10063;hCT2285905;hCT2285910;hCT2285909;hCT22 85904;hCT1963325 |
| 363974 | Preclinical | RBI-034 | Anti-RSV Drugs; Antisense Therapy | | AK095482 | hCT2350933 |

Although the mRNA data set may be inputted each time using an inputting means such as a keyboard or a mouse, it is preferable to memorize the data set in a memory means of a computer. As the memory means, a semiconductor memory means such as RAM or ROM may be used, and a recording medium such as a magnetic disc or a CD-ROM may be used. Alternatively, data can also be acquired by accessing to a server storing the data set via a telecommunication line such as an intranet/internet when necessary. Here, "inputted" means that the mRNA data set is in a state utilizable to execute step (1). Therefore, the means of inputting is not limited to manual inputting using a keyboard and the like, and encompasses selection of the mRNA data set stored in the above-described memory means by an operator using a mouse and the like, and reading into the computer, the mRNA data set on an optionally chosen recording medium or server, automatically selected by the computer.

"Capable of regulating" the translation of mRNA is referred to as having the capability of suppressing or promoting (enhancing) the translation. The secondary structure of RNA is referred to as a structure formed as a complementary sequence in the molecule locally becomes double-stranded; the double-stranded portion is referred to as a stem, and the single-stranded portion that connects complementary sequences that form the stem is referred to as a loop. "Local" means that the length of the sequence capable of assuming a secondary structure is not more than about 200 nucleotides, preferably about 100 to about 20 nucleotides, more preferably about 60 to about 30 nucleotides.

Although the local secondary structure selected in the present invention is not subject to limitation, as long as it is capable of regulating the translation of mRNA by interacting with a certain compound, preferably a low-molecular compound, to stabilize the structure, it is desirably of as low free energy (stable) as possible. Because the reduction in required free energy due to binding of a low-molecular compound decreases as the original free energy of the motif decreases (the interaction/hydrogen bond number formed between the low-molecular compound and motif decreases), drug discovery for conventional type lipophilic compounds is possible. The small number of hydrogen binding points offers another advantage that adverse reactions are unlikely to occur because there is no influence of any interaction with another portion of the mRNA. Conversely, although any motif may be selected here, selecting such a motif makes it difficult to obtain desired low-molecular compounds, and such compounds are nearly always in violation of Lipinski's rule.

Preferably, as an example of the local secondary structure selected in the present invention, a stem-loop structure can be mentioned. As mentioned herein, "a stem-loop structure" means any secondary structure that comprises one or more stem portions and one or more loop portions, and that is local to the mRNA as a whole. Preferably, the stem-loop structure selected in the present invention is a structure having one or more stem portions and one loop portion; if two or more stem portions are present, these stems are connected via a non-complementary (hence not forming a stem) sequence. Herein, a non-complementary portion present between stems is referred to as a bubble.

Herein, a stem-loop structure consisting of one stem and one loop, and not comprising a bubble, is referred to as stem-loop A, and a stem-loop structure having one bubble portion between two stems is referred to as stem-loop B (hereinafter referred to as stem-loop C, D, ... as the number of stems and bubbles increases one by one). The stem portion does not need to be completely complementary, and allows G-U base pair formation (wobbling) or a given frequency of mismatches. The loop and bubble portions do not need to be completely non-complementary, and allow a given frequency of base pair formation. Therefore, stem-loop B (C, D, ...) can also be viewed as a mismatch-rich stem-loop A, or a special stem-loop A structure having mismatches occurring in sequence in a certain portion thereof.

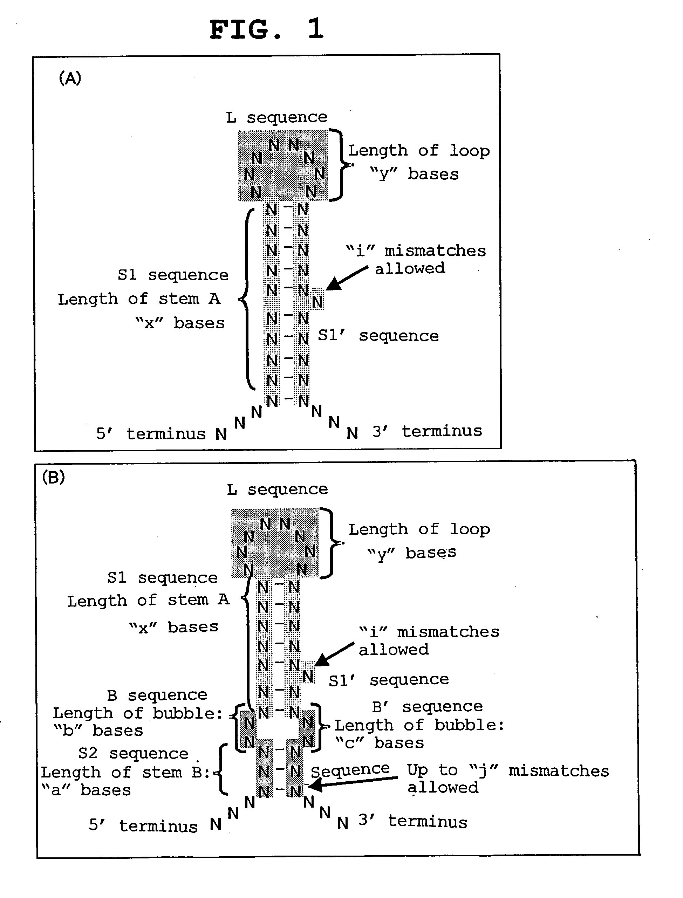

In more detail, in the present invention, stem-loop A means a part of continuous base sequence having an L sequence of a length of y bases inserted between an optionally chosen S1 sequence (length of x bases) and an S1' sequence nearly complementary to the S1 sequence, that is, a sequence wherein S1, L, and S1' are arranged in this order from the 5' terminus (but the complementarity of S1 and S1' should allow G-U wobbling and "i" mismatch(es); here, "a mismatch" is referred to as an extra base located in, or at an end of, the sequences S1 and S1', and not involved in stem formation; if there is no pairing base, one mismatch exists, and if the pairing base is not bound with hydrogen bondings, two mismatches exist) (see Figure 1A). In the above-described definition, "x" is an optionally chosen integer of 5 to 25, "y" is an optionally chosen integer of 3 to 20, and "i" is an optionally chosen integer of 0 to 10.

Stem-loop B means a sequence comprising the above-described stem-loop A with a B sequence of a length of "b" base(s) and an S2 sequence of a length of "a" bases, on the 5' side from the S1 sequence, and a B' sequence of a length of "c" base(s) and a S2' sequence nearly complementary to the S2 sequence, on the 3' side from the S1 sequence, resulting in the arrangement of S2, B, S1 L, S1', B', and S2' in this order from 5' (but the complementarity between S2 and S2' allows G-U wobbling and "j" mismatch(es)) (see Figure 1B). Here, "x" is an optionally chosen integer of 2 to 25, "y" is an optionally chosen integer of 3 to 20, "a" is an optionally chosen integer of 2 to 25, each of "b" and "c" is an optionally chosen integer of 0 to 25 (but one of "b" and "c" is not 0), and each of "i" and "j" is an optionally chosen integer of 0 to 5.

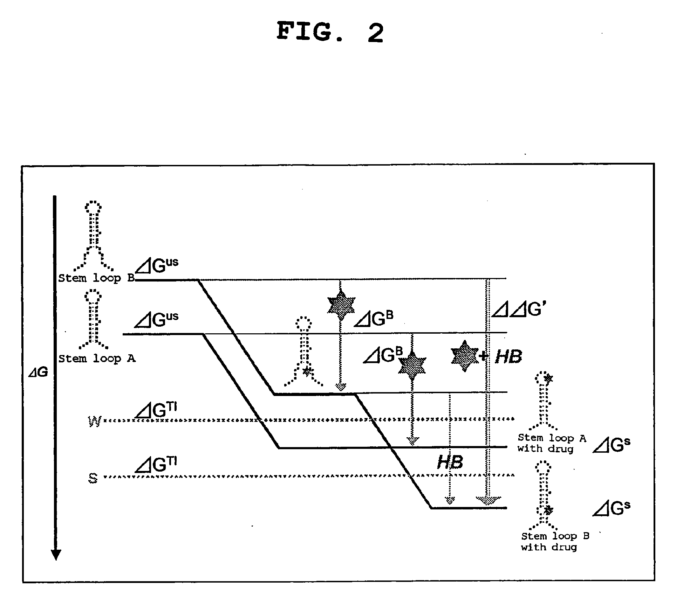

More preferably, the local secondary structure selected in the present invention is stem-loop A or stem-loop B, still more preferably stem-loop B. Figure 2 is to compare stem-loop A and stem-loop B from the viewpoint of the concept of dynamic range. Here, W and S are assumed limits of various helicase activities, and the free energy is written as ΔG^{TI}. A helicase activity as mentioned here is a total activity of helicase activities exhibited by various enzymes and the like on a case-by-case basis; therefore, ΔG^{TI} is a function of at least temperature and position on the mRNA. In vivo, structures having higher free energy than ΔG^{TI} are unwound and proteins are synthesized. The stability (ΔG^{TI}) of a stem-loop found in nature is surely higher than the helicase activity (S or W). In stem-loop B, the stem B in Figure 1 is not formed; therefore, the free energy thereof is considered to be higher than that of stem-loop A. When a low-molecular pharmaceutical is bound to a stem-loop, the stability of the stem-loop increases and ΔG^{S} decreases so that the stem-loop becomes more stabilized than the helicase activity, whereby the translation into protein is inhibited. For example, stem-loop A is stabilized due to the binding of a low-molecular pharmaceutical to the loop portion. On the other hand, because stem-loop B has an additional stem (stem B) formed due to the binding of a low-molecular pharmaceutical to the bubble portion, the reduction range of ΔG^{S} (dynamic range) is greater than that of stem-loop A. As the dynamic range increases, not only the choice of stem-loop broadens, but also it becomes more likely to exceed the helicase activity. That is, in the case of low helicase activity (W), regulation is possible even with stem-loop A, but in the case of high helicase activity (S), regulation is possible only with stem-loop B in some cases.

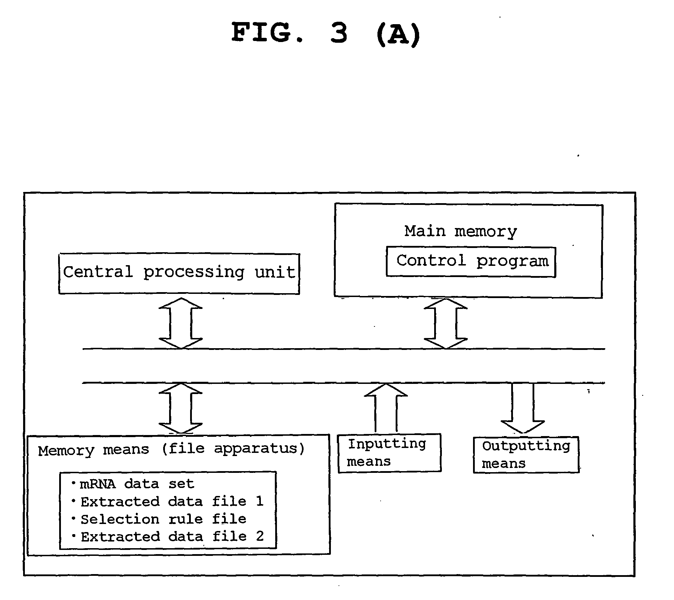

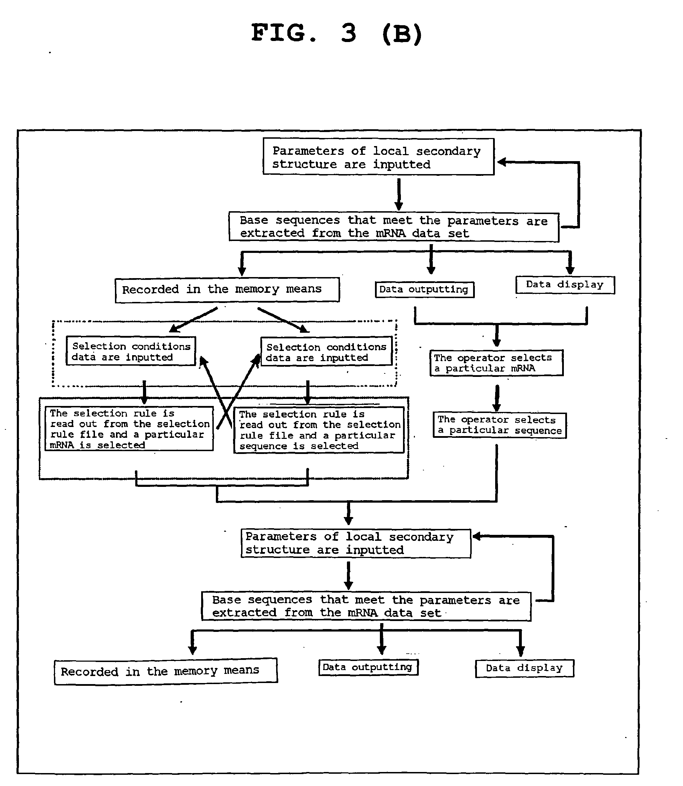

Figure 3A is a system configuration of an apparatus for executing the sequence selection method of the present invention; Figure 3B is a flow of the processing executed by the apparatus.

### Step (1): Extraction of an mRNA having a sequence capable of assuming a local secondary structure

First, parameters of a local secondary structure desired to be acquired by the operator are inputted via an inputting means such as a keyboard. For example, if the local secondary structure is stem-loop A, numerical values of the above-described variables "x", "y" and "i" are inputted; in the case of stem-loop B, numerical values of the above-described variables "x", "y", "a", "b", "c", "i" and "j" are inputted.

The central processing unit, at command from the control program in the main memory, searches the base sequence information of an mRNA data set memorized in a memory means (file apparatus), and extracts all partial base sequences that meet the combination of variables inputted. The results are recorded in a memory means as extracted data file 1, and can also be indicated on an indication means such as a display and/or outputted from an outputting means such as a printer. Although the mode of recording/indication and/or outputting is not subject to limitation, it is preferable, for example, that, the results be outputted in a form that allows the local secondary structure to be easily visually recognizable for each mRNA. If one or more sequences capable of assuming the desired local secondary structure are present in one mRNA, the sequences can be recorded/indicated/outputted in order of, for example, distance from the 5' side. If a plurality of sequences are found at the same position, all of them are recorded/indicated/outputted in an optionally chosen order. Preferably, information on the occurrence positions of the extracted sequences (for example, position numbers of bases at the starting point and ending point with the 5' terminus of the mRNA as 1, position numbers of bases at the starting point and ending point with the translation initiation codon as +1, and the like) is also recorded/indicated/outputted simultaneously.

If no local secondary structure that meets the combination of variables inputted is present in the mRNA data set inputted, or automatically, changed numerical values of variables may be inputted and the same processing may be repeated. The central processing unit, at command from the control program in the main memory, is capable of indicating a screen that makes a demand for the operator to change numerical values of variables by an indication means.

### Step (2) Selection of a particular mRNA

In step (2), a particular mRNA is selected from among the one or more mRNAs extracted in step (1). This step can be executed by subjective selection by the operator from among the one or more mRNAs recorded/indicated/outputted in view of the intended use (for example, subject disease and the like in the case of use as a drug discovery target) and the like. Alternatively, the step can also be executed by the central processing unit, which at command from the control program in the main memory, reads out a selection rule to be applied from among the selection rules previously recorded in a selection rule file stored in a memory means, on the basis of the selection conditions data inputted, and automatically selects a particular mRNA on the basis of the selection rule. In the latter case, one particular mRNA may be selected, or a plurality of mRNAs may be sorted out on the basis of the selection rule. The mRNA(s) selected can be recorded/indicated/outputted by the above-described recording/indication/outputting means. As examples of the selection conditions inputted, the intended use (e.g., kind of subject disease and the like), as well as the occurrence positions and number of sequences capable of assuming the local secondary structure, and the like can be mentioned. That is, if two or more mRNAs have a sequence capable of assuming the desired local secondary structure at different positions, priority is given to mRNAs having the sequence at positions expected to be capable of contributing more to the regulation of the translation of the mRNAs. If there are both an mRNA having only one sequence capable of assuming the desired local secondary structure at a position expected to have a major contribution to the regulation of the translation of the mRNA and an mRNA having a plurality of the same, priority is given to the former mRNA. Therefore, this step may be performed concurrently with the next step (3).

If only one mRNA is extracted in step (1), this step may be omitted to skip to step (3). If the operator or computer judges that the mRNA is inappropriate for use as a target in light of the above-described selection conditions, step (1) may be executed again after changing numerical values of variables.

### Step (3): Step for selecting a particular sequence capable of assuming a local secondary structure

In step (3), a particular sequence is selected from among one or more sequence capable of assuming the local secondary structure in the particular mRNA selected in step (2). This step can be executed by subjective selection by the operator of a sequence in the molecule of the particular mRNA selected which is capable of contributing more to the regulation of the translation from among one or more sequences capable of assuming the desired local secondary structure, in view of the occurrence position of each sequence and the like. Alternatively, the step can also be executed by the central processing unit, which at command from the control program in the main memory, reads out a selection rule to be applied from among the selection rules previously recorded in a selection rule file stored in a memory means, on the basis of the selection conditions data inputted, and automatically selects a particular sequence on the basis of the selection rule. In the latter case, one particular sequence may be selected, or a plurality of sequences may be selected. Also, a plurality of sequences may be sorted out on the basis of the selection rule. The sequence(s) selected can be recorded/indicated/outputted by the above-described recording/indication/outputting means. As examples of the selection conditions inputted, the occurrence position of the sequence capable of assuming the local secondary structure and the like can be mentioned. That is, if two or more sequences are capable of assuming the desired local secondary structure at different positions, priority is given to sequences present at positions expected to be capable of contributing more to the regulation of the translation of the mRNA. As described above, because the selection conditions also serve as the conditions for selecting a particular mRNA from among two or more mRNAs, this step can also be performed concurrently with step (2).

As examples of the position expected to have a major contribution to the suppression of the translation of the mRNA, a 5' non-translational region (5'UTR), a region covering the 5'UTR and coding region (CDS), preferably the translation initiation point and the vicinity thereof and the like can be mentioned. On the other hand, as the position expected to have a major contribution to the enhancement of the translation of the mRNA, a region involved in the degradation of the mRNA, for example, a region comprising a target sequence for RNA degrading enzyme and the like can be mentioned.

### Step (4): Confirmation of specificity of the particular sequence selected

In step (4), it is determined whether or not the particular sequence selected in step (3) is specific, that is, whether or not a compound that interacts with a certain portion of the sequence to regulate the translation of the mRNA does not regulate the translation of any other mRNA. This step is performed by confirming that a partial structure capable of interacting with the compound in the particular sequence selected is not present in the region involved in the regulation of the translation of any other mRNA, or if present, is actually incapable of regulating the translation. Because the selection conditions also serve as conditions for selecting a particular mRNA from among two or more mRNAs, or conditions for selecting a particular sequence, this step can also be performed concurrently with step (2) or step (3).

First, via an inputting means such as a keyboard, the base sequence of a portion of the sequence selected, which is expected to have a major contribution to the stabilization of the local secondary structure due to the binding of the compound is inputted. For example, when the local secondary structure is stem-loop A, a loop portion is preferable, and when the local secondary structure is stem-loop B, a bubble portion or loop portion is preferable, but these are not to be construed as limiting. Additionally, in the case of stem-loop A, numerical values of length x of the S1 sequence (not more than the numerical value of the sequence selected) and of mismatch number "i" (optionally chosen) are inputted. In the case of stem-loop B, numerical values of the sum of the length of the S1 sequence and S2 sequence x + a (not more than the numerical value of the sequence selected; but the value of each of "x" and "a" is optionally chosen within a possible range of numerical values), the value of each of mismatches "i" and "j" (each optionally chosen) and the length y of the loop are further inputted. It is also possible to input the sequence with the base at the tip of the stem adjacent to the loop or bubble portion included in the loop or bubble portion. In this case, the numerical value of "x" and x + a (maximum value) decreases proportionally.

The central processing unit, at command from the control program in the main memory, searches the base sequence information of an mRNA data set memorized in a memory means (file apparatus), and extracts all partial base sequences that meet the combination of the base sequence and variables inputted. The results are recorded in the memory means as an extracted data file 2, and also indicated on an indication means such as a display and/or outputted from an outputting means such as a printer. Although the mode of recording/indication and/or outputting is not subject to limitation, it is preferable, for example, that the results be outputted in a form that allows the local secondary structure to be easily visually recognizable for each mRNA. If one or more sequences capable of assuming the desired local secondary structure are present in one mRNA, the sequences can be recorded/indicated/outputted in order of, for example, distance from the 5' side. Preferably, information on the occurrence positions of the extracted sequences (for example, position numbers of bases at starting point and ending point with the 5' terminus of the mRNA as 1, position numbers of bases at starting point and ending point with the translation initiation codon as +1, distinction among 5'UTR, CDS, and 3'UTR, and the like) is also recorded/indicated/outputted simultaneously.

If each of the above-described variables is inputted with a range of numerical values, the central processing unit, at command from the control program in the main memory, repeatedly executes search of the base sequence information of the mRNA data set using all possible combinations of numerical values of variables, extracts a local secondary structure that meets each combination, and can record/indicate/output the same in the recording/indication/outputting means.

Of the mRNAs extracted, as the region involved in the regulation of the translation of an mRNA other than the target mRNA, for example, the region capable of suppressing the translation by assuming a local secondary structure, 5'UTR, a region covering 5'UTR and CDS, preferably a translation initiation point and the vicinity thereof, and the like can be mentioned; as the region capable of enhancing the translation by assuming a local secondary structure, a region involved in the degradation of mRNA, for example, a region comprising a target sequence for an RNA degrading enzyme and the like can be mentioned.

Provided that the above-described sequence selection method of the present invention is performed on an mRNA regulating the translation of which is effective in the prevention/treatment of one or more diseases, the sequence capable of assuming a particular local secondary structure in the mRNA, which is selected by the method, will become a useful target motif for screening for a compound having prophylactic/therapeutic activity for the disease. Provided that the sequence selection method of the present invention is intended to select a sequence capable of assuming a particular local secondary structure in a particular mRNA for screening for a compound capable of preventing/treating a particular disease, it is preferable to prepare a data set of one or more mRNAs by regulating the translation of which the subject disease can be prevented/treated in advance of execution of the above-described step (1). As examples of the method of preparing such a data set of mRNAs, a method comprising extracting a target mRNA for an antisense pharmaceutical candidate nucleic acid for a particular disease (for example, cancer) from a comprehensive human cDNA data set (for example, "CHGD_transcripts_R27f.fasta", supplied by Celera Genomics, "nuc_all.fa", supplied by H-invitational, and the like), obtained from the antisense pharmaceutical pipeline information of Isis Pharmaceuticals Company as described above, and the like can be mentioned.

The sequence selection method of the present invention is not limited to mRNAs; all RNAs capable of assuming a local secondary structure can be used as targets. As examples of RNAs expected to serve as targets for the prevention/treatment of a particular disease among RNAs other than mRNAs, that is, RNAs not translated into proteins (non-coding RNAs; ncRNAs), microRNAs (miRNAs) can be mentioned.

Small RNAs about 18 to 25 nucleotides in size, known as miRNAs, are essential to the transition of stage 1 to stage 2 in the larvae of nematodes, and have been drawing attention since they were shown to suppress the expression of a target mRNA by binding to the 3'UTR thereof at the translation stage. To date, more than 200 kinds of miRNAs have been reported, and have been suggested to be involved in the differentiation of hematopoietic stem cells and nerve stem cells, cancer, leukemia and the like. Although miRNAs suppress gene expression by a mechanism similar to that of siRNAs, siRNAs completely bind to the target mRNA sequence, whereas miRNAs only partially bind to the target mRNA; therefore, mRNAs are considered to be not degraded and to physically cause the suppression of the translation into protein. Because miRNAs target mRNAs in non-translational regions, they are considered to bind to regions involved in the regulation of gene expression to influence the same.

An miRNA is produced from a precursor having a stem-loop structure (pre-miRNA) by cleavage with an enzyme called a dicer. Therefore, provided that the stem-loop structure of the precursor is stabilized with a low-molecular compound to suppress miRNA production, the suppression of the translation of the target mRNA for the miRNA is hampered, and as a result, the translation of the mRNA can be promoted.

Lim, L. et al. constructed an original miRNA search software, and predicted that 200 to 255 miRNA genes would be present on the human genome.

The program for executing the sequence selection method of the present invention, developed by the present inventors, is capable of finding out novel miRNA genes present on the genome by extracting a desired local secondary structure, preferably a stem-loop structure, through search of genomic DNA sequences in place of an mRNA data set.

Therefore, the present invention also provides a method of detecting a genomic DNA that encodes a pre-miRNA, wherein a genomic DNA search means searches a genomic DNA data set inputted and detects a genomic DNA sequence comprising a sequence capable of assuming a local secondary structure, preferably a stem-loop structure, preserved in the pre-miRNA.

Specifically, for example, variables are set for plus strands and minus strands of Celera Company's total human genome sequence "CHGD_genes_R27f.fasta" and the like, on the basis of stem-loop information obtained from literature information and the like, after which comprehensive search using "multisl.pl" is performed.

The present invention also provides a method of selecting a sequence capable of assuming a particular local secondary structure in a precursor of a known miRNA, for screening for a compound capable of suppressing the production of the matured body from the precursor. The method comprises the following steps:
(1) a step wherein a homology search means searches the base sequence of miRNA inputted in comparison with a genomic DNA data set and maps the miRNA on the genome,
(2) a step wherein a pre-miRNA search means searches genomic DNA data in the vicinity of the miRNA sequence, and extracts a sequence capable of assuming a local secondary structure, preferably a stem-loop structure, which is capable of suppressing miRNA production in the pre-mRNA,
(3) a step for selecting a particular sequence from among the one or more sequences capable of assuming a local secondary structure, extracted in (2) above, and
(4) a step wherein a specificity confirmation means searches an mRNA data set inputted, and confirms that a portion capable of interacting with the compound in the particular sequence selected in (3) above is not present in the region involved in the regulation of the translation of the mRNA.

Here, as the homology search means, commonly known homology search software for nucleic acids (for example, NCBI BLAST (National Center for Biotechnology Information Basic Local Alignment Search Tool) NBLAST and XBLAST program (version 2.0), FASTA program in the GCG software package, and the like) can be used. As examples of the genomic DNA data set, the total human genome data supplied by Celera Company can be used.

Step (2) can be performed in the same manner as step (1) in the above-described sequence selection method for mRNA of the present invention with the data file to be stored in the memory means changed to the genomic DNA data set.

Step (3) is performed by selecting a sequence from among the one or more sequences capable of assuming a local secondary structure, extracted in step (2) which is present at a position capable of significantly inhibiting the cleavage of the pre-mRNA by a dicer. This selection can be performed by visual inspection by the operator, and can also be performed by the central processing unit, which, at command from the control program in the main memory, reads out an appropriate selection rule from the selection rule file stored in a memory means, and automatically selects a particular sequence on the basis of the selection rule, as in step (3) in the above-described sequence selection method for mRNA of the present invention.

Step (4) can be performed in the same manner as step (4) in the above-described sequence selection method for mRNA of the present invention.

Target genes for miRNAs have been elucidated one after another, and miRNAs that regulate the translation of mRNAs involved in a particular disease are already known. As examples of such miRNAs, miR15/miR16 (Calin GA, Dumitru CD, Shimizu M, Bichi R, Zupo S, Noch E, Aldler H, Rattan S, Keating M, Rai K, Rassenti L, Kipps T, Negrini M, Bullrich F, Croce CM. Frequent deletions and down-regulation of micro- RNA genes miR15 and miR16 at 13q14 in chronic lymphocytic leukemia. Proc Natl Acad Sci USA. 2002; 99: 15524-9.), miRNA23 (Kawasaki H, Taira K. Hesl is a target of microRNA-23 during retinoic-acid-induced neuronal differentiation of NT2 cells. Nature, 2003; 423: 838-42), miR-181 (Chen CZ, Li L, Lodish HF, Bartel DP. MicroRNAs modulate hematopoietic lineage differentiation. Science, 2004; 303: 83-6), bic (Tam W, Ben-Yehuda D, Hayward WS. bic, a novel gene activated by proviral insertions in avian leukosis virus-induced lymphomas, is likely to function through its noncoding RNA. Mol Cell Biol. 1997; 17: 1490-502) and the like can be mentioned.

In another aspect, the present invention provides a screening method for a compound that regulates the translation of a particular mRNA. The method comprises the following steps:
(1) a step for searching for mRNA(s) having a sequence, in the molecule, capable of assuming a local secondary structure capable of regulating the translation of the mRNA(s), and
(2) a step for bringing an RNA strand comprising a sequence capable of assuming a particular local secondary structure in the particular mRNA extracted in (1) above and a test compound into contact with each other, and measuring changes in the stability of the secondary structure of the RNA strand.

Here, "capable of regulating the translation" and "a local secondary structure" have the same definitions as those in the above-described sequence selection method for mRNA of the present invention. Preferably, the local secondary structure is a stem-loop structure, more preferably stem-loop A or stem-loop B, still more preferably stem-loop B.

Step (1) can be performed using any technique, as long as it can result in the extraction of one or more mRNAs having one or more sequences, in the molecule, capable of assuming a local secondary structure capable of regulating the translation of the mRNA(s), and it does not always need to be performed via computing using a computer and the like. However, for quickly searching a data set of a large amount of mRNA and extracting an mRNA having a desired local secondary structure, it is desirable that this step (1) be performed in the same manner as step (1) of the above-described sequence selection method for mRNA of the present invention.

Therefore, in a preferred mode of embodiment, the screening method of the present invention comprises bringing an RNA strand comprising a sequence capable of assuming a particular local secondary structure selected by the above-described sequence selection method for mRNA of the present invention and a test compound into contact with each other, and measuring changes in the stability of the secondary structure of the RNA strand.

Although the method of selecting a sequence capable of assuming a particular local secondary structure in a particular mRNA from among the one or more mRNAs extracted in step (1) having one or more sequences, in the molecule, capable of assuming a local secondary structure capable of regulating the translation of the mRNA(s), is also not subject to limitation, and can be preferably performed in the same manner as steps (2) and (3) in the above-described sequence selection method for mRNA of the present invention.

As examples of the RNA strand comprising a sequence capable of assuming a particular local secondary structure in the particular mRNA selected, the mRNA as is, an RNA strand synthesized by a technique known per se on the basis of the sequence information, and the like can be mentioned.

A measurement of changes in the stability of the secondary structure of the RNA strand may be performed using any commonly known method, and can be performed by, for example, the method using a reporter assay system, described below, analysis of RNA structural changes using UV, CD, or NMR, analysis by the in-line probing method, a method comprising measuring the RNase H resistance rate, and the like.

An example of the method of analyzing RNA structural changes is a method comprising adding an appropriate buffer solution comprising an RNA strand to each well of a microplate, further adding a test compound thereto, heating the plate to a temperature that causes the secondary structure of the RNA to be unwound in the absence of the test compound, and performing UV spectral, circular dichroism (CD) spectral or NMR spectral analysis on each well according to a conventional method. A test compound contained in a well showing no spectral changes is selected as a compound that stabilizes the secondary structure in the RNA strand.

As another preferred method of analyzing RNA structural changes, a method utilizing fluorescence resonance energy transfer (FRET) can be mentioned. That is, one terminus of the RNA strand is labeled with a fluorescent substance (e.g., FAM, VIC and the like), the other is labeled with a quenching substance (e.g., TAMRA and the like), and a test compound is added thereto. If the RNA strand is unwound, the fluorescent substance is not influenced by the quenching substance and fluorescence is detected, whereas if the RNA strand assumes a secondary structure, the two termini come close to each other, quenching occurs, and the fluorescence decreases (disappears). Therefore, if the intensity of fluorescence decreases in the presence of a test compound, the test compound can be selected as a compound that stabilizes the secondary structure in the RNA strand.

The choice of test compound is not subject to limitation; for example, proteins, peptides, non-peptide compounds, synthetic compounds, fermentation products, cell extracts, plant extracts, animal tissue extracts and the like can be mentioned, and these compounds may be novel ones or known ones. However, because the screening method of the present invention can be particularly preferably used for screening for a compound more suitable as a pharmaceutical, the test compound is preferably a low-molecular compound, particularly an lipophilic low-molecular compound, specifically a compound having a molecular weight of not more than 500, a ClogP of not more than 5, a hydrogen bond donor number of not more than 5, and a hydrogen bond acceptor number of not more than 10, and the like.

As examples of the method using a reporter assay system, the method described in J. Biol. Chem., 277: 45518-28 (2002) and the like can be mentioned. That is, an expression vector having a DNA corresponding to a sequence capable of assuming a particular local secondary structure in a particular mRNA inserted between a promoter and a first reporter gene, and having an internal ribosome entry site (IRES) arranged downstream of the first reporter gene, and further having a second reporter gene arranged downstream thereof, is prepared, and this is introduced to an appropriate host cell (for example, CHO cell, COS7 cell, HeLa cell, 293 cell and the like) to obtain a transformant. As the reporter genes, known ones in common use can be used; for example, the luciferase, (β-galactosidase, β-glucuronidase, peroxidase, and alkaline phosphatase genes and the like can be mentioned. The promoter is not subject to limitation, as long as it is capable of functioning in the host cell; for example, when an animal cell such as a CHO cell is used, a promoter of an animal- or animal virus-derived constitutive gene can be used. Gene transfection can also be performed using a commonly known method in common use, for example, the lipofection method, PEG method, electroporation method and the like. Analysis is also possible using a stable strain prepared using one of the above-described gene transfection methods.

A test compound is added to the medium of the transformant obtained, and the transformant is cultured under ordinary culture conditions for a given period of time, after which the expression level of each reporter gene is measured. If the test compound interacts with a sequence capable of assuming a particular local secondary structure in a particular mRNA to stabilize the same, the expression of the first reporter gene is suppressed, but ribosome can translate the second reporter gene via IRES. Therefore, if the expression of the first reporter gene decreases whereas the expression of the second reporter gene remains unchanged, the compound is selected as a compound that stabilizes the secondary structure in the RNA strand.

Alternatively, for example, in the case of screening for a compound that specifically interacts with each of a plurality of sequences capable of assuming a local secondary structure to stabilize the secondary structure, an assay is possible without using a special reporter plasmid as described above. That is, a plurality of kinds of plasmids having a DNA corresponding to each sequence capable of assuming a particular local secondary structure inserted between a promoter and a reporter gene are prepared, and these are separately introduced to an appropriate host cell. A test compound is added to each of the media for the plurality of kinds of transformants obtained, and the transformants are cultured under ordinary culture conditions for a given period of time, after which the expression level of the reporter gene in each cell is measured. If the test compound interacts with a sequence capable of assuming a particular local secondary structure in a particular mRNA to stabilize the same, the expression of the reporter gene decreases in the cells transfected with a plasmid comprising the sequence, whereas the expression level remains unchanged in the cells transfected with a plasmid comprising another sequence; therefore, the test compound is selected as a compound that stabilizes the secondary structure of the particular sequence.

In another preferred mode using reporter assay, an assay method using a cell-free translation system can be mentioned. That is, a plasmid having a DNA corresponding to a sequence capable of assuming a particular local secondary structure in a particular mRNA inserted between an appropriate promoter (e.g., T7, T3 promoter and the like) and a reporter gene is prepared, and with this as the template, and using a commonly known cell-free transcription/translation system (for example, those derived from Escherichia coli such as the E. coli S30 extract system (produced by Promega Company) and the RTS 500 Rapid Translation System (produced by Roche Company) can be mentioned; those derived from rabbit reticulocytes such as the Rabbit Reticulocyte Lysate System (produced by Promega Company) can be mentioned; those derived from wheat germ such as PROTEIOSTM (produced by TOYOBO Company) can be mentioned), synthesis of the reporter protein is measured; if the synthesis is suppressed compared to a case wherein a reporter plasmid not comprising the particular sequence is used as the template, the test compound can be selected as a compound that stabilizes the secondary structure of the particular sequence.

Provided that the particular mRNA is an mRNA by regulating the translation of which a particular disease can be prevented/treated, the above-described screening method can be used for screening for a compound capable of preventing/treating the particular disease. The method comprises the following steps:
(1) a step for selecting one or more mRNAs by regulating the translation of which the disease can be prevented/treated,
(2) a step for searching for mRNA(s) having a sequence, in the molecule, capable of assuming a local secondary structure capable of regulating the translation of the mRNA(s) from among the mRNAs selected in (1) above,
(3) a step for selecting a particular sequence from among sequences capable of assuming a local secondary structure in the particular mRNA extracted in (2) above,
(4) a step for confirming that a portion capable of interacting with the compound in the particular sequence selected in (3) above is not present in the region involved in the regulation of the translation of any other mRNA, and
(5) a step for bringing an RNA strand comprising a particular sequence confirmed to be not present in the region involved in the regulation of the translation of any other mRNA in (4) above and a test compound into contact with each other, and measuring changes in the stability of the secondary structure of the RNA strand.

Although steps (1) to (4) can be performed by any method, they are preferably performed in the same manner as the above-described sequence selection method for mRNA of the present invention. The changes in the stability of the secondary structure of the RNA strand in step (5) can be performed using the various methods described above.

The present invention also provides a method of predicting a compound that exhibits an adverse reaction by regulating the translation of mRNA, comprising the following steps:
(1) a step for searching for mRNA(s) having a sequence, in the molecule, capable of assuming a local secondary structure capable of regulating the translation of the mRNA(s), and
(2) a step for bringing an RNA strand comprising a sequence capable of assuming a local secondary structure in the mRNA extracted in (1) above and a test compound into contact with each other, and detecting the presence or absence of an RNA strand having the secondary structure thereof stabilized in the presence of the compound.

In the above-described screening method for a compound that regulates the translation of a particular mRNA, the method does not comprise selecting a particular mRNA and a particular sequence from among one or more sequences capable of assuming a local secondary structure in the one or more mRNAs selected in step (1), but comprises comprehensively synthesizing RNA strands comprising any of all sequences extracted, bringing each RNA strand and a test compound into contact with each other, and detecting the presence or absence of an interaction thereof. In step (2), a method for analyzing RNA structural changes or a method for detecting sensitivity to RNaseH and the like, used in the above-described screening method for a compound that regulates the translation of a particular mRNA, can be preferably used. For example, in the case of structural change analysis using UV spectrometry and the like, an RNA strand comprising each of the sequences extracted in step (1) is dissolved in an appropriate buffer solution, this solution is added to each well of a microplate, a test compound desired to be evaluated for the presence or absence of adverse reactions is further added thereto, the plate is heated to a temperature that causes the secondary structure of the RNA to be unwound in the absence of the test compound, and UV spectral, circular dichroism (CD) spectral or NMR spectral analysis is performed on each well by a conventional method. It can be judged that the secondary structure of a particular sequence contained in the RNA strand in a well showing no spectral changes is stabilized by the test compound, and that the translation of an mRNA comprising the sequence can be regulated by administration of the compound.

As preferable examples of a sequence capable of assuming a local secondary structure capable of regulating the translation of mRNA, that can be obtained by the sequence selection method for mRNA of the present invention, sequences capable of assuming a stem-loop B structure located at the translation initiation codons of the mRNAs that encode Survivin, PKCα, A20, NOTCH1, and NUP, respectively, and the vicinities thereof (sometimes abbreviated Suv SL1, PKC SL1, A20 SL1, NOTCH1 SL1, and NUP SL1, respectively, in this order) (SEQ ID NO:1, 38, 39, 40, 41 in this order) can be mentioned. These mRNAs are important targets in cancer treatment and the like, as suggested by the fact that antisense pharmaceuticals for cancer treatment that target them have been developed. For example, because the Survivin mRNA has a short 5'UTR and has no structure higher than Suv SL1 anywhere including ORF, Suv SL1 is considered to be one of the best local secondary structures as drug innovation targets. Such a local secondary structure can be utilized per se in the above-described screening method of the present invention.

Therefore, the present invention also provides a nucleic acid substantially consisting of the same or substantially the same base sequence as the base sequence shown by any of SEQ ID NO:1 and 38 to 41. Here, "substantially the same base sequence" means a base sequence that has a homology of about 90% or more, preferably about 95% or more, to the base sequence shown by any of SEQ ID NO:1 and 38 to 41 (or has the base sequence shown by any of SEQ ID NO:1 and 38 to 41 wherein one to several bases are substituted, deleted, inserted or added), and is capable of assuming the same stem-loop B structure as the base sequence shown by any of SEQ ID NO:1 and 38 to 41, and has the structure stabilized to a sufficient extent to cause translation suppression by modifying several bases. Also, "substantially consisting" means that a base sequence other than the same or substantially the same base sequence as the base sequence shown by any of SEQ ID NO:1 and 38 to 41 is contained, but this other base sequence has no effect on the above-described properties of the same or substantially the same base sequence as the base sequence shown by any of SEQ ID NO:1 and 38 to 41. The nucleic acid may be RNA or DNA; in the case of DNA, the nucleic acid can be provided in a state in which the DNA can be transcribed to RNA, for example, in a state placed downstream of an appropriate promoter, preferably as an expression vector comprising an expression cassette wherein the nucleic acid is inserted between a promoter and an appropriate gene such as a reporter gene. In this case, because Suv SL1 and the like, for example, have a translation initiation codon in the loop thereof, it is necessary that the downstream gene be joined in a way such that it is translated in-frame if the translation is initiated from the AUG codon.

Furthermore, the present invention also provides the above-described nucleic acid wherein one or more base pairs can be formed in the bubble thereof by mutagenesis. For example, because the sequence of the bubble portion of Suv SL1 is (GC..CCGA), four base pairs with (TCGG..CCGA) are formed by inserting TC in the 5' side of the GC of B sequence, and replacing the C of the GC with G. For example, by preparing a series of variants with the number of base pairs increased one by one, and examining changes in translation suppression, the number of base pairs necessary for the translation suppression (hydrogen bond number) can be known and the ΔG^{TI} value can be estimated. These variants may have the stability thereof improved as required by further removing a mismatch nucleotide from the stem portion, or inserting a base that matches the same.

As described above, the present invention can also be provided as an expression vector comprising an expression cassette wherein the above-described nucleic acid is inserted between a promoter and a reporter gene. As preferable examples of the promoter and the reporter gene, those that can be used in the above-described screening method of the present invention can be mentioned.

The present invention also provides a transformant obtained by transforming an appropriate host cell with the above-described expression vector. The host cell is not subject to limitation, as long as it allows the promoter activity to be exhibited; as preferable examples, those that can be used in the above-described screening method of the present invention can be mentioned.

The present invention provides a screening kit for a compound capable of suppressing the translation of the mRNA that encodes Survivin, which comprises the above-described nucleic acid, expression vector or transformant. The kit can be used by a method in accordance with the above-described screening method of the present invention. The kit may comprise, in addition to the above-described ones, an appropriate buffer solution, medium and the like that are necessary for performing the screening method of the present invention, or other preferable constituents.

The symbols used herein show the following ΔG or ΔΔG values, respectively.

ΔGₘ: ΔG value calculated using mfold. All ΔG values shown herein are ΔG^{us} values (see below).

ΔGₚ: ΔG value calculated using parameters of Genetyx on the basis of a structure searched/outputted using OOPSeqSearch (ΔG, predicted). In most cases, the structure searched is not the optimum structure; therefore, the value obtained is often higher by about 20 to 30% than the value obtained using mfold.

ΔG^{TI}: ΔG value of the structure of mRNA, required to cause translation suppression due to the structure in eukaryotic organisms (ΔG, translation inhibition). This is a function of the position of the structure on the mRNA, temperature, stem-loop structure (particularly bubble appearance) and the like.

ΔG^{US}: ΔG value of mRNA structure under physiological conditions (ΔG, unstabilized). Not stabilized by compound binding and the like.

ΔG^{S}: ΔG value of the structure of mRNA stabilized by compound binding and the like (ΔG, stabilized).

ΔG^{B}: ΔG value of compound binding (ΔG, binding net energy). In literature information, 7 to 10 kcal/mol is common (in DNA).

ΔΔG': ΔΔG value of the difference between ΔG^{US} and ΔG^{S}. Difference in stability to be compensated for by compound binding and the like to obtain a drug effect.

The present invention is hereinafter described in more detail by means of the following Examples, which, however, are not to be construed as limiting the scope of the present invention.

### Examples

### Example 1: Selection of a particular sequence (Survivin SL1) in a particular mRNA (Survivin)

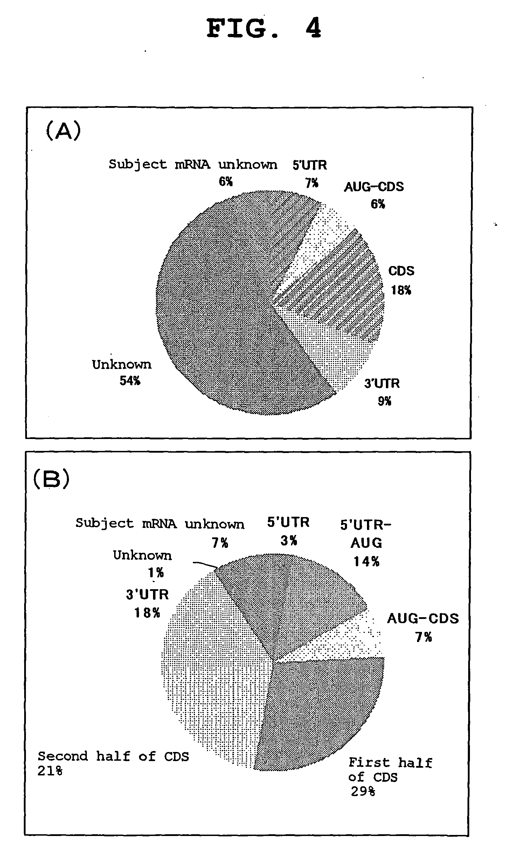

Target mRNAs for antisense pharmaceuticals were found out by a literature and patent survey on antisense pharmaceuticals, and extensive examination was performed to obtain ones for use as target mRNAs for the present invention. Patent information on 291 antisense pharmaceuticals was obtained from Integrity, and base sequences were obtained for 199 thereof. They were applied to BlastN using the Celera Discovery System and H-inv DB; for 161, subject mRNAs were identified. Of them, 135 had cancers as subject diseases; the patent information located the action site in 5'UTR for 10, vicinity of start codon for 8, CDS for 24, 3'UTR for 12, and unknown for 81 (Figure 4A). Furthermore, actual action sites were identified by applying each piece of sequence information to cDNA data by automatic processing (Figure 4B). While analyzing the information, the present inventors took note of the fact that although antisense pharmaceuticals with CDS as the action site accounted for more than 50%, antisense pharmaceuticals for a very narrow site, namely, the vicinity of start codon (140 bases), accounted for 21% (Figure 4B). It is seen that translation suppression is caused due to forming structures in the vicinity of the start codon, which agrees with the mechanism of the present invention. Hence, as the first target, stem-loops present in the vicinity of AUG were selected.

Next, with a focus on 133 target mRNAs with known sequences as anticancer antisense pharmaceuticals, 343 mRNAs (Table 2) were selected. Here, comprehensive search was performed using the RNA secondary structure search program "multisl.pl software (Perl 5.8)", developed by the present inventors. The search was performed for stem-loop B in 117 cases (assuming x + a = 18, y = 4 to 8, and b + c = 4 to 8; 13 different values of "x" from 3 to 15 (accordingly "a" changes from 15 to 3) and 3 different values of each of "i" and "j" from 0 to 2 for each "x" were used; hence, 13 x 3 x 3 = 117 cases). As a result, 50910 (including overlaps) stem-loop B meeting the conditions were discovered.

**Table 2-1**

| Human cDNA sequences corresponding to mRNAs targeted by 133 antisense pharmaceuticals of known sequences as anticancer agents (shown by Celera Discovery ID and RefSeq ID; NO indicates the absence of corresponding RefSeq sequence) | | | | | |
|---|---|---|---|---|---|
| Celera | RefSeq | Celera | RefSeq | Celera | RefSeq |
| hCT201188.2 | NM_004049 | hCT1833851.2 | XM_375224 | hCT1954749.1 | XM_375224 |
| hCT11217.2 | NM_004364 | hCT2271966 | NO | hCT2268568 | NM_198255 |
| hCT2268566 | NM_198255 | hCT1954120.1 | NM_212472 | hCT19614.3 | NM_212472 |
| hCT1954118.1 | NM_212472 | hCT13179.3 | NM_003234 | hCT1965327.1 | NM_003234 |
| hCT2287773 | NM_003234 | hCT16405.2 | NM_213566 | hCT22589.2 | NM_198993 |
| hCT22580.2 | NM_199248 | hCT22591.3 | XM_372668 | hCT2254989 | NM_001011725 |
| hCT2254990 | NM_001011725 | hCT2254988 | NM_001011725 | hCT11514.3 | NM_057182 |
| hCT2256463 | NM_057182 | hCT28273.2 | NM_000095 | hCT2285441 | NM_000095 |
| hCT1641256.1 | NO | hCT2266735 | NM_031157 | hCT18170.3 | NM_003324 |
| hCT15432.2 | NM_001065 | hCT2286539 | NM_001065 | hCT22976.2 | NM_014680 |
| hCT33098.3 | NM_203351 | hCT18953.2 | NM_001168 | hCT1962326.1 | NM_001168 |
| hCT1967440.1 | NM_001168 | hCT2336835 | NM_001168 | hCT2336882 | NO |
| hCT19537.2 | NM_001661 | hCT2278785 | NM_175744 | hCT2278778 | NM_175744 |
| hCT2278786 | NM_175744 | hCT2282183 | NM_001542 | hCT2282184 | NM_001542 |
| hCT1779207.1 | NO | hCT22751.3 | NM_004402 | hCT19627.1 | NM_000201 |
| hCT19653.3 | NM_006202 | hCT19629.3 | NM_007065 | hCT18745.2 | NM_000212 |
| hCT2272752 | NM_000212 | hCT2272750 | NM_000212 | hCT2272751 | NM_000212 |
| hCT6401.3 | NM_181690 | hCT2293797 | NM_181690 | hCT2293796 | NM_181690 |
| hCT2250748 | NM_000657 | hCT2250747 | NM_000657 | hCT25293.4 | NM_000657 |
| hCT2250746 | NM_000657 | hCT6175.2 | NM_002908 | hCT1822951.1 | NM_003108 |
| hCT1963325.1 | NM_003376 | hCT2285904 | NM_003376 | hCT2285909 | NM_003376 |
| hCT2285910 | NM_003376 | hCT2285905 | NM_003376 | hCT30920.4 | NM_032496 |
| hCT2309354 | NM_032496 | hCT2309356 | NM_032496 | hCT2309355 | NM_032496 |
| hCT29713.3 | NM_002466 | hCT29128.3 | NM_005194 | hCT19508.3 | NM_001068 |
| hCT2286519 | NM_001664 | hCT11211.3 | NM_001664 | hCT2289154 | NM_005902 |
| hCT13618.3 | NM_005902 | hCT401223.2 | NM_005239 | hCT2295171 | NM_005239 |
| hCT2295169 | NM_005239 | hCT16106.2 | NM_001982 | hCT2307678 | NM_001982 |
| hCT2307676 | NM_001982 | hCT2307680 | NM_001982 | hCT2311222 | NM_031157 |
| hCT2311223 | NM_031157 | hCT2311227 | NM_031157 | hCT2311225 | NM_031157 |
| hCT2311226 | NM_031157 | hCT2311224 | NM_031157 | hCT2311229 | NM_031157 |
| hCT2311228 | NM_031157 | hCT20312.3 | NM_007106 | hCT2315670 | NM_007106 |
| hCT2315674 | NM_007106 | hCT7807.3 | NM_000838 | hCT22646.3 | NM_138620 |
| hCT1960108.1 | NM_003804 | hCT13972.3 | NM_003804 | hCT1960109 | NM_003804 |
| hCT2250685 | NM_003804 | hCT2250682 | NM_003804 | hCT7420.2 | NM_000597 |
| hCT2313722 | NM_138578 | hCT31269.3 | NM_152854 | hCT1957438.1 | NM_152854 |
| hCT2312895 | NM_152854 | hCT2312891 | NM_152854 | hCT2312893 | NM_152854 |
| hCT30057.3 | NM_138578 | hCT2313724 | NM_138578 | hCT2313721 | NM_138578 |
| hCT1968044.1 | NM_181353 | hCT1968043.1 | NM_181353 | hCT2314450 | NM_080426 |

**Table 2-2**

| Celera | RefSeq | Celera | RefSeq | Celera | RefSeq |
|---|---|---|---|---|---|
| hCT2314077 | NM_080625 | hCT30060.3 | NM_080625 | hCT2314076 | NM_080625 |
| hCT1956032.1 | NM_080426 | hCT1956028.1 | NM_080426 | hCT2314459 | NM_080426 |
| hCT1956027.1 | NM_080426 | hCT1827487.2 | NM_080426 | hCT1950750 | NM_080426 |
| hCT1950751.1 | NM_080426 | hCT1956031.1 | NM_080426 | hCT2314458 | NM_080426 |
| hCT2314443 | NM_080426 | hCT2314438 | NM_080426 | hCT2314452 | NM_080426 |
| hCT2314435 | NM_080426 | hCT22753.3 | NM_002253 | hCT2326453 | NM_002253 |
| hCT2327544 | NM_000584 | hCT7408.2 | NM_000584 | hCT13449.3 | NM_005252 |
| hCT2331264 | NM_006290 | hCT23367.2 | NM_006380 | hCT2266529 | NM_181524 |
| hCT2250895 | NO | hCT2252375 | NM_005124 | hCT2258078 | NO |
| hCT2259480 | XM_372050 | hCT2259481 | XM_372050 | hCT2277743 | NM_001904 |
| hCT18957.3 | NM_001904 | hCT2277746 | NM_001904 | hCT22549.3 | NM_002629 |
| hCT22538.3 | NM_016446 | hCT31740.3 | NM_005484 | hCT1967705.1 | NM_005484 |
| hCT2316340 | NM_031157 | hCT2316339 | NM_031157 | hCT2316337 | NM_031157 |
| hCT2316338 | NM_031157 | hCT2318242 | NM_021138 | hCT1958141 | NM_021138 |
| hCT1958140 | NM_021138 | hCT18775.3 | NM_021138 | hCT2318247 | NM_021138 |
| hCT2318248 | NM_021138 | hCT19541.3 | NM_016520 | hCT18274.1 | NM_022914 |
| hCT1952578.1 | NM_201284 | hCT2259141 | NM_201284 | hCT12368.3 | NM_000228 |
| hCT20429.3 | NM_002737 | hCT2280552 | NM_182763 | hCT2280554 | NM_182763 |
| hCT14045.2 | NM_007121 | hCT2296173 | NM_007121 | hCT2296170 | NM_007121 |
| hCT2296169 | NM_007121 | hCT1952531 | NM_007121 | hCT1961253.1 | NM_006572 |
| hCT1961254.1 | NM_006572 | hCT2262305 | NM_006572 | hCT13651.3 | NM_145185 |
| hCT2290660 | NM_145185 | hCT2290661 | NM_145185 | hCT2290663 | NM_145185 |
| hCT1951272.1 | NM_145185 | hCT19324.2 | NM_004448 | hCT2294818 | NM_004448 |
| hCT20246.3 | NM_152345 | hCT2271790 | NM_152345 | hCT2271789 | NM_152345 |
| hCT2277437 | NM_139049 | hCT2277433 | NM_139049 | hCT2277429 | NM_139049 |
| hCT2277430 | NM_139049 | hCT2277431 | NM_139049 | hCT2277436 | NM_139049 |
| hCT2277435 | NM_139049 | hCT2277438 | NM_139049 | hCT14846.3 | NM_139049 |
| hCT1950805.1 | NM_139049 | hCT22918.3 | NM_004040 | hCT2266410 | NM_004040 |
| hCT1798029.1 | NM_000791 | hCT2308850 | NM_000791 | hCT29166.2 | NO |
| hCT19512.2 | NM_199160 | hCT1829609.2 | NO | hCT2258951 | NM_006540 |
| hCT9508.3 | NM_006540 | hCT2264600 | NO | hCT19464.2 | NM_138712 |
| hCT1817010.1 | NO | hCT12330.2 | NM_002210 | hCT2300428 | NM_002880 |
| hCT19462.3 | NM_002880 | hCT1953375.1 | NM_198437 | hCT30342.3 | NM_198437 |
| hCT1950770 | NM_198437 | hCT1953374.1 | NM_198437 | hCT1965747.1 | NM_198437 |
| hCT1953376.2 | NM_198437 | hCT2311850 | NM_198437 | hCT2311845 | NM_198437 |
| hCT22821.2 | NM_153437 | hCT1644759.2 | NO | hCT24617.3 | NM_006724 |
| hCT22719.2 | NM_017582 | hCT7194.3 | NM_001665 | hCT2273845 | NM_001665 |
| hCT2273843 | NM_001665 | hCT2259143 | NM_201284 | hCT1950009.1 | NM_006724 |
| hCT2318240 | NM_021138 | hCT23504.3 | NM_003821 | hCT2295836 | NM_203351 |
| hCT2254987 | NM_001011725 | hCT1956029.1 | NM_080426 | hCT1957437.1 | NM_152854 |
| hCT2311219 | NM_031157 | hCT2336837 | NM_001168 | hCT2307681 | NM_001982 |
| hCT2273848 | NM_001665 | hCT28187.3 | NM_016270 | hCT18460.2 | NM_024426 |
| hCT14945.3 | NM_182626 | hCT2290980 | NM_002210 | hCT2275112 | NM_138712 |

**Table 2-3**

| Celera | RefSeq | Celera | RefSeq | Celera | RefSeq |
|---|---|---|---|---|---|
| hCT1960916.1 | NM_006572 | hCT2280551 | NM_182763 | hCT2318245 | NM_021138 |
| hCT2252377 | NM_005124 | hCT2250896 | NO | hCT7828.3 | NM_006290 |
| hCT2326452 | NM_002253 | hCT6218.2 | NM_001134 | hCT1956030.1 | NM_080426 |
| hCT2314454 | NM_080426 | hCT28373.3 | NM_181353 | hCT2323737 | XM_370982 |
| hCT2311220 | NM_031157 | hCT2286520 | NM_001664 | hCT10063.3 | NM_003376 |
| hCT2336738 | NM_000201 | hCT2282185 | NM_001542 | hCT1958629.1 | NM_001168 |
| hCT1965835 | NM_057182 | hCT2252047 | NO | hCT28562.3 | NM_181524 |
| hCT29147.3 | NM_005225 | hCT2313231 | NM_005225 | hCT1969180 | NM_024426 |
| hCT1969179.1 | NM_024426 | hCT1823775.1 | NM_024426 | hCT2268156 | NM_145803 |
| hCT2268158 | NM_145803 | hCT2268155 | NM_145803 | hCT2267461 | NM_182626 |
| hCT22623.4 | NM_030665 | hCT27983.4 | NM_001949 | hCT2251810.1 | NM_001949 |
| hCT1970790.1 | NO | hCT1952441.2 | NM_001949 | hCT2274764 | NM_001071 |
| hCT2274763 | NM_001071 | hCT2343740 | NO | hCT2308523 | NM_005269 |
| hCT2263104.1 | NM_182641 | hCT2343416 | NM_001071 | hCT2263098.1 | NM_182641 |
| hCT2274765 | NM_001071 | hCT2279450 | NM_006496 | hCT2306992 | NM_006882 |
| hCT2307001 | NM_006882 | hCT2306997 | NM_006882 | hCT2306995 | NM_006882 |
| hCT2307002 | NM_006882 | hCT2307004 | NM_006882 | hCT2307005 | NM_006882 |
| hCT2307009 | NM_006882 | hCT2307014.1 | NM_006882 | hCT2307011 | NM_006882 |
| hCT1963326.1 | NM_003376 | hCT22667.3 | NM_016040 | hCT2277439 | NM_139049 |
| hCT2297651.1 | NM_002467 | hCT2297650.1 | NM_002467 | hCT6947.2 | NM_002467 |
| hCT2348021 | NM_002467 | hCT2253836.1 | NM_001167 | hCT1953707.1 | NM_001167 |
| hCT1967493.2 | NM_001167 | hCT5866.3 | NM_001167 | hCT31091.4 | NM_000684 |
| hCT22973.4 | NM_177402 | hCT16404.4 | NM_002247 | hCT22937.3 | NM_017865 |
| hCT23004.3 | NM_174887 | hCT19691.2 | NM_004941 | hCT2292881.1 | NO |
| hCT2350480 | NM_004091 | hCT2256381 | NM_004091 | hCT28817.3 | NM_004091 |
| hCT2350481 | NM_004091 | hCT2350412 | NO | hCT2350562 | NM_172175 |
| hCT2350564 | NM_172175 | hCT1966588.1 | NM_172175 | hCT1966585.2 | NM_172175 |
| hCT2350563 | NM_172175 | hCT2297386 | NO | hCT22909.3 | NO |
| hCT1782979.2 | NO | hCT2321107 | XM_208373 | hCT1971122.2 | NO |
| hCT2345413.1 | NO | hCT2351135 | NM_145687 | hCT1955205.1 | NM_145687 |
| hCT2306239.1 | NM_145687 | hCT1962990.2 | NM_145687 | hCT1640566.3 | NM_145687 |
| hCT1955204.2 | NM_145687 | hCT2256476.1 | NM_134447 | hCT2256475.1 | NM_134447 |
| hCT1955256.2 | NM_134447 | hCT13206.4 | NM_134447 | hCT1955257.2 | NM_134447 |
| hCT1640468.3 | NM_014812 | | | | |

The search results obtained were analyzed using the RNA secondary structure analysis program "Licorice software (Perl 5.8, HTML, JavaScript (registered trademark))", developed by the present inventors. Condition "stem-loop B is present in the AUG region, with no mismatch in the stem A portion thereof" was set, and mRNAs meeting these conditions were selected. 143 such mRNAs were found, which were derived from 22 genomes. Selected from among them were those targeted by antisense pharmaceuticals at the preclinical stage or beyond; hCG27811, targeted by the antisense pharmaceutical ISIS-23722, and hCG1999404, hCG1979965, and hCG37306, targeted by ODNas750/15, were found. Here, it was realized that the mRNAs targeted by ODNas750/15 were derived from a plurality of genomes, and the biological significance thereof remained unknown; hence, hCG27811, targeted by ISIS-23722, was first selected.

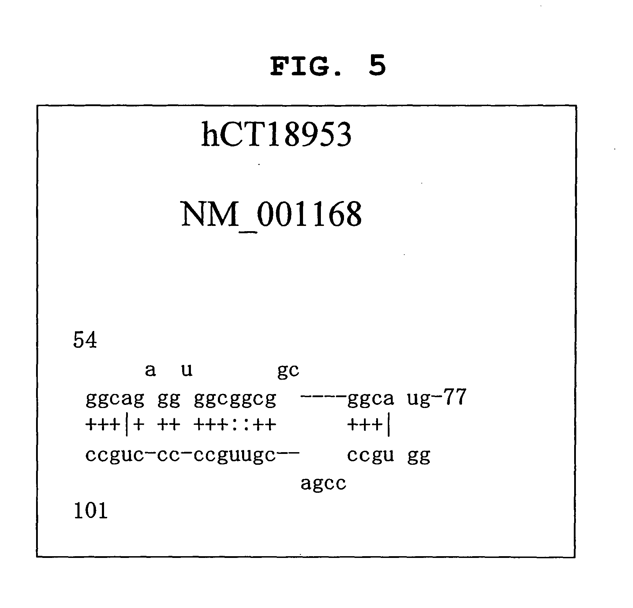

The mRNAs corresponding to hCG27811 are hCT18953, hCT1962326, hCT2336837, hCT1967440, hCT1958629, and hCT2336835 (although there is another mRNA corresponding to hCG27811, namely hCT1962327, the above-described search was not performed since the evidence level was low); in all transcription products, stem-loop B (ΔG predicted by multisl.pl was -24.83 kcal/mol) was present in a form spanning AUG. Figure 5 shows the stem-loop B that appears in hCT18953. This stem-loop was found to maintain this form in both itself (from 54 to 103; each numerical value indicates the position of the structure with the 5' terminus of hCT18953 denoted as position 1; the same applies below) and in 100 nucleotides in the vicinity thereof (from 29 to 128), as a result of an analysis using the commonly known RNA secondary structure prediction program "mfold" (GCG Software) (ΔG predicted using mfold was -25.8 kcal/mol). Since literature information also supported the primary sequences of these transcription products (Refseq: NM_001168), this motif was named Survivin Stem Loop 1 (Suv SL1), and drug discovery for this was attempted.

### Example 2: Prediction of adverse reactions arising from a compound capable of binding selectively to Suv SL1

As the portion that interacts with a low-molecular compound in stem-loop B, the bubble portion, as well as the loop portion (and other portions in some cases) are likely; however, in the case of the stem-loop B obtained in Example 1 [Survivin Stem Loop 1 (Suv SL1)], the low-molecular compound binding portion is judged to be the bubble portion because the loop is relatively stable, and also because the stability of the stem-loop is reduced mainly by the bubble. If a stable stem-loop of exactly the same bubble structure is present on another mRNA, serious adverse reactions are of concern. Hence, similar-structure search for all cDNA data possessed by the present inventors (subjects were the 62936 cDNAs in hCT_R27g_CDS3.fasta), with a focus on stem-loop B having the same bubble portion, was performed using the multisl.pl program. The structure search was performed under the following conditions 1 to 3:

| (Conditions 1) | |
|---|---|
| Length of stem A and stem B (x + a): 16 (but each of "x" and "a" is 3 or more) | 11 cases |
| Sequence of B sequence: GGCG | |
| Sequence of B' sequence: CCCGAC | |
| Mismatches (i, j): Each 0 to 2 | 9 cases |
| Length of loop (y): 4 to 20 | |
| | In total 99 cases |

| (Conditions 2) | |
|---|---|
| Length of stem A and stem B (x + a): 14 (but each of "x" and "a" is 3 or more) | 9 cases |
| Sequence of B sequence: GGCG | |
| Sequence of B' sequence: CCCGAC | |
| Mismatches (i, j): Each 0 to 2 | 9 cases |
| Length of loop (y): 4 to 20 | |
| | In total 81 cases |

| (Conditions 3) | |
|---|---|
| Length of stem A and stem B (x + a): 18 (but each of "x" and "a" is 4 or more) | 11 cases |
| Sequence of B sequence: GC | |
| Sequence of B' sequence: CCGA | |
| Mismatches (i, j): Each 0 to 2 | 9 cases |
| Length of loop (y): 4 to 20 | |
| | In total 99 cases |

Here, the bubble sequence differs between search conditions 1 and 2 (GGCG..CCCGAC) and 3 (GC..CCGA). Since base pairs involved in stem formation at an end of a loop or bubble are often important to the structure of the loop or bubble, it is necessary to perform search with a sequence comprising those base pairs deemed as a bubble portion in order to find a truly similar bubble structure.

In accordance with the conditions shown above, search was performed using a multisl.pl program written in Perl 5.8 language (and HTML, JavaScript (registered trademark)). When a UNIX (registered trademark) computer (Precision 360 [3.40 GHz Pentium (registered trademark) 4, 1.0 GB memory], Miracle 3.0, Perl 5.8) was used, each calculation was completed within 1 to 2 days.

Since the search results involved a total of about 2000 files with a volume of about 12 GB, they were stored in a web server (Think Pad R40e [2.20 GHz Pentium (registered trademark) 4, 512 MB memory, 35 GB HD], Windows (registered trademark) 2000 Service Pack 3, Apache HTTP Server 2.0.49, ActiveState ActivePerl 5.8). The results obtained under the respective search conditions are shown below.

Since serious adverse reactions are of particular concern if there is a stable stem-loop of exactly the same bubble structure as Suv SL1, search was performed using conditions 1. Search was performed in 99 cases; as a result, 37 structures were found (21 in 5'UTR, 8 in CDS, 8 in 3'UTR). Of these, 21 came from Suv SL1 per se, and those found in CDS and 3'UTR came from one and two transcription products, respectively (one genome). It was confirmed that no structure was present, other than the targeted stem-loop, in 5'UTR and the vicinity of AUG, where it is considered that translation suppression occurs effectively.

The transcription products found using conditions 1 are shown below. The ΔGp values shown below are neither actual measured values nor values obtained using mfold, but are ΔG values predicted using multisl.pl. If a structure to be predicted is actually present, its actual ΔG value is often lower than this predicted value.
1) Survivin SL1A1 ΔGₚ = -19.65 kcal/mol; ΔGₘ = -25.2 kcal/mol CDS (1186-1233)
   hCT1640575 (hCG1640448)
2) Survivin SLIA2 ΔGₚ = -14.73 kcal/mol 3'UTR (1201-1261) hCT2287046 (hCG17893)
3) Survivin SLIA3 ΔGₚ = -14.73 kcal/mol 3'UTR (1858-1918) hCT8947 (hCG17893)
   hCT1640575 is a cDNA corresponding to Refseq: NM_001004302 (GenBank Description: Homo sapiens hypothetical protein LOC155060 (LOC155060), mRNA.), and hCT2287046 and hCT8947 are cDNAs corresponding to RefSeq: NM_003277 (GenBank Description: Homo sapiens claudin 5 (transmembrane protein deleted in velocardiofacial syndrome) (CLDN5), mRNA). Survivin SL1A1 (hCT1640575) is considered to have nearly the same bubble structure as Suv SL1, and mfold also supports the retention of this stem-loop B structure (ΔGₘ = -25.2 kcal/mol; if 1185-position C and 1234-position G are added, this intensifies to ΔGₘ = -26.9 kcal/mol). It is reasonably likely that a drug that targets Suv SL1 exhibits an adverse reaction on this portion. However, when mfold is applied to a total of 100 bases in the vicinity of this motif, this stem B of Survivin SL1A1 interacts preferentially with another region, resulting in a reduction of the total number of stem base pairs of Survivin SL1A1 to 15 (ΔGₘ = -22.8 kcal/mol). Furthermore, the position of this Survivin SL1A1 is not in the vicinity of AUG but in the latter half of CDS. Because the findings that have been obtained to date suggest that the translation suppressive effect in CDS may be low, it is anticipated that the translation suppression by this Survivin SL1A1, if any, would not be serious as an adverse reactions. The function of the protein encoded by this hCT1640575 has not yet been analyzed.

On the other hand, regarding Survivin SL1A2 (hCT2287046) and Survivin SL1A3 (hCT8947), the results obtained by processing with mfold confirmed that they retain no bubble structure, that their ΔG values are low because of the large loop, and that they are located in a region known as 3'UTR, which is relatively irrelevant to translation suppression. For this reason, it seems unlikely that adverse reactions occur due to the structure observed in these two.

In the same manner as described above, search was performed using conditions 2 on whether or not there is a stem-loop of exactly the same bubble structure as Suv SL1 and is stable to some extent. In this case, even if the number of stems is small, all ones having sufficiently low ΔG can be detected without omission. On the other hand, there should be ones that have high ΔG and are detected in the search. Theoretically, there should be a number of hits not less than 16 times that obtained under conditions 1. Search was performed for 81 combinations; as a result, 57 structures were found (28 in 5'UTR, 19 in CDS, 10 in 3'UTR). Of these, 21 came from Surv SL1 per se, 18 came from Survivin SL1A1, 5 cane from Survivin SLIA2, and 5 came from Survivin SLIA3. This search also confirmed that no structure is present except for targeted Suv SL1 in 5'UTR and the vicinity of AUG, where it is considered that translation suppression occurs effectively. As a result, those newly found under conditions 2 came from four cDNAs and three genomes.
1) Survivin SL1A4 ΔGp = -10.55 kcal/mol; ΔGₘ = -20.20 kcal/mol CDS (578-621)
   hCT14517 (hCG23410)
2) Survivin SL1A5 ΔGp = -16.18 kcal/mol; ΔGₘ = -35.5 kcal/mol 5'UTR (379-439)
   hCT18284 (hCG27150)
3) Survivin SL1A6 ΔGp = -1.0 kcal/mol 5'UTR (94-156)
   hCT2321009 (hCG1741805)
4) Survivin SL1A7 ΔGp = -1.00 kcal/mol 5'UTR (94-156)
   hCT1779961 (hCG1741805)

hCT14517 is a cDNA corresponding to Refseq: NM_006602 (GenBank Description: Homo sapiens transcription factor-like 5 (basic helix-loop-helix) (TCFL5), mRNA.), hCT18284 is a cDNA corresponding to Refseq: NM_016353 (GenBank Description: Homo sapiens zinc finger, DHHC domain containing 2 (ZDHHC2), mRNA.) and hCT2321009 and hCT1779961 are cDNAs corresponding to RefSeq: NM_017650 (GenBank Description: No entry).

None of the motifs was sufficiently stable, and the results obtained using mfold were individually checked; none conferred the same bubble structure as Suv SL1. Regarding Survivin SL1A5 to Survivin SL1A7, the loop is too large and stability is lacked.

Under conditions 3, an attempt was made to find a larger number of similar stem-loops with a shorter length of the structure of the bubble portion (GC..CCGA). Thus, 533 (breakdown: 147 in 5'UTR, 279 in CDS, 107 in 3'UTR; including 127 stem-loops in the vicinity of AUG) stem-loops having similar bubbles were discovered.

Here, the search results obtained under conditions 3 were evaluated. When 387 cDNAs found to contain 426 stem-loops excluding those present in 3'UTR were examined one by one, it was found that they originated from 78 genomes. Of the 77 genes excluding the target mRNA having Suv SL1, only Survivin SL1A1 (hCT1640575; described above) maintained the bubble structure as is even when subjected to mfold. Also, Survivin SL1A8 (hCT2311028; hCT2311031; hCT1951006; hCT2311029) maintains a similar bubble structure, GCA..CCGA, and has a stem-loop structure of ΔGₘ = -31.2 kcal/mol. Likewise, Survivin SL1A9 (hCT1960516; hCT2264998) maintains a similar bubble structure, GCU..CCGA, and has a stem-loop structure of ΔGₘ = -21.3 kcal/mol. Among the hit compounds obtained by subsequent compound screening, there may be some that also interacts with this bubble portion. However, even in this case, it is considered to be possible to avoid adverse reactions by taking appropriate measures in synthesis because there is a difference in bubble structure from Suv SL1.

As described above, it is considered that whatever conditions are used for the search, it is little feared that serious adverse reactions are caused by a low-molecular compound created for Suv SL1.

In the search process under conditions 3, very potent stem-loops Survivin SL1A10 (hCT1784311) and Survivin SL1A11 (hCT2309121; hCT235067) were found. Because these are present in CDS or 5'UTR, it is seen that the ΔG^{TI} in these regions is not more than this value.

Characteristic stem-loops found by the search using conditions 3 are shown below.
1) Survivin SL1A8 ΔGₘ = -31.2 kcal/mol (bubble: GCA..CCGA) CDS or 5'UTR
   hCT2311028; hCT2311031; hCT1951006; hCT2311029 (hCG1810991)
   Refseq: NM_0015080; NM_138732; NM_138734
   Neurexin 1 or 2 (NRXN2)
2) Survivin SL1A9 ΔGₘ = -21.3 kcal/mol (bubble: GCU..CCGA) 5'UTR hCT1960516; hCT2264998 (hCG1817936)
   Refseq: NM_024600
   Chromosome 16 open reading frame 30 (C16orf30); hypothetical protein (FLJ20898)
3) Survivin SL1A10 ΔGₘ = -33.9 kcal/mol CDS
   hCT1784311 (hCG1746033)
   Refseq: NM_006161
   Neurogenin
4) Survivin SL1A11 ΔGₘ = -36.6 kcal/mol 5'UTR
   hCT2309121; hCT235067 (hCG1782075)
   Refseq: NM_002834
   Protein tyrosine phosphatase, non-receptor type 11 (Noonan syndrome 1)

### Example 3: Translation suppression by stable stem-loop (SL31)

SL31, an artificial sequence improved with reference to a sequence that exhibited translation suppression in a stable stem-loop (Kozak M. Influences of mRNA secondary structure on initiation by eukaryotic ribosomes. Proc Natl Acad Sci USA. 1986. 83. 2850-4) (free energy: -55.8 kcal/mol), was inserted to the 5'-UTR of a reporter gene, and the effect thereof on the translation activity of the gene was examined.

### (1) Construction of expression vector

A sense oligomer (5'-aagttcaccacggcccccaagctt; SEQ ID NO:2) and an antisense oligomer (5'-aagttcaccacggcccccaagctt; SEQ ID NO:3) were synthesized (Hokkaido System Science). A cDNA was prepared with ExTaq polymerase (TAKARA) using these oligomers, and the cDNA was inserted to pTOPO-XL (Invitrogen) (pSL31). Subsequently, an Eco RI fragment comprising the stem-loop was cut out from pSL31, and inserted to the Eco RI site of pBluescript II SK+ (pSL31E). The orientation and sequence of the stem-loop were confirmed by sequencing.

pEGFP/SL31/IC was prepared by cutting out an Eco RV and Bam HI fragment comprising the stem-loop from pSL31E, blunting the Nhe I site, and inserting the fragment to Bgl II-treated pEGFP-N1 (CLONTECH). pEGFP/SL31/IS was prepared by cutting out an Xho I and Pst I fragment comprising the stem-loop from pSL31E, and inserting the fragment to the Xho I and Pst I site of pEGFP-N1. pEGFP/SL31/IT was prepared by cutting out an Eco RV and Sac II fragment comprising the stem-loop from pSL31E, and inserting the fragment to the Sma I and Sac II site of pEGFP-N1.

pEGFP/SL31/IC is designed to arrange the stem-loop structure (SL31) just under the transcription initiation point of the pEGFP-N1 reporter plasmid (the stem-loop is expected to begin at the fifth nucleotide of the 5'-cap), and is expected to suppress the 5'-cap recognition reaction by ribosome. pEGFP/SL31/IS is designed to arrange the stem-loop structure (SL31) in the middle of the 5'-cap and translation initiation codon of the pEGFP-N1 reporter plasmid, and is expected to inhibit the AUG codon scanning reaction by ribosome. pEGFP/SL31/IT is designed to arrange the stem-loop structure (SL31) at 35 nt upstream of the translation initiation codon (AUG) of the pEGFP-N1 reporter plasmid, and is capable of inhibiting the formation of the ternary complex necessary for initiation of the translation occurring on the translation initiation codon by steric hindrance.

### (2) CHO cell culture and gene transfection/translation activity measurements

CHO cells were suspended in Nucleofector Solution T (amaxa: cat no. 507-99031), the test plasmids constructed in (1) above and control plasmid (pGL3-control (Promega; including the luciferase gene); 0.5 µg) were mixed, and gene transfection was performed using Nucleofector on the A-23 program. After the gene-transfected cells were cultured for 24 hours, the culture broth was removed, Steady-Glo Luciferase Assay System (Promega: cat no. E2520) was added to lyse the cells, and fluorescence values (excitation wavelength/absorption wavelength = 485 nm/535 nm) and subsequently luminescence values were measured by using Wallac Arvo SX.

Quantitation of mRNA was performed as described below. Total RNA was recovered from the gene-transfected cells, and cDNA was prepared using TaqMan Gold RT-RCR Reagents (N808-0232, Applied Biosystems). Subsequently, TaqMan analysis on firefly luciferase, EGFP, and GAPDH was performed using the cDNA prepared. The mRNA copy numbers of the luciferase and EGFP genes were calculated as ratios to the GAPDH gene mRNA content analyzed using TaqMan GAPDH Control Reagents (Part Number: 402869, Applied Biosystems). The primers and TaqMan probes for the luciferase and EGFP genes are shown in Table 3.

**Table 3**

| Primers and probes (purchased from Applied Biosystems) | | | |
|---|---|---|---|
| Gene | Primer 1 | Primer 2 | Probe |
| Luciferase | AGGTCCTATGATTA TGTCCGGTTATG (SEQ ID NO:4) | GAATGTAGCCATCC ATCCTTGTC (SEQ ID NO:5) | AAACAATCCGGAAGCG ACCAACGC (SEQ ID NO:6) |
| EGFP | CTGACCCTGAAGTT CATCTGCA (SEQ ID NO:7) | GAAGAAGTCGTGCT GCTTCATGT (SEQ ID NO:8) | CCACCCTGACCTACGG (SEQ ID NO:9) |

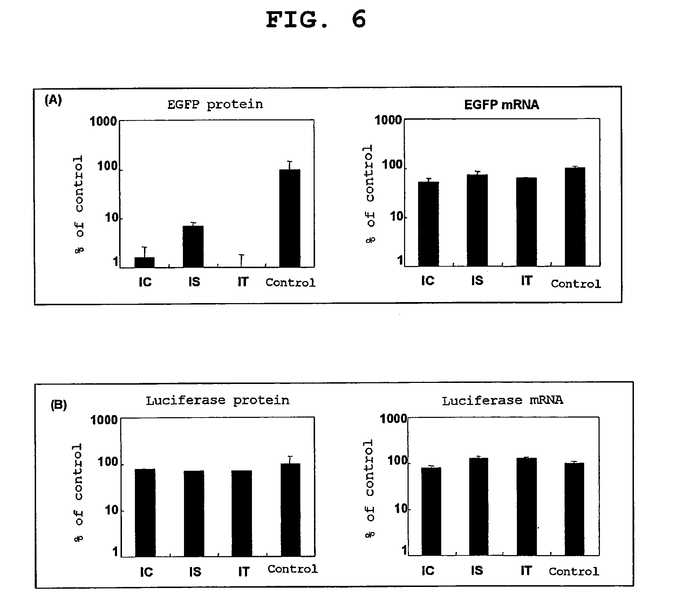

The results are shown in Figure 6. The EGFP protein expression levels from the plasmids having SL31 inserted to the 5'UTR thereof were 1.64% for pEGFP/SL31/IC, 7.00% for pEGFP/SL31/IS, and 0.98% for pEGFP/SL31/IT, compared to the EGFP protein expression level from pEGFP-N1, all showed a remarkable reduction (Figure 6A left). The EGFP mRNA contents were determined to be 52.9% for pEGFP/SL31/IC, 73.0% for pEGFP/SL31/IS, and 62.5% for pEGFP/SL31/IT, showing slight reductions, but the reductions were smaller than those in translation levels (Figure 6A right). Regarding firefly luciferase protein contents (Figure 6B left) and mRNA contents (Figure 6B right), no significant change was observed in any sample.

From these results, it was suggested that when SL31, which is expected to assume a stable stem-loop structure, is inserted to 5'UTR, the translation activity decreases. Furthermore, the EGFP expression levels obtained with SL31 inserted to pEGFP/SL31/IT and pEGFP/SL31/IC were more potently suppressed, compared to the EGFP expression levels obtained with SL31 inserted to pEGFP/SL31/IS. From these results, it was postulated that a stem-loop expected to inhibit the 5'-cap recognition reaction by ribosome, or the formation of a ternary complex necessary for initiation of the translation, caused a remarkable reduction in the translation. From this fact, it is considered that the target stem-loop is preferably selected near the 5'-cap or near the AUG initiation codon. However, because it is difficult to completely identify the 5' terminus of mRNA, it is considered to be easier to select a target near the AUG initiation codon. Regarding the inhibition of the formation of the ternary complex taking place on the AUG initiation codon, because the radius of ribosome is estimated to be about 40 nt, the stem-loop formation starting point is considered to be within 40 bases.

### Example 4: Apoptosis induction in cells having Survivin mRNA knocked down by RNAi

Suv SL1, obtained in Example 1, is a relatively stable stem-loop structure having the AUG initiation codon of Survivin mRNA in the loop thereof, wherein the ΔG value predicted using multisl.pl is -25.8 kcal/mol.

Survivin is a 16.5-kDa protein known as the least inhibitor of apoptosis (IAP). Survivin is known to have two functions, and is known as a factor involved in apoptosis suppression and cell division (Li et al., 1998; Ambrosini et al., 1998). Regarding apoptosis suppression, Survivin has been shown to be involved in the regulation of the activities of caspase-3 and caspase-7, and is considered to suppress the activities of these apoptosis execution factors (Li et al., 1998). Regarding cell division, Survivin has been shown to bind to spindle microtubules and Aurora B kinase, and has been reported to be involved in the regulation of the G2/M phase of the cell cycle (Bolton et al., 2002).

Although Survivin is widely expressed in juvenile tissue, almost no expression is observed in normal tissue of adults. In fact, the present inventors confirmed that when expression analysis was performed by e-northern with Gene Express (Gene logic Company), almost no expression was observed in normal tissue of adults. However, Survivin has been found to be expressed selectively and widely commonly in cancer cells in the lung, large intestine, pancreas, breast, prostate and the like (Nasu et al. 2002; Tamm et al. 1998). Furthermore, it is known that by introducing an antisense nucleic acid that suppresses expression, cell proliferation suppression and apoptosis are induced (Beltrami et al., 2004).

Hence, the present inventors performed expression suppression on Survivin with RNAi in HeLa cells to confirm that apoptosis is induced by Survivin suppression, and performed apoptosis detection with mitochondrial membrane collapse as an index using Depsipher (TM). Details are shown below.

### (1) HeLa cell culture and RNAi

HeLa cells were suspended in Nucleofector Solution R (amaxa: cat no. 500-99021). The cell suspension and siRNA (SuperArray Bioscience Corporation: cat no. 0212-37-2000) were mixed, and transfection was performed using Nucleofector on the 1-13 program. After the siRNA-transfected cells were cultured for 48 hours, total RNA was recovered, and cDNA was prepared using TaqMan Gold RT-RCR Reagents (Applied Biosystems: cat no. N808-0232). After RT-PCR was performed using a Survivin detection primer (SuperArray Bioscience Corporation: cat no. 0212-37-2000) or a GAPDH detection primer (TaqMan GAPDH Control Reagents (Applied Biosystems: cat no. 402869), electrophoresis was performed, and each mRNA content was detected.

Apoptosis detection was performed per the protocol attached to Depsipher (TM) as described below. After the siRNA-transfected cells were cultured for 48 hours, the culture broth was removed and replaced with a reaction buffer comprising a stabilization solution, and the cells were washed. Subsequently, a reaction buffer comprising Depsipher (TM) was added, and the mixture was allowed to stand at room temperature for 15 minutes. The reaction buffer comprising Depsipher (TM) was removed, and the cells were washed with a reaction buffer, after which the cells were examined using a fluorescence microscope.

The results are shown in Figure 7. Survivin mRNA decreased by not less than 90% in the RNA extracted from the Survivin siRNA-transfected cells, compared to the RNA extracted from the control siRNA-transfected cells. On the other hand, the abundance of GAPDH mRNA in the Survivin siRNA-transfected cells decreased only slightly (Figure 7A).

When the cells at 48 hours after Survivin siRNA transfection were examined using an inverted fluorescence microscope, a morphological change into spherical form was observed compared to the group of the control siRNA-transfected cells, and a large number of cells were floating. Furthermore, when the appearance of mitochondrial membrane collapse was examined with the addition of Depsipher (TM), green fluorescence, which indicates mitochondrial membrane collapse, was little observed, and red fluorescence, which indicates viable cells, was observed, in the control siRNA-transfected cells. On the other hand, in the group of Survivin siRNA-transfected cells, most cells exhibited green fluorescence; it was suggested that apoptosis was being induced (Figure 7B).

### Example 5: Stabilization of stem-loop structure by mutagenesis in Suv SL1

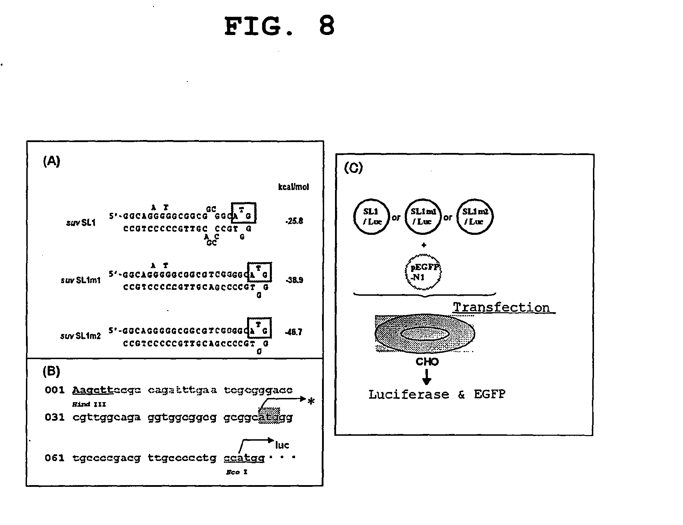

To determine whether or not a potent translation suppressive effect can be obtained by stabilizing Suv SL1, reporter plasmids with firefly luciferase having Suv SL1m1 and Suv SL1m2 structures mutated to stabilize Suv SL1 were prepared (Figure 8A). The reporter was cloned into the Hind III/Nco I site of the pGL3-control plasmid (Promega) in a way such that the full length of the 5'UTR and 27 bases of CDS of Survivin would be contained (Figure 8B). All the pGL3-control-derived 5'UTRs were replaced with the Survivin-derived 5'UTR. Because the reporter had been constructed not to break the translation frame of firefly luciferase, and not to introduce a mutation in the CDS portion, reporter analysis with firefly luciferase can be performed. Suv SL1m1 eliminated the bubble structure observed in Suv SL1 by 2 bases insertion (TC) and 1 base substitution (C→G) to stabilize the stem-loop (free energy: -38.9 kcal/mol). Furthermore, Suv SL1m2 removed the mismatches (A and T) on the stem B of Suv SL1m1 to stabilize the stem-loop (free energy: - 46.7 kcal/mol). These plasmids and pEGFP-N1, as a control, were transfected to CHO cells simultaneously in the same protocol as Example 3(2), and the luciferase activity and EGFP activity were measured (Figure 8C).

The pGL3/Suv SL1, pGL3/Suv SL1m1, and pGL3/Suv SL1m2 plasmids were constructed as described below. A sense oligomer (final concentration 12.5 µM) and an antisense oligomer (final concentration 12.5 µM) were mixed in annealing buffer, and the mixture was incubated at 75°C for 5 minutes, after which it was incubated at room temperature for 5 minutes or more. The annealed oligomer (125 nM) was cloned using the TOPO-XL cloning kit. After confirmation of the base sequences by sequencing, a fragment containing Suv SL1, Suv SL1m1, or Suv SL1m2 was inserted to the Hind III/Nco I site of the pGL3-control plasmid (Promega). Finally, the base sequences were confirmed by sequencing. The sense oligomers and antisense oligomers used are shown in Table 4 (in the Table, each uppercase letter indicates the 5' terminus).

**Table 4**

| | Sense oligomer | I Antisense oligomer |
|---|---|---|
| Suv SL1 | Aagcttccgccagatttgaatcgcgg gacccgttggcagaggtggcggcggc ggcatgggtgccccgacgttgccccc tgccatgga (SEQ ID NO:10) | Ccatggcagggggcaacgtcggggca cccatgccgccgccgccacctctgcc aacgggtcccgcgattcaaatctggc ggaagctta (SEQ ID NO:11) |
| Suv SL1m1 | Aagcttccgccagatttgaatcgcgg gacccgttggcagaggtggcggcgtc ggggcatgggtgccccgacgttgccc cctgccatgga (SEQ ID NO:12) | Ccatggcagggggcaacgtcggggca cccatgccccgacgccgccacctctg ccaacgggtcccgcgattcaaatctg gcggaagctta (SEQ ID NO:13) |
| Suv SL1m2 | Aagcttccgccagatttgaatcgcgg gacccgttggcagggggcggcgtcgg ggcatgggtgccccgacgttgccccc tgccatgga (SEQ ID NO:14) | Ccatggcaggggcaacgtcggggca cccatgccccgacgccgccccctgcc aacgggtcccgcgattcaaatctggc ggaagctta (SEQ ID NO:15) |

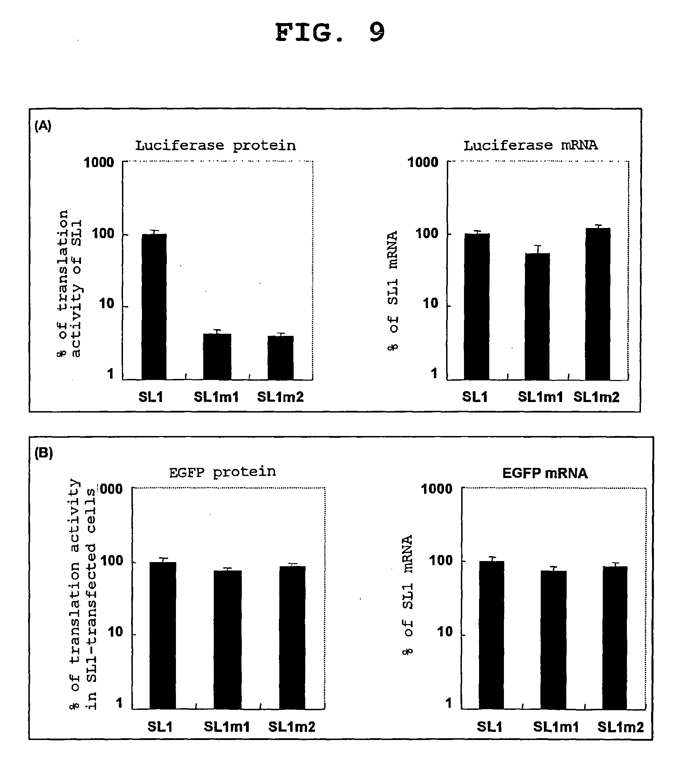

The results are shown in Figure 9. The luciferase translation levels from the plasmids having Suv SL1m1 or Suv SL1m2 decreased to 1/10 or less compared to the plasmid having Suv SL1 (Figure 9A left). On the other hand, the EGFP translation levels did not show a remarkable reduction in any cells transfected with plasmids (Figure 9B left). No major change was observed in any mRNA quantities (Figure 9A right and B right). From these results, it was suggested that a potent translation suppressive effect might be obtained by stabilizing Suv SL1. The energy of compound required for the translation suppression at that time is at least -13.1 kcal/mol as calculated from the energy difference between Suv SL1 and Suv SL1m1; it can be concluded that if a similar level of energy is added by a compound, the translation from the Survivin gene mRNA would be remarkably suppressed.

### Example 6: Stabilization of stem-loop structure by mutation of various stem-loops

To confirm the generality that a potent translation suppressive effect can be obtained by stabilizing a stem-loop as observed in Suv SL1, the same experiment as Example 5 was performed on four different kinds of stem-loops discovered on different transcripts. Specifically, the four stem-loops were PKC SL1, found in hCT20429 (RefSeq: NM_002737), A20 SL1, found in hCT7828 (RefSeq: NM_06290), NOTCH1 SL1, found in hCT1961915.1 (RefSeq: NM_017617), and NUP SL1, found in hCT2252375 (RefSeq: NM_005124).

The sense oligomers and antisense oligomers used are shown in Table 5 (in the Table, each uppercase letter indicates the 5' terminus).

**Table 5-1 PKCα**

| | Sense oligomer | Antisense oligomer |
|---|---|---|
| PKC SL1 | Aagcttggccgcagctccccggcgga ggcaagaggtggttgggggggaccat ggctgacgttttcccgggcaacatgt a (SEQ ID NO:16) | Acatgttgcccgggaaaacgtcagcc atggtccccccaaccacctcttgcc tccgccggggagctgcggccaagctt a (SEQ ID NO:17) |
| PKC SL1m1 | Aagcttggccgcagctccccggcgga ggcgtcagaggtggttgggggggacc atggctgacgttttcccgggcaacat gta (SEQ ID NO:18) | Acatgttgcccgggaaaacgtcagcc atggtcccccccaaccacctctgacg cctccgccggggagctgcggccaagc tt (SEQ ID NO:19) |
| PKC SL1m2 | Aagcttggccgcagctccccggcgga ggcgtcagccgtggttgggggggacc atggctgacgttttcccgggcaacat gta (SEQ ID NO:20) | Acatgttgcccgggaaaacgtcagcc atggtcccccccaaccacggctgacg cctccgccggggagctgcggccaagc tta (SEQ ID NO:21) |

**Table 5-2 A20**

| | Sense oligomer | Antisense oligomer |
|---|---|---|
| A20 SL1 | Aagctttgccttgaccaggacttggg actttgcgaaaggatcgcggggcccg gagaggtgttggagagcacaatggcc atgga (SEQ ID NO:22) | Ccatggccattgtgctctccaacacc tctccgggccccgcgatcctttcgca aagtcccaagtcctggtcaaggcaaa gctta (SEQ ID NO:23) |
| A20 SL1m1 | Aagctttgccttgaccaggacttctc tgggactttgcgaaaggatcgcgggg cccggagaggtgttggagagcacaat ggccatgga (SEQ ID NO:24) | Ccatggccattgtgctctccaacacc tctccgggccccgcgatcctttcgca aagtcccagagaagtcctggtcaagg caaagctt (SEQ ID NO:25) |

**Table 5-3 NOTCH1**

| | Sense oligomer | Antisense oligomer |
|---|---|---|
| NOTCH1 SL1 | Ggaagcttcgcaggaggccgccgccc gggcgcagagggcagccggtggggag gcatgccgccgctcctggcgcccctg cccatggct (SEQ ID NO:26) | Agccatgggcaggggcgccaggagcg gcggcatgcctccccaccggctgccc tctgcgcccgggcggcggcctcctgc gaagcttcc (SEQ ID NO:27) |
| NOTCH1 SL1m1 | Ggaagcttcgcaggaggccgccgccc gggcgcagagggcagccaggggcggc atgccgccgctcctggcgcccctgcc catggct (SEQ ID NO:28) | Agccatgggcaggggcgccaggagcg gcggcatgccgcccctggctgccctc tgcgcccgggcggcggcctcctgcga agcttcc (SEQ ID NO:29) |

**Table 5-4 NUP**

| | Sense oligomer | I Antisense oligomer |
|---|---|---|
| NUP SL1 | Ggaagcttggcgggggaggctctcta aggcctccgcctctgcctctcccgcc cccttacccgccccggagcgggaagc ggcggaggctccgccatggcc (SEQ ID NO:30) | Ggccatggcggagcctccgccgcttc ccgctccggggcgggtaagggggcgg gagaggcagaggcggaggccttagag agcctcccccgccaagcttcc (SEQ ID NO:31) |
| NUP SL1m1 | Ggaagcttggcgggggaggcaaaggc ctccgcctctgcctctcccgcccccct tacccgccccggagcgggaagcggcg gaggctccgccatggcc (SEQ ID NO:32) | Ggccatggcggagcctccgccgcttc ccgctccggggcgggtaagggggcgg gagaggcagaggcggaggcctttgcc tcccccgccaagcttcc (SEQ ID NO:33) |
| NUP SL1m2 | Ggaagcttggcggggaggcagaggc ctccgcctctgcctctcccgcccct tacccgccccggagcgggaagcggcg gaggctccgccatggcc (SEQ ID NO:34) | Ggccatggcggagcctccgccgcttc ccgctccggggcgggtaagggggcgg gagaggcagaggcggaggcctctgcc tcccccgccaagcttcc (SEQ ID NO:35) |
| NUP SL1m3 | Ggaagcttggcgggggaggcagaggc ctccgcctctgcctcccccgcccccct tacccgccccggagcgggaagcggcg gaggctccgccatggcc (SEQ ID NO:36) | Ggccatggcggagcctccgccgcttc ccgctccggggcgggtaagggggcgg gggaggcagaggcggaggcctctgcc tcccccgccaagcttcc (SEQ ID NO:37) |

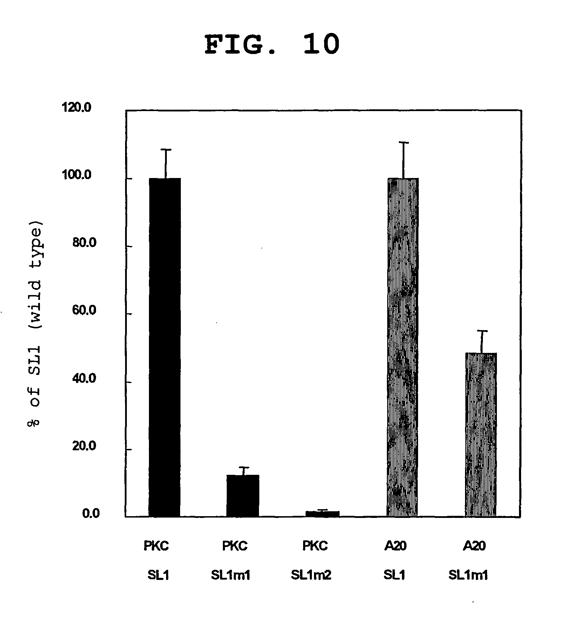

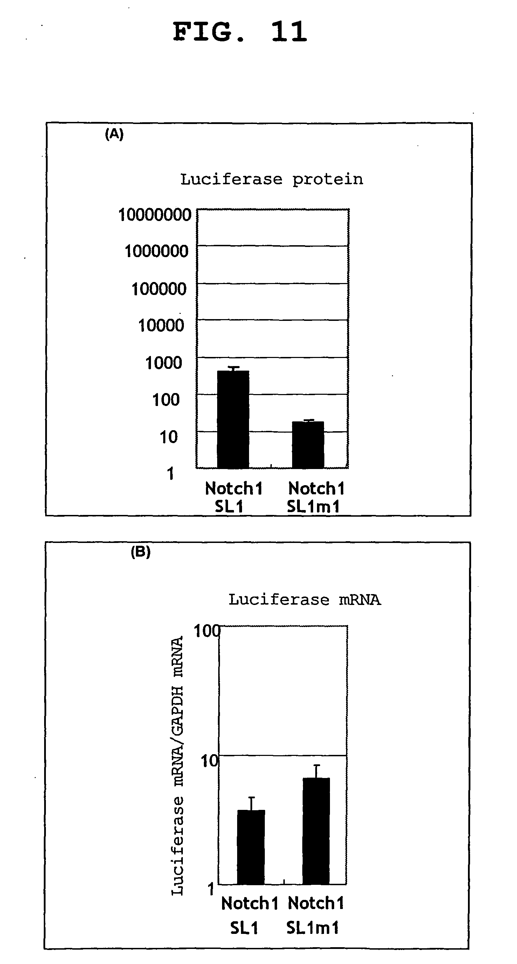

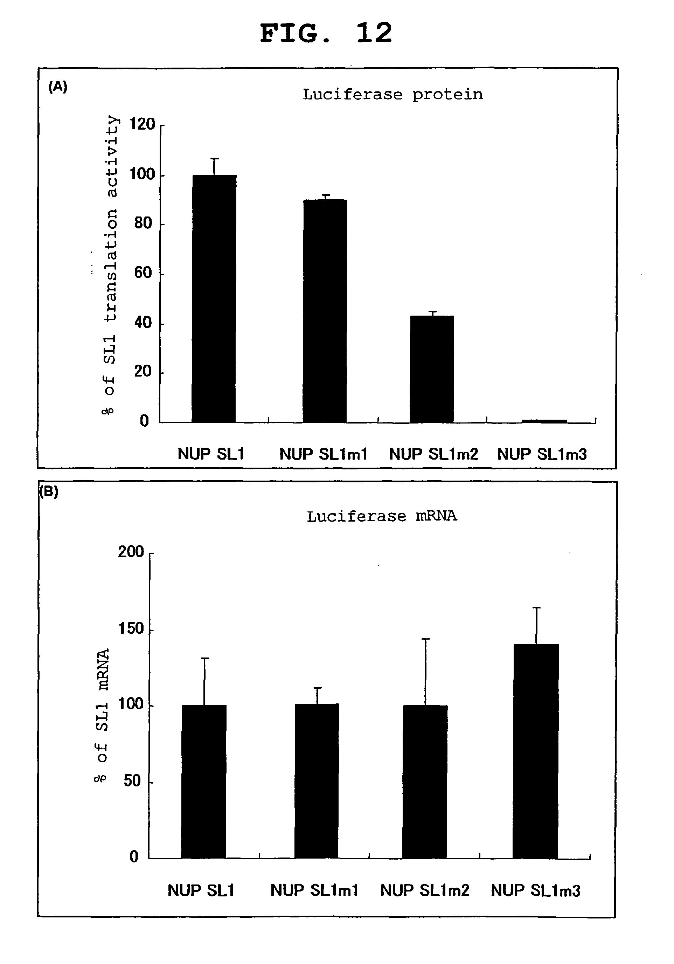

The results are shown in Figures 10, 11, and 12. Compared to the plasmids having PKC SL1, A20 SL1, NUP SL1, or NOTCH1 SL1, the luciferase translation levels from the plasmids having respective corresponding mutant stem-loops decreased significantly (Figures 10, 11, and 12). For NUP SL1 and NOTCH1 SL1, no major change was observed in these mRNA levels (Figures 11B and 12B). From these results, it was suggested that a potent translation suppressive effect might be obtained generally by stabilizing the stem-loop structure. It was also confirmed that ΔG^{TI} differed depending on the region and the structure of the stem-loop. Anyhow, judging from the energy difference between the original stem-loop and the mutant, it can be concluded that if a similar level of energy is added by a compound, the translation from the gene mRNA is suppressed remarkably.

### Example 7: Cell-based screening

Screening for compounds that bind to Suv SL1, PKC SL1, and A20 SL1 to suppress the translation thereof was performed. First, the screening vectors pGL3/Suv SL1, pGL3/PKC SL1, and pGL3/A20 SL1 for Suv SL1 (Table 4), PKC SL1 (Table 5-1), and A20 SL1 (Table 5-2) were transfected to CHO-K1 cells using FuGENE6 in the same protcol as Example 5. Next, using our library (a library consisting of wells each containing 10 kinds of particular compounds), the cells were separately reacted with the wells, and luciferase activities were measured; the luciferase activities obtained were compared, and a compound that selectively reduced the luciferase activity in one well (a well containing the compound) was selected (the luciferase activity on one motif was determined using two motifs for control experiments).

The experiments were specifically performed as described below. First, cells transfected with each screening vector were sown separately to each well containing 3 µM of each of 10 kinds of compounds. 22 hours later, the luciferase activities in all wells were measured, and the separate luciferase activities from each cell using the same well were compared. If the luciferase activity was reduced selectively in a well, that well was selected (a total of 400 wells selected). 10µM solutions of the respective compounds contained in the wells selected were freshly prepared to obtain a new library, and the separate luciferase activities from each cell were compared using the library in the same procedures as those described above. Then, compounds that selectively reduced the luciferase activity (about 80 samples) were selected from among them. Regarding the compounds selected, a concentration dependency test (1, 3, 10, 30 µM) on the subject cells was performed, and a compound tending to selectively reduce the luciferase activity from a certain cell was selected.

As a result, no compound was obtained that selectively reduced the luciferase activity. Possible reasons are that generally the compound concentration was too low, that compounds likely to pass through the cell membrane are essentially unlikely to interact with RNA, and that when a library constructed for proteins is used, a compound that interacts with RNA is unlikely to be obtained, and the like.

### Example 8: Screening by the RNA FRET method

In the RNA FRET method, an experiment was performed to find a compound that binds to and stabilizes a motif having FITC and a quencher at either end of the stem portion of the subject motif structure (Table 6) by examining how the Tm value changes in the presence of the compound. Because the motif per se has too high a Tm value that is disadvantageous for the experiment, a model motif structure having its stem portion shortened around the portion of the motif thought to be bound by a low-molecular compound (bubble portion) was used in the experiment to reduce the Tm value. The sequences thereof are shown in Table 6. The fact that these model motif structures have the same partial structure as the motif structure in the portion thought to be bound by a low-molecular substance was confirmed using mfold. Furthermore, measuring temperatures were determined by an actual experiment wherein the Tm value of each model motif was measured in the absence of the compound with reference to Tm values obtained using mfold. Subsequently, an attempt was made to search for a compound that stabilizes each motif structure using a library.

**Table 6 RNA sequence and measuring temperatures**

| |
|---|
| Suv SL1.4 (67°C) |
| 5'- AGCGGCGGCGGCAUGGGUGCCCCGACGUUGCUAAAA (36 mer) (SEQ ID NO:42) |
| Modification: Double-labeled with FITC at 5' and with Eclipse™Dark Quencher at 3'. |
| TNF ARE (25°C) |
| 5' - GUGAUUAUUUAUUAUUUAUUUAUUAUUUAUUUAUUUAG (38 mer) (SEQ ID NO:43) |
| Modification: Double-labeled with FITC at 5' and with Eclipse™Dark Quencher at 3'. |
| TS SL1.1 (67°C) |
| 5'- UCCGUCCCCCGCCCGCCGCGCCAUGCCUGUGGCCGGCUCGGA (42 mer) (SEQ ID NO:44) |
| Modification: Double-labeled with Eclipse™Dark Quencher at 5' and with FITC at 3'. |
| PKC SL1.2 (45°C) |
| 5'- AGGCAAGAGGUGGUUGGGGGGGACCAUGGCUGACGUUU (38 mer) (SEQ ID NO:45) |
| Modification: Double-labeled with Eclipse™Dark Quencher at 5' and with FITC at 3'. |

The actual experiment was performed as described below. The library used consisted of 279 compounds expected to bind to RNA, selected from our library and commercially available antibiotics. Synthesis of each RNA molecule was outsourced to Takara Bio Inc. A 10-µ1 solution was mixed to obtain a final concentration of 120 nM RNA, 10 mM Tris-HCl (pH 7.5), 50 mM KC1, 0.1 µg/µl acetylated Bovine Serum Albumin (Promega), 2 to 4 µM Rox (5-CARBOXY-X-RHODAMINE, SU; Invitrogen Corp.), and 5 µM EDTA, and the solution was incubated in the presence of each compound at room temperature for 10 minutes. Subsequently, at various measuring temperatures, FITC and Rox values were measured, the FITC/Rox ratio was calculated, and this ratio was analyzed as the FITC value (alternatively, although it was not used in this Example, a method is also available which comprises dispensing the compound to the solution, incubating the solution at an increased temperature of 79°C or more, measuring the FITC value as a control, subsequently reducing the temperature to the measuring temperature to promote the binding of a low-molecular compound to RNA structure, and calculating the FITC value as a percentage in the same manner). Each compound was dispensed to a reaction liquid comprising the model motif to obtain concentrations of 0.1, 1, 10, and 100 µM, and FITC values were obtained at corresponding temperatures to examine the effect of FRET (N=1). With the FITC value in the reaction solution not supplemented with the compound as the control, the FITC value in the reaction solution supplemented with the compound was calculated as a percentage. Then, concentration dependency graphs were drawn, and compounds that concentration-dependently reduced the FITC value were selected.

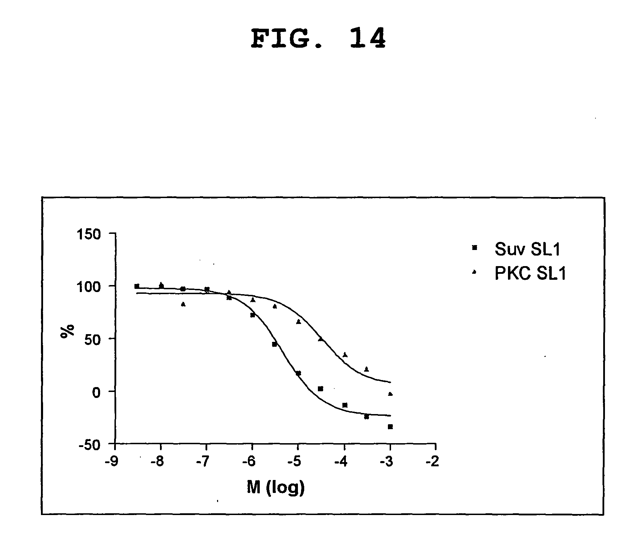

As a result, 20 samples exhibited a FRET effect on one of the RNA strands, suggesting that the RNA might be stabilized. For these 20 samples, a concentration dependency test was performed at concentrations of 3, 10, 30, and 100 µM (N=3). In particular, the present inventors took note of the result that Kanamycin selectively stabilized Suv SL1.4. To date, Kanamycin has been shown to bind to a motif found in Thymidilate Synthase mRNA (TS SL1) (Tok et al. Biochemistry. 38, 199-2061, 999; Cho et al. Nucleic Acids Res. 28, 2158-63, 2000; Lee et al. J. Am. Chem. Soc. 126, 1956-7, 2004); according to an analysis by the present inventors, it was shown that Suv SL1.4 became stabilized at lower concentrations of Kanamycin than TS SL1.1. Selectivity for PKC SL1.2 was also observed. (Figure 14).

### Example 9: Confirmation of in vitro translation inhibition by structure on mRNA

To confirm whether or not the in vitro translation method can be used for screening, an experiment was performed. Luciferase-encoding RNAs having Suv SL1, Suv SL1m1, and SL1m2, respectively, were constructed, and the luciferase activities thereof in an in vitro translation system were measured, whereby it was confirmed that the activities were inhibited proportionally to the ΔG (stability) of the motif structure introduced.

The experiment was performed using Suv SL1, which had been used for demonstrative experiments, and Suv SL1m1 and Suv SL1m2, which had been stabilized by base-modification of Suv SL1, (Figure 15C). The 5'-terminus of a Survivin mRNA comprising each of these motifs was replaced with the firefly luciferase 5'UTR of the pGL3-control plasmid, and a plasmid for transcription from the T7 promoter was prepared. Specifically, first, a sense oligomer (GCTAG CTAAT ACGAC TCACT ATAGG AAGCT TA (SEQ ID NO:46): final concentration 12.5 µM) and an anti-sense oligomer (AAGCT TCCTA TAGTG AGTCG TATTA GCTAG CA (SEQ ID NO:47): final concentration 12.5 µM) were mixed in annealing buffer (final concentration: 10 mM Tris HCL, pH 8.0/1 mM EDTA/100 mM NaCl), and incubated at 95°C for 4 minutes and at room temperature for 5 minutes. Then, the annealed oligomers were cloned into pCR-XL-TOPO^{®}. Furthermore, an Nhe I/Hind III fragment comprising the T7 promoter region (Figure 15A) was cut out from this plasmid, and inserted to the Nhe I/Hind III site of each of pGL3-control (Promega), pGL3/Suv SL1, pGL3/Suv SL1m1, and pGL3/Suv SL1m2. The SV40 promoter contained in these plasmids was thereby replaced with the T7 promoter. The plasmids prepared were named pGL3/T7, pGL3/Suv SL1/T7, pGL3/Suv SL1m1/T7, and pGL3/Suv SL1m2/T7, respectively. Finally, the base sequence of the T7 promoter was confirmed by sequencing.

pGL3/Suv SL1/T7, which comprises Suv SL1, pGL3/Suv SL1m1/T7, which comprises Suv SL1m1, pGL3/Suv SL1m2/T7, which comprises Suv SL1m2, and pGL3/T7, which comprises a sequence having no remarkable motif, were mixed with an in vitro translation system, and luciferase activity was monitored, whereby translation levels were quantified. In vitro translation was performed using TNT T7 Quick Coupled Transcription/Translation System (Promega) per the protocol thereof with a partial modification. Specifically, TNT Quick Master Mix (3.75 µL), 1 mM (0.09375 µL) of methionine, and 0.5 µg/µL (0.1875 µL) of plasmid were mixed to obtain a final volume of 5 µL. Then, the mixture was incubated at 30°C for 1 hour. Subsequently, Bright-Glo^{™} Luciferase Assay System (Promega) (30 µL), previously diluted 2 fold with distilled water, was added, and the mixture was incubated at room temperature for 5 minutes. Then, luciferase activities were measured using Wallac ARVO-SX.

As a result, the luciferase activities from the respective plasmids were 4451 K units for pGL3/T7, 1999 K units for pGL3/Suv SL1/T7, 198 K units for pGL3/Suv SL1m1/T7, and 37 K units for pGL3/Suv SL1m2/T7; translation levels decreased as the stability of the motif structure increased, in agreement with the result of the cell-based reporter assay. This result suggested that in this in vitro translation system as well, the protein expression level changes proportionally to the stability of 5'UTR. Furthermore, it was suggested that using the same system, it might be possible to perform screening for a low-molecular compound that stabilizes the motif structure to reduce the translation level.

### Example 9: Screening by the in vitro translation method

In screening for a low-molecular compound by the in vitro translation method, screening was performed by measuring and comparing luciferase activities in the presence of a given amount of a low-molecular compound in an in vitro translation system of transcripts having a target motif.

An example of screening for Survivin SL1 is shown below. As in Example 8, pGL3/T7, pGL3/Suv SL1/T7, pGL3/Suv SL1m1/T7, and pGL3/Suv SL1m2/T7 were constructed. Furthermore, the low-molecular compound was prepared as a DMSO solution, fully dissolved by sonication and heat treatment. The in vitro translation was also performed using TNT T7 Quick Coupled Transcription/Translation System (Promega) per the protocol thereof with a partial modification. Specifically, the low-molecular compound (final concentration was 100 µM; the final DMSO concentration was not more than 2%.) was mixed in TNT Quick Master Mix (3.75 µL), 1 mM (0.09375 µL) of methionine, and 0.5 µg/µL (0.1875 µL) of plasmid to obtain a final volume of 5 µL. Then, the mixture was incubated at 30°C for 1 hour. Subsequently, Bright-Glo^{™} Luciferase Assay System (Promega) (30 µL), previously diluted 2 fold with distilled water, was added, and the mixture was incubated at room temperature for 5 minutes. Then, luciferase activities were measured using Wallac ARVO-SX.

Hence, if the activity from pGL3/Suv SL1, which has the motif, is significantly lower than the activity from pGL3/T7, the compound can be judged to be a promising compound. Furthermore, by comparing the activity from pGL3/Suv SL1 with the activities from pGL3/Suv SL1m1/T7 and pGL3/Suv SL1m2/T7, the selectivity of the compound can be estimated. That is, because pGL3/Suv SL1/T7 and pGL3/Suv SL1m1/T7 differ from each other only in terms of the structure on the motif, the compound is considered to bind to the vicinity of the different place to stabilize the motif. A measurement of this selectivity can sometimes be accomplished using a plasmid prepared from a different target motif (for example, PKCα described above).

### Industrial Applicability

Because the method of the present invention enables screening for a low-molecular compound that is more advantageous in terms of properties for pharmaceuticals, and also because the steps to obtainment of a lead compound are constant irrespective of the subject disease, unlike in protein-targeting drug discovery, the method of the present invention is highly useful in that it enables time reduction, streamlining and cost reduction in drug discovery scheme. Because the method is applicable to drug discovery for a low-molecular pharmaceutical that targets any RNA having a structure and function, that specifically acts in vivo, the method is also highly useful in drug discovery for functional RNAs such as miRNAs, which are suggested to play an important role in the regulation of gene expression. Furthermore, the secondary structure search software used in the present invention can also be applied to the detection of a novel miRNA on the genome.

While the present invention has been described stressing the preferable embodiments, it will, however, be evident for those of ordinary skill in the art that changes may be made to the preferable embodiments. The present invention intends to be practiced by methods other than those described in the present specification. Therefore, the present invention includes any changes encompassed in the spirit and scope of the appended claims.

This application is based on a patent application No. 2004-336267 filed in Japan, the contents of which are incorporated in full herein by this reference. In addition, the references cited herein, including patents and patent applications, are hereby incorporated in full by reference, to the extent that they have been disclosed herein.

## Claims

1. A method of selecting a sequence capable of assuming a particular local secondary structure in a particular mRNA for screening for a compound capable of regulating the translation of said mRNA, which comprises the following steps:
(1) a step wherein an mRNA search means searches an mRNA data set inputted and extracts mRNA(s) having a sequence, in the molecule, capable of assuming a local secondary structure capable of regulating the translation of the mRNA(s),
(2) a step for selecting a particular mRNA from among the one or more mRNAs extracted in (1) above,
(3) a step for selecting a particular sequence from among one or more sequences capable of assuming a local secondary structure in the particular mRNA selected in (2) above,
(4) a step wherein a specificity confirmation means searches an mRNA data set inputted and confirms that a partial structure capable of interacting with said compound in the particular sequence selected in (3) above is not present in the region involved in the regulation of the translation of other mRNA, or even if present, is incapable of regulating the translation.

2. The method of claim 1, wherein the local secondary structure is a stem-loop structure.

3. The method of claim 2, wherein the stem-loop structure has two or more stem portions and has a bubble portion not having complementarity between the stems.

4. The method of claim 2 or 3, wherein the compound that regulates the translation of a particular mRNA is capable of interacting with the loop portion or bubble portion of the stem-loop structure.

5. A method of selecting a sequence capable of assuming a particular local secondary structure in a particular mRNA for screening for a compound capable of suppressing the translation of said mRNA, which comprises the following steps:
(1) a step wherein an mRNA search means searches an mRNA data set inputted and extracts mRNA(s) having a sequence, in the molecule, capable of assuming a local secondary structure capable of suppressing the translation of the mRNA(s),
(2) a step for selecting a particular mRNA from among the one or more mRNAs extracted in (1) above,
(3) a step for selecting a particular sequence from among one or more sequence capable of assuming a local secondary structure in the particular mRNA selected in (2) above,
(4) a step wherein a specificity confirmation means searches an mRNA data set inputted and confirms that a partial structure capable of interacting with said compound in the particular sequence selected in (3) above is not present in the region involved in the regulation of the translation of other mRNA, or even if present, is incapable of suppressing the translation.

6. The method of claim 5, wherein the sequence capable of assuming a particular local secondary structure is present in a region consisting of a 5' non-translational region and a coding region.

7. The method of claim 6, wherein the sequence capable of assuming a particular local secondary structure is present in a region consisting of a translation initiation point and the vicinity thereof.

8. The method of claim 1, wherein regulating the translation of the particular mRNA is effective in the prophylaxis/treatment of one or more diseases.

9. A method of selecting a sequence capable of assuming a particular local secondary structure in a particular mRNA, for screening for a compound capable of preventing/treating a particular disease, which comprises the following steps:
(1) a step for selecting one or more mRNAs whose regulation of translation can result in the prevention/treatment of the disease,
(2) a step wherein an mRNA search means searches a data set of the one or more mRNAs selected in (1) above, and extracts mRNA(s) having a sequence, in the molecule, capable of assuming a local secondary structure capable of regulating the translation of the mRNA(s),
(3) a step for selecting a particular mRNA from among the one or more mRNAs extracted in (2) above,
(4) a step for selecting a particular sequence from among one or more sequences capable of assuming a local secondary structure in the particular mRNA selected in (3) above,
(5) a step wherein a specificity confirmation means searches an mRNA data set inputted and confirms that a partial structure capable of interacting with said compound in the particular sequence selected in (4) above is not present in the region involved in the translation of other mRNA, or even if present, is incapable of regulating the translation.

10. A screening method for a compound that interacts with a sequence capable of assuming a particular local secondary structure selected by the method of claim 1 to regulate the translation of a particular mRNA, which comprises a step for bringing an RNA strand comprising said sequence and a test compound into contact with each other, and measuring changes in the stability of said secondary structure of said RNA strand.

11. A screening method for a compound that regulates the translation of a particular mRNA, which comprises the following steps:
(1) a step for searching mRNA(s) having a sequence, in the molecule, capable of assuming a local secondary structure capable of regulating the translation of the mRNA(s),
(2) a step for bringing an RNA strand comprising a sequence capable of assuming a particular local secondary structure in the particular mRNA extracted in (1) above and a test compound into contact with each other, and measuring changes in the stability of said secondary structure of said RNA strand.

12. A screening method for a compound that suppresses the translation of a particular mRNA, which comprises the following steps:
(1) a step for searching mRNA(s) having a sequence, in the molecule, capable of assuming a local secondary structure capable of suppressing the translation of the mRNA(s),
(2) a step for bringing an RNA strand comprising a sequence capable of assuming a particular local secondary structure in the particular mRNA extracted in (1) above and a test compound into contact with each other, and measuring changes in the stability of said secondary structure of said RNA strand.

13. A screening method for a compound capable of preventing/treating a particular disease, which comprises the following steps:
(1) a step for selecting one or more mRNAs whose regulation of translation can result in the prevention/treatment of the disease,
(2) a step for searching mRNA(s) having a sequence, in the molecule, capable of assuming a local secondary structure capable of regulating the translation of the mRNA(s) from among the mRNAs selected in (1) above,
(3) a step for selecting a particular sequence from among sequences capable of assuming a local secondary structure in the particular mRNA extracted in (2) above,
(4) a step for confirming that a partial structure capable of interacting with the compound in the particular sequence selected in (3) above is not present in the region involved in the regulation of the translation of other mRNA, or even if present, is incapable of regulating the translation,
(5) a step for bringing an RNA strand comprising a particular sequence confirmed to be not present in the region involved in the regulation of the translation of other mRNA in (4) above and a test compound into contact with each other, and measuring changes in the stability of said secondary structure of said RNA strand.

14. A method of predicting a compound that exhibits an adverse reaction by regulating the translation of mRNA, which comprises the following steps:
(1) a step for searching mRNA(s) having a sequence, in the molecule, capable of assuming a local secondary structure capable of regulating the translation of the mRNA(s),
(2) a step for bringing an RNA strand comprising a sequence capable of assuming a local secondary structure in the mRNA extracted in (1) above and a test compound into contact with each other, and detecting the presence or absence of an RNA strand whose secondary structure is stabilized in the presence of said compound.

15. A nucleic acid substantially consisting of the same or substantially the same base sequence as the base sequence shown by any of SEQ ID NO:1 and 38 to 41.

16. The nucleic acid of claim 15, wherein one or more base pairs can be formed in a bubble by introducing a mutation.

17. An expression vector comprising an expression cassette having the nucleic acid of claim 15 or 16 inserted between a promoter and a reporter gene.

18. A transformant obtained by transforming a host cell with the expression vector of claim 17.

19. A kit for screening for a compound capable of suppressing the translation of an mRNA that encodes a protein selected from the group consisting of Survivin, PKCα, A20, NOTCH1 and NUP, which comprises the nucleic acid of claim 15, the expression vector of claim 17 or the transformant of claim 18.

20. A prophylactic/therapeutic agent for cancer comprising a compound capable of suppressing the translation from an mRNA that encodes a protein selected from the group consisting of Survivin, PKCα, A20, NOTCH1 and NUP, wherein the translation is suppressed by the compound that causes the mRNA to form a local secondary structure in the molecule of said mRNA.

21. The agent of claim 20, wherein the local secondary structure is formed in the base sequence shown by any of SEQ ID NO:1 and 38 to 41.
